# EUROPEAN PATENT APPLICATION

(11) **EP 4 293 018 A1**
(43) Date of publication of application: **20.12.2023**
(21) Application number: 22752345.3
(22) Date of filing: 10.02.2022
(51) Int. Cl.: C07D 401/06, C07D 401/12, C07D 401/14, C07D 231/18, C07D 405/00, C07D 407/12, C07D 413/12, C07D 417/12, C07D 487/04, C07D 213/52, A61K 31/64, A61P 25/00, A61P 29/00, A61P 35/00, A61P 37/00

(54) **COMPOUND SERVING AS NLRP3 INHIBITOR**

(30) Priority: 10.02.2021 CN 202110185418; 20.05.2021 CN 202110554272
(71) Applicant: Hangzhou Innogate Pharma Co., Ltd., Hangzhou, Zhejiang 311121 (CN)
(72) Inventor: ZHANG, Hancheng, Hangzhou, Zhejiang 311121 (CN); JIA, Wei, Hangzhou, Zhejiang 311121 (CN); CAI, Congcong, Hangzhou, Zhejiang 311121 (CN)
(74) Representative: Lavoix
(86) International application number: PCT/CN2022/075940
(87) International publication number: WO 2022/171185

(57) **Abstract**

The present invention provides a compound serving as an NLRP3 inhibitor. Specifically, the present invention provides a compound having a structure shown in the following formula (I), or an optical isomer, pharmaceutically acceptable salt, prodrug, deuterated derivative, hydrate, and solvate thereof. The compound can be used for treating or preventing diseases or disorders associated with the activity or expression level of NLRP3.

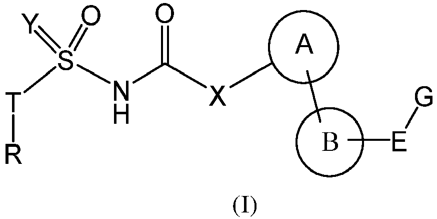

## Description

### Technical field

The present invention relates to the field of medicinal chemistry; in particular, the present invention relates to a novel class of derivatives containing a tricyclic heteroaryl group, a method for its synthesis and its use as inhibitor for NLRP3 in the preparation of a medicament for the treatment of related diseases such as tumors.

### Background

NLRP3 (nod like receptor family, pyrin domain containing protein 3) inflammasome is a complex composed of a variety of proteins, including the core component of nucleotide binding oligomerization domain-like receptor protein 3 (NLRP3), apoptosis-associated speck-like protein containing a caspase activation and recruitment domain (ASC), and the precursor of caspase-1, which is mainly involved in the inflammatory response process of the body. As an intracellular receptor protein, NLRP3 can sense specific inflammatory signals. After receiving stimulation, NLRP3 binds to the adapter protein ASC, forming the NLRP3-ASC complex. The polymerized NLRP3-ASC and caspase-1 precursor form a larger complex, which is the inflammasome. The generation of inflammasome leads to the activation of caspase-1, which in turn cleaves the inflammatory factors IL-1β and IL-18 precursors, processes and secretes active IL-1β and IL-18, promoting the occurrence of inflammatory reactions and even leading to inflammatory cell death, namely pyroptosis. ASC can also recruit and activate caspase-8, cleave IL-1β and IL-18 precursors, and induce cell apoptosis.

Caspase-1 cleaves IL-1β and IL-18 precursors, generates active IL-1β and IL-18, and secretes them out of the cell. Activated caspase-1 can also cleave GSDMD (gasdermin-D) and induce cell pyroptosis. By regulating the cell death pathway of pyroptosis, caspase-1 can also mediate the release of warning factors such as IL-33 and HMGB1 (high mobility group box 1). Caspase-1 can also cleave intracellular IL-1R2 receptor, leading to its degradation and release IL-1α. In addition, other substrates of caspase-1, such as cytoskeleton proteins and proteins involved in glycolysis signaling pathway, may also participate in caspase-1 dependent inflammatory response.

The cytokines activated by NLRP3 inflammasomes can promote the occurrence of inflammatory reactions and, together with other cytokine signaling pathways, shape the immune response of the body to infection and injury. For example, the IL-1β signaling pathway induces the release of pro-inflammatory factors IL-6 and tumor necrosis factor. Without the participation of T-cell receptor, IL-1β, IL-18 and IL-23 synergistically induce memory CD4 Th17 cells andγδT cells to produce IL-17. IL-18 and IL-12 can also work together to induce memory T cells and natural killer cells to secrete IFN - γ and promote Th1 immune response.

Other intracellular pattern recognition receptors can also form inflammasomes, such as NLRP1 and NLRP4, members of the NLR (nod like receptor) family, and non NLR family members, such as the double- stranded DNA sensor AIM2 (absent in melanoma 2) and IFI16 (interference, gamma inducible protein 16). The indirect, non-canonical signaling pathway downstream of caspase-11 can also activate NLRP3 dependent IL-1β.

Abnormal activation of NLRP3 can lead to many diseases, such as genetic disorder cryopyrin-associated periodic syndrome (CAPS) caused by NLRP3 acquired mutation, including Muckle-Wells syndrome (MWS), familial cold autoinflammatory syndrome (FCAS), neonatal-onset multisystem inflammatory disease. In addition, NLRP3 is also involved in a variety of complex diseases, such as multiple sclerosis, type 2 diabetes, obesity, Alzheimer's disease, gout and atherosclerosis. The role of NLRP3 in central nervous system, lung, liver and kidney diseases is also getting more and more attention. Therefore, NLRP3 inhibitors have broad clinical application prospects.

### Summary of the invention

The purpose of the present invention is to provide a novel NLRP3 inhibitor.

In the first aspect of the present invention, a compound of the following formula (I), or the optical isomers, pharmaceutically acceptable salts, prodrugs, deuterated derivatives, hydrates, or solvates thereof is provided: 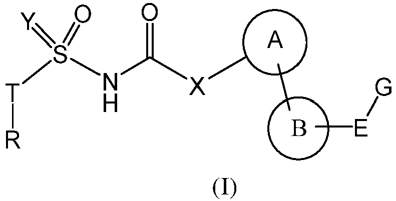 wherein:
ring A is selected from the group consisting of substituted or unsubstituted 8-15 membered bicyclic or tricyclic fused ring systems; wherein, the "substituted" means the hydrogen atoms on the group are substituted by one or more R^{e}; the bicyclic or tricyclic fused ring system comprises at least one aromatic ring and one or two saturated or unsaturated rings fused with the aromatic ring, and the connecting site of ring A and X locating on the aromatic ring;
ring B is selected from the group consisting of none, substituted or unsubstituted aryl, substituted or unsubstituted 5-12 membered heterocycle(including partially unsaturated or saturated heterocycle), or substituted or unsubstituted heteroaryl; wherein, the "substituted" means the hydrogen atoms on the group are substituted by one or more R^{f}; and when B is none, E and G are absent;
X is selected from -NR⁵-, -CR⁶R⁷-;
Y is selected from O, -NR⁵-;
T is selected from chemical bond, -NR⁵-, -(CR^{a}R^{b})₁₋₂-, C₃₋₆cycloalkyl, 3-8 membered heterocyclyl, aryl, and heteroaryl;
R is selected from the group consisting of hydrogen, C₁₋₄ alkyl, C₁₋₄haloalkyl, C₂₋₄ alkenyl, C₂₋₄alkynyl, C₃₋₈cycloalkyl, 3-8membered heterocyclyl, aryl, heteroaryl, and NR⁸R⁹; wherein, the alkyl, alkenyl, alkynyl, cycloalkyl, heterocyclyl, aryl, or heteroaryl groups are optionally substituted by one or more substituentss selected from the group consisting of halogen, C₁₋₄ alkyl, C₁₋₄haloalkyl, C₂₋₄haloalkenyl, C₁₋₄alkoxy, C₁₋₄haloalkoxy, NR⁸R⁹, CN, NO₂, SR^{h}, C (O) R^{t}, C (O) OR^{h}, C(O)NR^{h}R^{h}, NR^{h}C(O)R^{t}, NRhS(O)₂R^{t}, and S(O)₂R^{t}; or the cycloalkyl or heterocyclic group is substituted by =M, where M is selected from O or CR¹⁰R¹¹;
E is selected from the group consisting of chemical bond, -O-, -O(CR^{a}R^{b})₁₋₂-, -(CR^{a}R^{b})₁₋₂O-, -S-, -S(CR^{a}R^{b})₁₋₂-, -(CR^{a}R^{b})₁₋₂S-, -NR⁵-, -(CR^{a}R^{b})₁₋₂NR⁵-, -NR⁵(CR^{a}R^{b})₁₋₂-, C₁₋₂alkylene, -C=C-, -C=C-, and C₃₋₆cycloalkyl;
G is selected from the group consisting of hydrogen, C₁₋₄ alkyl, C₁₋₄haloalkyl, C₂₋₄ alkenyl, C₂₋₄alkynyl, saturated C₃₋₈cycloalkyl, unsaturated C₃₋₈cycloalkyl, saturated 3-12 membered heterocyclyl, unsaturated 3-12 membered heterocyclyl, aryl, heteroaryl, and NR⁸R⁹; wherein, the cycloalkyl, heterocyclyl, aryl, or heteroaryl groups are optionally substituted by one or more substituents selected from the group consisting of halogen, C₁₋₄ alkyl, C₁₋₄haloalkyl, C₂₋₄haloalkenyl, C₁₋₄alkoxy, C₁₋₄haloalkoxy, NR⁸R⁹, CN, NO₂, SR^{h}, C(O)R^{t}, C(O)OR^{h}, C(O)NR^{h}R^{h}, NR^{h}C(O)R^{t}, NR^{h}S(O)₂R^{t}, and S(O)₂R^{t};
R⁵ is selected from the group consisting of H, C₁₋₄ alkyl, and C₃₋₆ cycloalkyl;
R⁶ and R⁷ are independently selected from the group consisting of hydrogen, halogen, C₁₋₄ alkyl, and C₃₋₆cycloalkyl; or R⁶ and R⁷ together with their connected carbon atoms form a C₃₋₆cycloalkyl, or a 4-6 membered heterocyclylhaving 1 or 2 heteroatoms selected from N, O, S;
R⁸ and R⁹ are independently selected from the group consisting of hydrogen, C₁₋₄ alkyl, C₃₋₆cycloalkyl, and 4-8 membered heterocyclyl; the cycloalkyl or heterocyclyl is optionally substituted by "=M", wherein M is selected from O or CR¹⁰R¹¹; or R⁸ and R⁹ together with their connected nitrogen atoms form a 4-8 membered heterocyclyl, wherein the heterocyclyl contains 1 or 2 N atoms and 0 or 1 heteroatom selected from O, S;
R¹⁰ and R¹¹ are independently selected from the group consisting of hydrogen, deuterium, halogen, and C₁₋₄ alkyl; wherein, the alkyl is optionally substituted by one or more substituents selected from the group consisting of hydrogen, halogen, C₁₋₄ alkyl, C₁₋₄haloalkyl, C₁₋₄alkoxy, C₁₋₄ haloalkoxy, NR⁸R⁹, C₃₋₈cycloalkyl, 3-8 membered heterocyclyl, aryl, and heteroaryl; or R¹⁰ and R¹¹ together with their connected carbon atoms form a 3-6 membered cycloalkyl, or a 4-8 membered heterocyclyl having 1 or 2 heteroatoms selected from N, O, and S;
R^{a} and R^{b} are independently selected from the group consisting of H, halogen, C₁₋₄ alkyl, or C₃₋₆ cycloalkyl;
R^{e} and R^{f} are independently selected from the group consisting of deuterium, halogen, C₁₋₄ alkyl, C₁₋₄haloalkyl, C₂₋₄ alkenyl, C₂₋₄alkynyl, C₃₋₆ cycloalkyl, 4-8 membered heterocyclyl, aryl, heteroaryl, CN, NO₂, OR^{h}, SR^{h}, NR^{h}R^{h}, C(O)R^{t}, C(O)NR^{h}R^{h};
R^{t} is C₁₋₄ alkyl, C₂₋₄ alkenyl, C₂₋₄alkynyl, C₃₋₈cycloalkyl, 4-8 membered heterocyclyl, aryl, or heteroaryl;
each R^{h} is independently hydrogen or C₁₋₄ alkyl; or two R^{h} together with their connected nitrogen atoms form a 3-8 membered heterocyclyl having 1 or 2 N atoms and 0 or 1 heteroatom selected from O and S;
wherein, each of the abovementioned alkyl, alkenyl, alkynyl, cycloalkyl, heterocyclyl, aryl, and heteroaryl is optionally and independently substituted by 1-3 substituents selected from the group consisting of halogen, C₁₋₄ alkyl, C₁₋₄haloalkyl, C₂₋₄ alkenyl, C₂₋₄alkynyl, C₃₋₈cycloalkyl, 3-8 membered heterocyclyl, aryl, heteroaryl, CN, NO₂, OR^{h}, SR^{h}, NR^{h}R^{h}, C(O)R^{t}, C(O)OR^{h}, C(O)NR^{h}R^{h}, NR^{h}C(O)R^{t}, NR^{h}S(O)₂R^{t}, and S(O)₂R^{t}, the premise is that the resulting chemical structure is stable and meaningful; wherein, R^{h} and R^{t}are as described above;
unless otherwise specified, the aryl described above is aromatic group containing 6-12 carbon atoms; the heteroaryl is 5-15 membered (preferably 5-12 membered) heteroaromatic groups.

In another preferred embodiment, ring A is a tricyclic fused ring system, and R is an unsaturated C₃₋₈cycloalkyl (nonaromatic) or unsaturated 3-8 membered heterocyclyl (nonaromatic).

In another preferred embodiment, ring A is a tricyclic fused ring system, and R is an C₃₋a cycloalkyl or unsaturated 3-8 membered heterocyclyl, and R is substituted at least by one =M.

In another preferred embodiment, R is a C₃₋₈ cycloalkyl or 3-8 membered heterocyclyl, and T is -NR⁵-, then R is at least substituted by one substituent selected from the group consisting of fluorine, C₁₋₄ fluoroalkyl, and C₂₋₄ fluoroalkenyl.

In another preferred embodiment, ring A is a bicyclic fused ring system, and G is not an unsaturated 3-12 membered heterocyclyl (nonaromatic), saturated 3-12 membered spiro-heterocyclyl, saturated 3-12 membered fused-heterocyclyl, and saturated 3-12 membered bridge-heterocyclyl.

In another preferred embodiment, R is C₃₋₈ cycloalkyl or 3-8 membered heterocyclyl, and R is at least substituted by one =M.

In another preferred embodiment, R is C₃₋₈ cycloalkyl or 3-8 membered heterocyclyl, and T is -NR⁵-, and R is at least substituted by one substituent selected from the group consisting of fluorine, C₁₋₄ fluoroalkyl, and C₂₋₄ fluoroalkenyl.

In another preferred embodiment, R is selected from C₃₋₈cycloalkyl or 3-8 membered heterocyclyl; wherein, the cycloalkyl or heterocyclyl is optionally substituted by one or more substituents selected from the group consisting of halogen, C₁₋₄ alkyl, C₁₋₄ haloalkyl, C₂₋₄haloalkenyl, C₁₋₄alkoxy, C₁₋₄haloalkoxy, NR⁸R⁹, and =M, wherein, M is selected from O or CR¹⁰R¹¹;

A, B, E, G, X, Y, T, R⁸, R⁹, R¹⁰, R¹¹ are as described as above.

In another preferred embodiment, R is selected from C₃₋₈ cycloalkyl or 3-8 membered heterocyclyl; wherein, the cycloalkyl or heterocyclyl is optionally substituted by =M, wherein M is CR¹⁰R¹¹; wherein, R¹⁰ and R¹¹ are as above.

In another preferred embodiment, 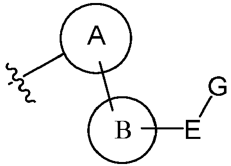 is selected from 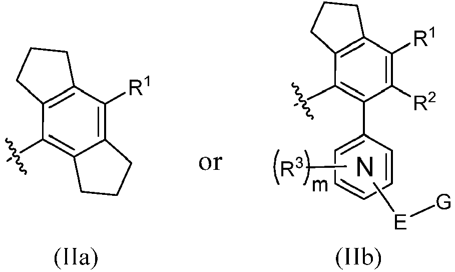
" 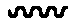 " represents the connecting site of the above structural fragments of formula (IIa) or (IIb) with the rest moiety of formula(I);
R¹ and R² are independently selected from the group consisting of hydrogen, deuterium, halogen, C₁₋₄ alkyl, C₁₋₄haloalkyl, C₁₋₄alkoxy, and C₁₋₄haloalkoxy;
each R³ is independently selected from the group consisting of hydrogen, deuterium, halogen, and C₁₋₄alkyl;
m is 0, 1, 2 or 3; 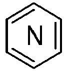 is pyridine, pyrimidine, or pyridazine;
E is selected from the group consisting of chemical bond, -O-, -O(CR^{a}R^{b})₁₋₂-, -(CR^{a}R^{b})₁₋₂O-, -S-, -S(CR^{a}R^{b})₁₋₂-, -(CR^{a}R^{b})₁₋₂, -NR⁵-, -(CR^{a}R^{b})₁₋₂NR⁵-, -NR⁵(CR^{a}R^{b})₁₋₂-, C₁₋₂alkylene, -C=C-, and -C=C-; wherein, R^{a} and R^{b} are independently selected from hydrogen and C₁₋₄alkyl;
G is selected from unsaturated C₃₋₈cycloalkyl or unsaturated 3-12 membered heterocyclyl; wherein the cycloalkyl or heterocyclyl is optionally substituted by one or more substituents selected from the group consisting of halogen, C₁₋₄ alkyl, C₁₋₄haloalkyl, C₂₋₄haloalkenyl, C₁₋₄alkoxy, C₁₋₄haloalkoxy, NR⁸R⁹, CN, NO₂, SR^{h}, C(O)R^{t}, C(O)OR^{h}, C(O)NR^{h}R^{h}, NR^{h}C(O)R^{t}, NR^{h}S(O)₂R^{t}, and S(O)₂R^{t};
R⁵, R⁸, R⁹, R^{h} and R'are as described above.

In another preferred embodiment, the formula(I) has the structure of formula (III): 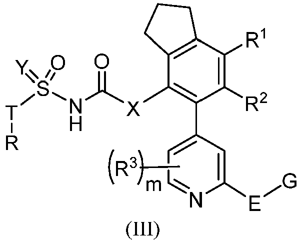
R¹ and R² are independently selected from the group consisting of hydrogen, deuterium, halogen, C₁₋₄ alkyl, C₁₋₄haloalkyl, C₁₋₄alkoxy, and C₁₋₄haloalkoxy;
each R³ is independently selected from the group consisting of hydrogen, deuterium, halogen, and C₁₋₄alkyl;
m is 0, 1 or 2;
E is selected from the group consisting of chemical bond, -O-, -O(CR^{a}R^{b})₁₋₂-, -(CR^{a}R^{b})₁₋₂O-, -S-, -S(CR^{a}R^{b})₁₋₂-, -(CR^{a}R^{b})₁₋₂S-, -NR⁵-, -(CR^{a}R^{b})₁₋₂NR⁵-, -NR⁵(CR^{a}R^{b})₁₋₂-, C₁₋₂alkylene, -C=C-, -C=C-; wherein, R^{a} and R^{b} are independently selected from hydrogen and C₁₋₄alkyl;
G is selected from unsaturated C₃₋₈cycloalkyl or unsaturated 3-12 membered heterocyclyl; wherein, the cycloalkyl or heterocyclyl is optionally substituted by one or more substituents selected from the group consisting of halogen, C₁₋₄ alkyl, C₁₋₄haloalkyl, C₂₋₄haloalkenyl, C₁₋₄alkoxy, C₁₋₄haloalkoxy, NR⁸R⁹, CN, NO₂, SR^{h}, C(O)R^{t}, C(O)OR^{h}, C(O)NR^{h}R^{h}, NR^{h}C(O)R^{t}, NR^{h}S(O)₂R^{t}, and S(O)₂R^{t};
X, Y, T, R, R⁵, R⁸, R⁹, R^{h} and R^{t} are as described above.

In another preferred example, said G is selected from the group consisting of: 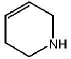 or 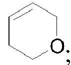 and said G may be optionally substituted with one or more groups selected from the group consisting of: halogen, C₁₋₄ alkyl, C₁₋₄ haloalkyl, C₂₋₄ haloalkenyl, C₁₋₄ alkoxy, C₁₋₄ haloalkoxy, NR⁸R⁹, CN, NO₂, SR^{h}, C(O)R^{t}, C(O)OR^{h}, C(O)NR^{h}R^{h}, NR^{h}C(O)R^{t}, NR^{h}S(O)₂R^{t}, or S(O)₂R^{t}.

In another preferred embodiment, E is selected from the group consisting of chemical bond, -O-, -O(CR^{a}R^{b})₁₋₂-, -(CR^{a}R^{b})₁₋₂O-, -NR⁵-, - (CR^{a}R^{b})₁₋₂NR⁵-, - NR⁵(CR^{a}R^{b})₁₋₂-, and - C=C-; wherein, R^{a} and R^{b} are independently selected from hydrogen and C₁₋₄alkyl.

In another preferred embodiment, the formula(I) has the structure of formula (IV): 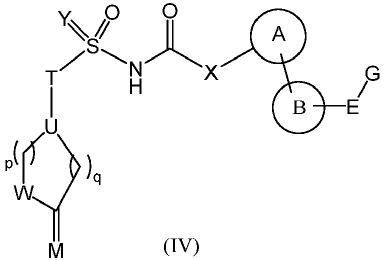
M is selected from CR¹⁰R¹¹; wherein, R¹⁰ and R¹¹ are as described above;
U is selected from N or CR¹²; wherein, R¹² is selected from hydrogen, halogen and C₁₋₄alkyl;
W is selected from chemical bond, -NR¹³(CR^{c}R^{d})₁₋₂-, and -O(CR^{c}R^{d})₁₋₂-; wherein, R¹³ is selected from hydrogen, C₁₋₄alkyl, C₃₋₆cycloalkyl, 3-6 membered heterocyclyl, aryl, heteroaryl, C(O)R^{t}, and S(O)₂R^{t}; R^{c} and R^{d} are independently selected from hydrogen and C₁₋₄alkyl;
p and q are independently selected from 0, 1, 2, 3, 4, 5 and 6; the premise is that p and q are not 0 at the same time;
A, B, E, G, X, Y, T and R'are as described as above.

In another preferred embodiment, the formula(I) has the structure of formula (Va) or formula (Vb): 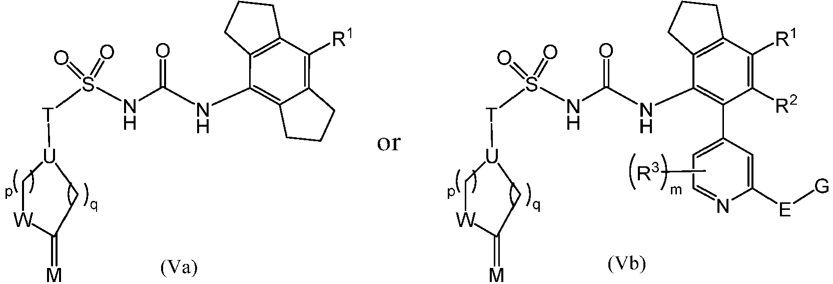
T is selected from chemical bond, -NR⁵-, aryl, and heteroaryl;
M, U, W, p, and q are as described as above;
R¹, R², R³, and m are as described as above; and
E, G, and R⁵ are as described as above.

In another preferred embodiment, the formula(I) has the structure of formula (VIa) or formula (VIb): 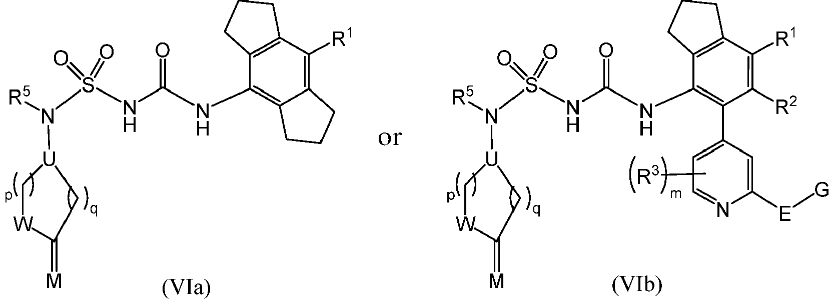
R⁵ is selected from hydrogen and C₁₋₄alkyl;
U is selected from CR¹²; wherein, R¹² is selected from hydrogen, and C₁₋₄alkyl;
M, W, p, and q are as described as above;
R¹, R², R³, and m are as described as above; and
E, and G are as described as above.

In another preferred embodiment, the formula(I) has the structure of formula (VIIa) or formula (VIIb): 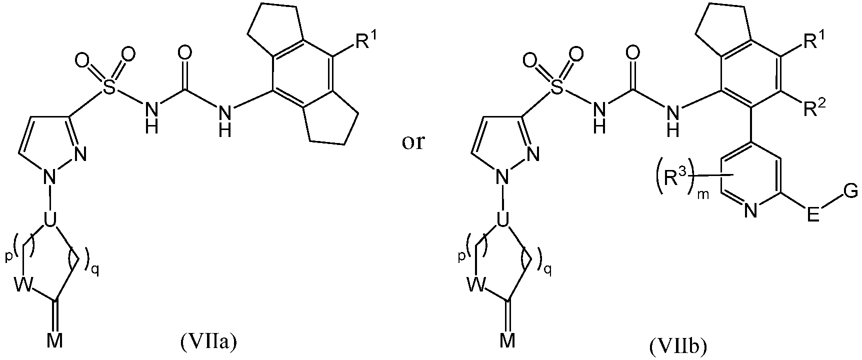
U is selected from CR¹²; wherein, R¹² is selected from hydrogen, and C₁₋₄alkyl;
M, W, p, and q are as described as above;
R¹, R², R³, and m are as described as above; and
E, and G are as described as above.

In another preferred embodiment, the formula(I) has the structure of formula (VIIIa) or formula (VIIIb): 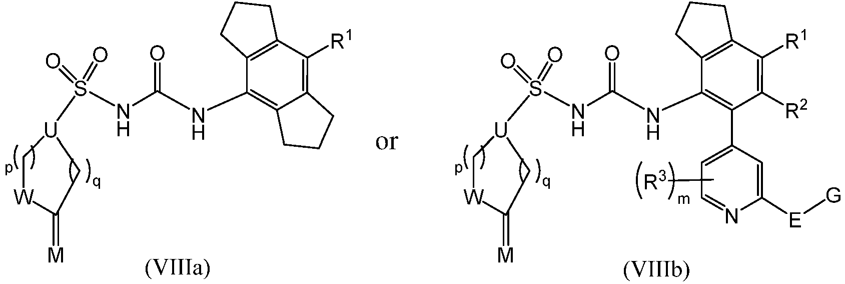 M, U, W, R¹, R², R³, E, G, m, p, and q are as described as above.

In another preferred embodiment, the formula(I) has the structure of formula (IXa) or formula (IXb): 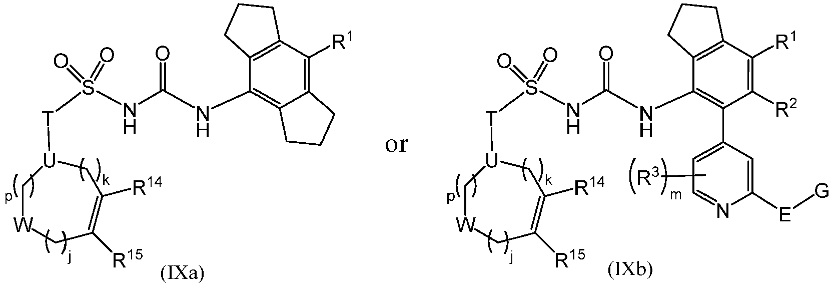
k and j are independently 0, 1 or 2;
R¹⁴ and R¹⁵ are independently selected from the group consisting of H, halogen, C₁₋₄ alkyl, and C₃₋₆ cycloalkyl; and
T, U, W, R¹, R², R³, E, G, m, and p are as described as above.

In another preferred embodiment, the formula(I) has the structure of formula (X): 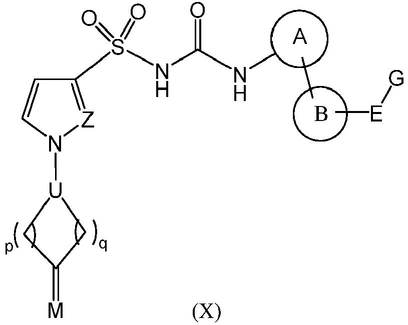
Z is selected from N and CR¹⁶; wherein, R¹⁶ is selected from hydrogen, halogen and C₁₋₄alkyl;
p and q are independently selected from 0, 1, 2, 3, 4, 5, and 6;
U is selected from CR¹²; wherein, R¹² is selected from hydrogen, and C₁₋₄ alkyl;
M is selected from CR¹⁰R¹¹; wherein, R¹⁰ and R¹¹ are as described as above; and
A, B, E and G are as described as above.

In another preferred embodiment, the formula(I) has the structure of formula (XIa) or formula (XIb): 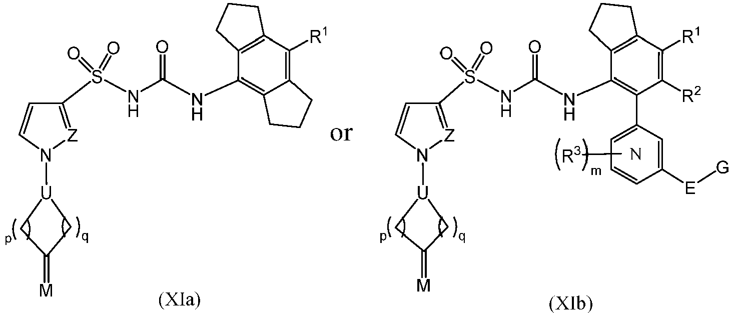 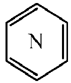 is selected from pyridine, pyrimidine, and pyridazine;
Z, U, M, p, and q are as described as above;
R¹, R², R³, and m are as described as above; and
E, and G are as described as above.

In another preferred embodiment, the formula(I) has the structure of formula (XII): 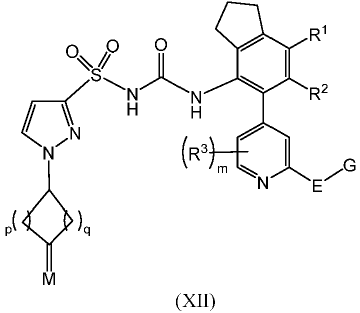
p and q are independently selected from0, 1, 2, 3, 4, 5, and 6;
M is selected from CR¹⁰R¹¹; wherein, R¹⁰ and R¹¹ are independently selected from the group consisting of hydrogen, fluorine, and C₁₋₂ alkyl; wherein, the alkyl is optionally substituted by one or more substituents selected from the group consisting of hydrogen, halogen, C₁₋₄ alkyl, C₁₋₄haloalkyl, C₁₋₄alkoxy, C₁₋₄ haloalkoxy, NR⁸R⁹, C₃₋₈cycloalkyl, and 3-8 membered heterocyclyl;
R¹ and R² are independently selected from hydrogen, halogen, C₁₋₄ alkyl, C₁₋₄haloalkyl, C₁₋₄alkoxy, and C₁₋₄haloalkoxy;
each R³ is independently selected from hydrogen, halogen, and C₁₋₄alkyl;
m is 0, 1 or 2;
E is selected from the group consisting of chemical bond, -O-, -O(CR^{a}R^{b})₁₋₂-, -(CR^{a}R^{b})₁₋₂O-, - S-, -NR⁵-, - (CR^{a}R^{b})₁₋₂NR⁵-, - NR⁵(CR^{a}R^{b})₁₋₂-, C₁₋₂alkylene, -C=C-, and C₃₋₆cycloalkyl; wherein, R^{a} and R^{b} are independently selected from group consisting hydrogen and C₁₋₄ alkyl; R⁵ is selected from the group consisting of H, C₁₋₄ alkyl, and C₃₋₆cycloalkyl;
G is selected from the group consisting of hydrogen, C₁₋₄ alkyl, C₁₋₄haloalkyl, C₂₋₄ alkenyl, C₂₋₄alkynyl, saturated C₃₋₈cycloalkyl, unsaturated C₃₋₈cycloalkyl, saturated 3-12 membered heterocyclyl, unsaturated 3-12 membered heterocyclyl, aryl, heteroaryl, and NR⁸R⁹; wherein, the cycloalkyl, heterocyclyl, aryl, or heteroaryl are optionally substituted by one or more substituents selected from the group consisting of halogen, C₁₋₄ alkyl, C₁₋₄haloalkyl, C₁₋₄alkoxy, C₁₋₄haloalkoxy, NR⁸R⁹, CN, C(O)R^{t}, and S(O)₂R^{t}; wherein, R^{t} is C₁₋₄ alkyl, C₂₋₄ alkenyl, C₂₋₄alkynyl, C₃₋₈cycloalkyl, 4-8-membered heterocyclyl, aryl, or heteroaryl;
R⁸ and R⁹ are independently selected from the group consisting of H, C₁₋₄ alkyl, C₃₋₆cycloalkyl, and 4-8 membered heterocyclyl;

In another preferred embodiment, the compound of formula(I) is selected from group consisting of 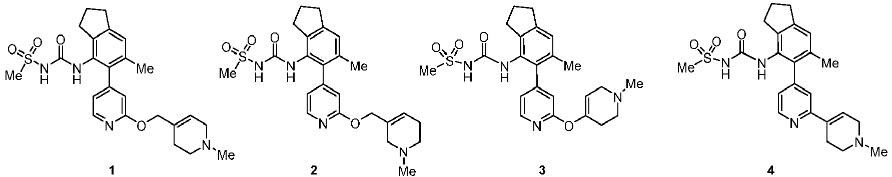 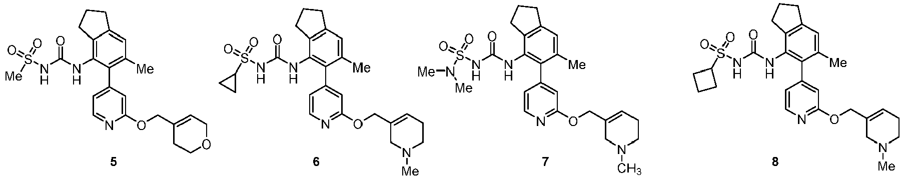 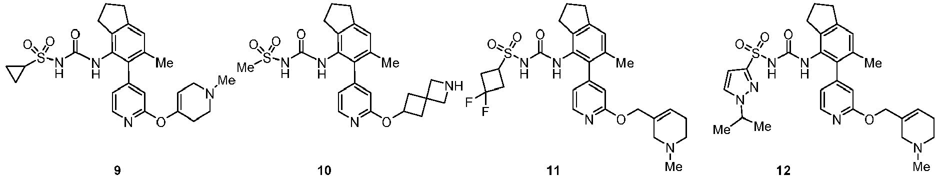 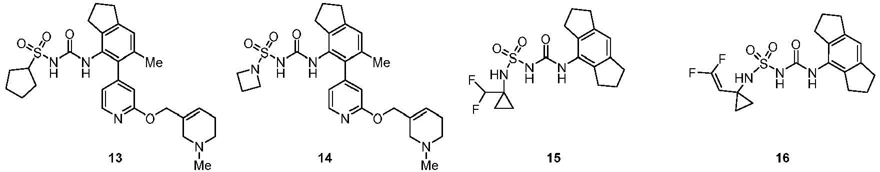 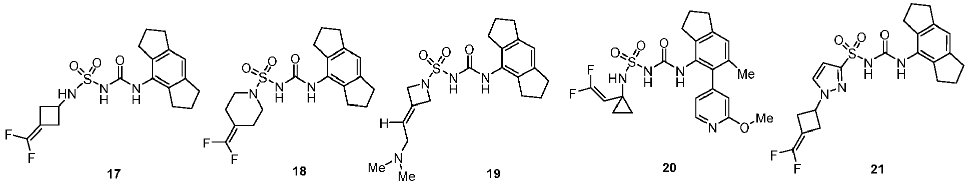 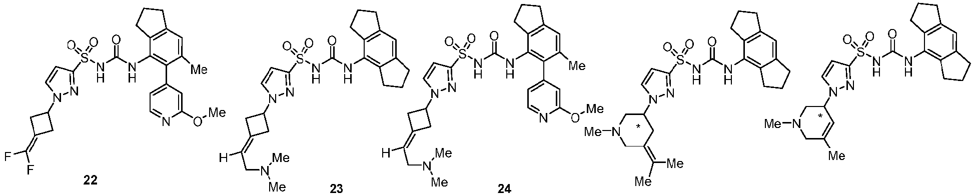 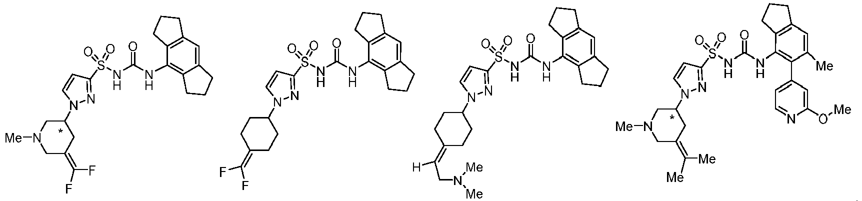 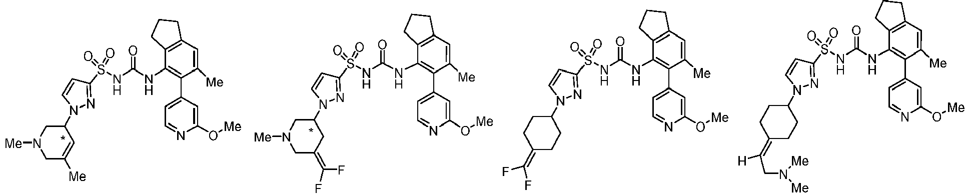 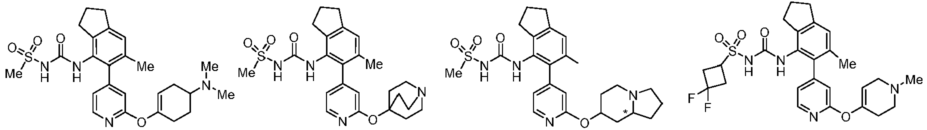 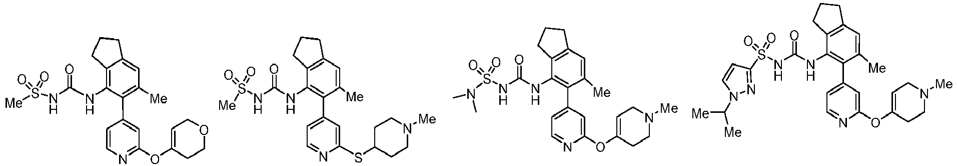 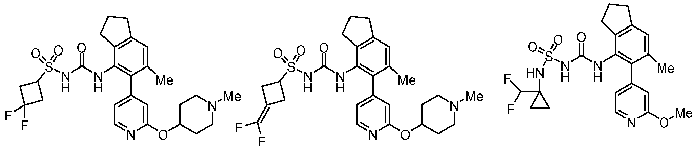 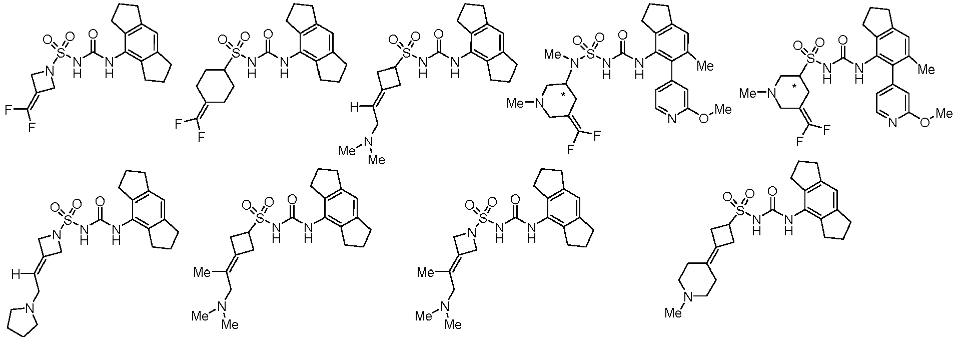 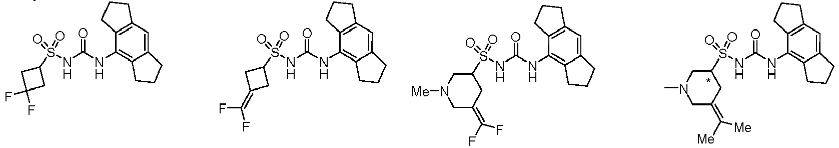 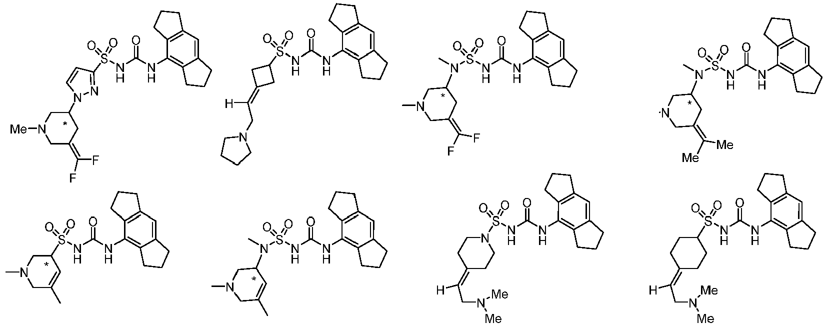 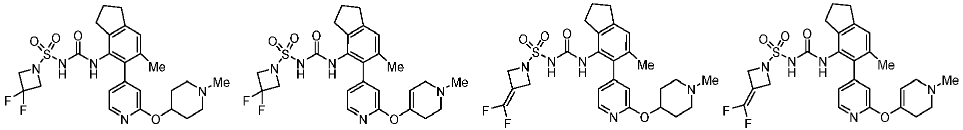 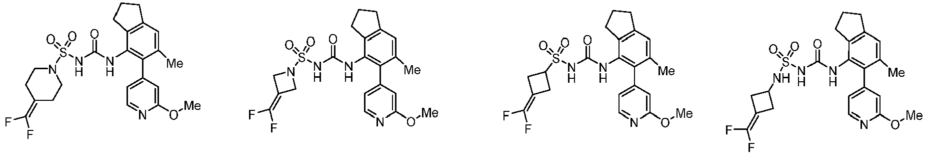 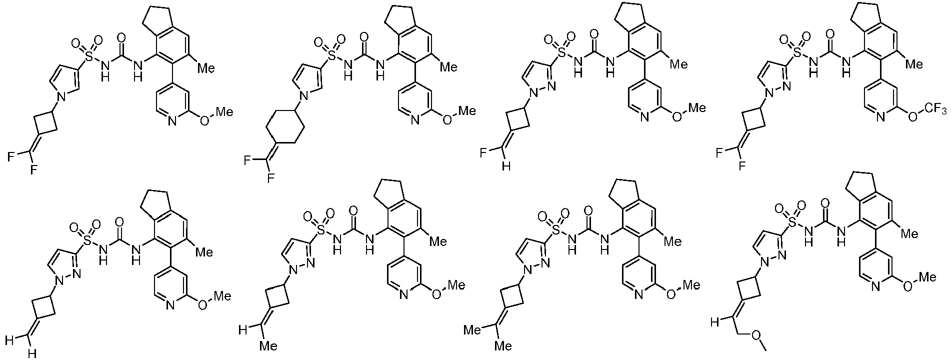 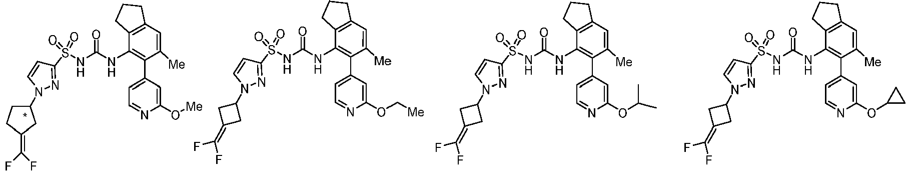 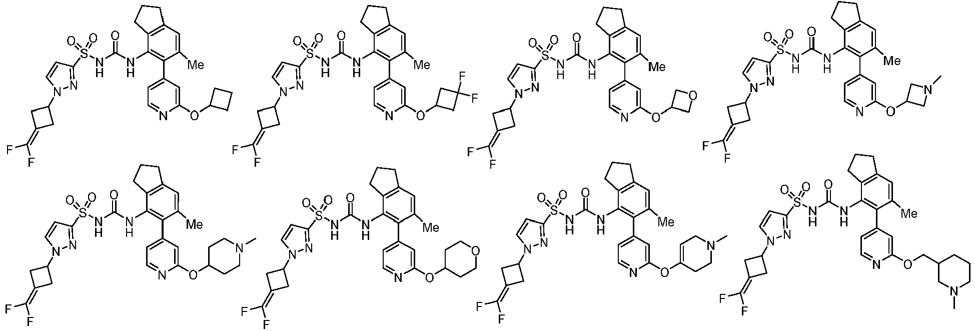 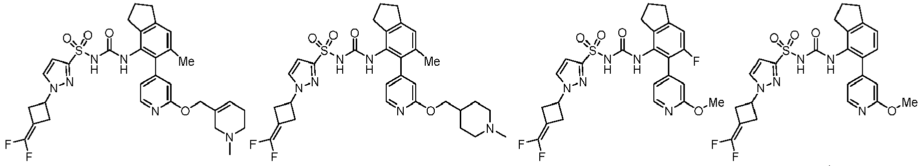 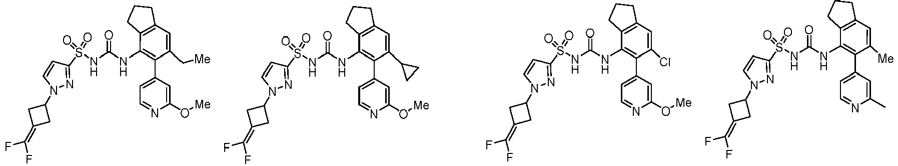 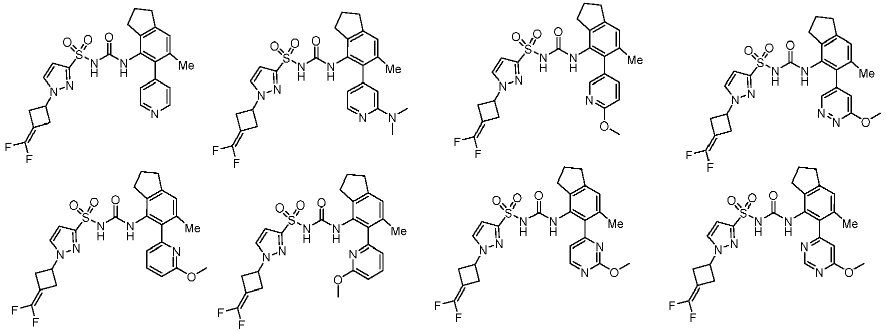 "*"represents chiral center.

In another preferred embodiment, the pharmaceutically acceptable salt is alkali metal salts, preferably, is the salt selected from the group consisting of sodium salt, potassium salt, and lithium salt.

In the second aspect of the present invention, a pharmaceutical composition is provided, comprising a compound of the first aspect of the present invention, or optical isomers, pharmaceutically acceptable salts, prodrugs, deuterated derivatives, hydrates, or solvates thereof, and pharmaceutically acceptable carriers.

In the third aspect of the present invention, the use of a compound of the first aspect of the present invention, or optical isomers, pharmaceutically acceptable salts, prodrugs, deuterated derivatives, hydrates, or solvates thereof is provided, wherein in the preparation of a pharmaceutical composition for the treatment of diseases, disorders or symptoms associated with NLRP3 activity or expression.

In another preferred embodiment, the disease, disorder or symptom is selected from the group consisting of inflammation, autoimmune disease, knee osteoarthritis, cancer, infection, central nervous system disease, metabolic disease, cardiovascular disease, respiratory disease, liver disease, kidney disease, eye disease, skin disease, lymphatic condition, psychological disorder, graft versus host disease, allodynia, cryoglobulin-associated periodic syndrome (CAPS), Muckle-Wells syndrome (MWS), familial cold autoinflammatory syndrome (FCAS), neonatal onset of multisystem inflammatory disease (NOMID), familial mediterranean fever (FMF), septic arthritis, pyoderma gangrenosum and acne syndrome (PAPA), hyperimmunoglobulinemia D syndrome (HIDS), tumor necrosis factor (TNF) receptor -associated periodic syndrome (TRAPS), systemic juvenile idiopathic arthritis, adult onset Still's disease (AOSD), relapsing polychondritis, schnitzler's syndrome, angelman syndrome, behcet's disease, anti synthetase syndrome, deficiency of interleukin-1 receptor antagonist (DIRA) and haploid deficiency of A2o (HA2o).

### Detail Implementation

After long-term and intensive research, the present inventors have unexpectedly discovered a novel class of tricyclic aryl containing compounds as NLRP3 inhibitors, as well as their preparation methods and applications. The compounds of the invention may be applied to the treatment of various diseases associated with the activity of said kinases. Based on the above findings, the inventors completed the present invention.

### Terminology

Unless otherwise stated, "or" as used herein has the same meaning as "and/or" (refers to "or" and "and").

Unless otherwise specified, among all compounds of the present invention, each chiral carbon atom (chiral center) may optionally be in the R configuration or the S configuration, or a mixture of the R configuration and the S configuration.

As used herein, the term "alkyl", alone or as part of another substituent, refers to a straight (ie, unbranched) or branched saturated hydrocarbon group containing only carbon atoms, or a combination of straight and branched chains. When the alkyl group has a carbon number limitation (e.g., C₁₋₁₀), it means that the alkyl group has 1 to 10 carbon atoms. For example, C₁₋₈ alkyl refers to an alkyl group containing from 1 to 8 carbon atoms, including methyl, ethyl, propyl, isopropyl, butyl, isobutyl, sec-butyl, tert-butyl, or the like.

As used herein, the term "alkenyl", when used alone or as part of another substituent, refers to a straight or branched, carbon chain group having at least one carbon-carbon double bond. Alkenyl groups can be substituted or unsubstituted. When the alkenyl group has a carbon number limit (e.g., C₂₋₈), it means that the alkenyl group has 2-8 carbon atoms. For example, C₂₋₈ alkenyl refers to alkenyl groups having 2-8 carbon atoms, including ethenyl, propenyl, 1,2-butenyl, 2,3-butenyl, butadienyl, or the like.

As used herein, the term "alkynyl", when used alone or as part of another substituent, refers to an aliphatic hydrocarbon group having at least one carbon-carbon triple bond. The alkynyl group can be straight or branched, or a combination thereof. When the alkynyl group has a carbon number limitation (e.g., C₂₋₈ alkynyl group), it means that the alkynyl group has 2 to 8 carbon atoms. For example, the term "C₂₋₈ alkynyl" refers to a straight or branched alkynyl group having 2-8 carbon atoms, including ethynyl, propynyl, isopropynyl, butynyl, isobutynyl, secondary Butynyl, tert-butynyl, or the like.

As used herein, when used alone or as part of another substituent, the term "cycloalkyl" refers to a unit ring having a saturated or partially saturated ring, a bicyclic or polycyclic (fused ring, bridged or spiro) ring system.. When a certain cycloalkyl group has a carbon number limitation (e.g., C₃₋₁₀), it means that the cycloalkyl group has 3 to 10 carbon atoms. In some preferred embodiments, the term "C3-8 cycloalkyl" refers to a saturated or partially saturated monocyclic or bicyclic alkyl group having from 3 to 8 carbon atoms, including cyclopropyl, cyclobutyl, cyclopentyl. , cycloheptyl, or the like. "Spirocycloalkyl" refers to a bicyclic or polycyclic group that shares a carbon atom (called a spiro atom) between the monocyclic rings. These may contain one or more double bonds, but none of the rings have fully conjugated π electrons. system.

"Fused cycloalkyl" means an all-carbon bicyclic or polycyclic group in which each ring of the system shares an adjacent pair of carbon atoms with other rings in the system, wherein one or more of the rings may contain one or more Key, but none of the rings have a fully conjugated π-electron system. "Bridge cycloalkyl" refers to an all-carbon polycyclic group in which two rings share two carbon atoms that are not directly bonded, which may contain one or more double bonds, but none of the rings have a fully conjugated pi-electron system . The atoms contained in the cycloalkyl group are all carbon atoms. Some examples of cycloalkyl groups are as follows, and the present invention is not limited to the following cycloalkyl groups.

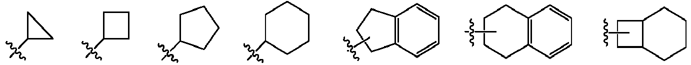

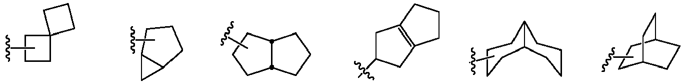

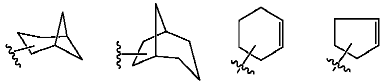

Unless otherwise stated, the following terms used in the specification and claims have the following meanings. "Aryl" means an all-carbon monocyclic or fused polycyclic (ie, a ring that shares a pair of adjacent carbon atoms) groups having a conjugated π-electron system, such as phenyl and naphthyl. The aryl ring may be fused to other cyclic groups (including saturated and unsaturated rings), but may not contain heteroatoms such as nitrogen, oxygen, or sulfur, while the point of attachment to the parent must be on the carbon atoms of the ring in a conjugated pi-electron system. The aryl group can be substituted or unsubstituted. The following are some examples of aryl groups, and the present invention is not limited to the aryl groups described below.

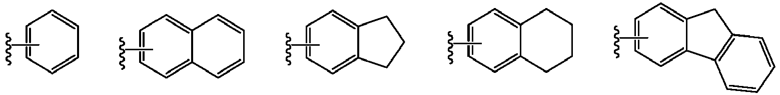

"Heteroaryl" means an aromatic monocyclic or polycyclic group comprising one to more heteroatoms selected from nitrogen, oxygen and sulfur, or a polycyclic group comprising a heterocyclic group comprising one to more heteroatoms selected from nitrogen, oxygen and sulfur in combination with an aryl group, where the linkage site is located on the aryl group.The heteroaryl group can be optionally substituted or unsubstituted. The following are some examples of heteroaryl groups, and the present invention is not limited to the following heteroaryl groups.

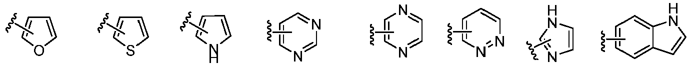

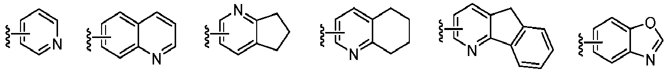

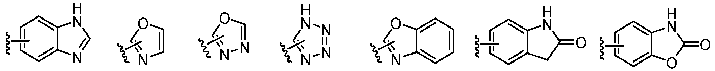

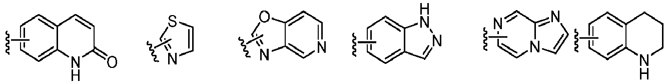

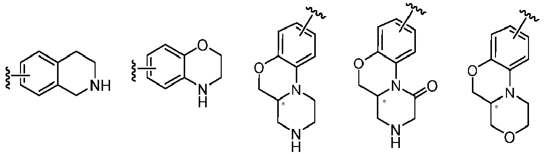

"Heterocyclyl" means a saturated or partially unsaturated monocyclic or polycyclic cyclic hydrocarbon substituent wherein one or more of the ring atoms are selected from nitrogen, oxygen or sulfur and the remaining ring atoms are carbon. Non-limiting examples of monocyclic heterocyclic groups include pyrrolidinyl, piperidinyl, piperazinyl, morpholinyl, thiomorpholinyl, homopiperazinyl. Polycyclic heterocyclic group refers to a heterocyclic group including a spiro ring, a fused ring, and a bridged ring. "Spirocyclic heterocyclyl" refers to a polycyclic heterocyclic group in which each ring of the system shares an atom (referred to as a spiro atom) with other rings in the system, wherein one or more of the ring atoms is selected from the group consisting of nitrogen and oxygen. Or sulfur, the remaining ring atoms are carbon. "Fused ring heterocyclyl" refers to a polycyclic heterocyclic group in which each ring of the system shares an adjacent pair of atoms with other rings in the system, and one or more rings may contain one or more double bonds, but none One ring has a fully conjugated pi-electron system, and wherein one or more ring atoms are selected from nitrogen, oxygen or sulfur, and the remaining ring atoms are carbon. "Bridged heterocyclyl" refers to a polycyclic heterocyclic group in which any two rings share two atoms which are not directly bonded, these may contain one or more double bonds, but none of the rings have a fully conjugated pi-electron system, and wherein one or more of the ring atoms are selected from nitrogen, oxygen or sulfur, and the remaining ring atoms are carbon. If a heterocyclic group has both a saturated ring and an aromatic ring (for example, the saturated ring and the aromatic ring are fused together), the point attached to the parent must be on the saturated ring. Note: When the point attached to the parent is on the aromatic ring, it is called a heteroaryl group and is not called a heterocyclic group. Some examples of the heterocyclic group are as follows, and the present invention is not limited to the following heterocyclic group.

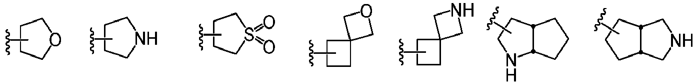

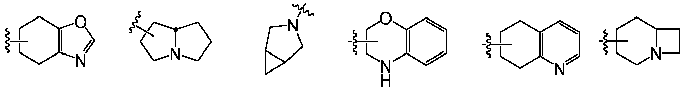

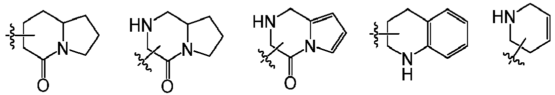

As used herein, the term "halogen", when used alone or as part of another substituent, refers to F, Cl, Br, and I.

As used herein, the term "substituted" (when with or without "optionally") means that one or more hydrogen atoms on a particular group are replaced by a particular substituent. Particular substituents are the substituents described above in the corresponding paragraphs, or the substituents which appear in the examples. Unless otherwise stated, an optionally substituted group may have a substituent selected from a particular group at any substitutable position of the group, and the substituents may be the same or different at each position. A cyclic substituent, such as a heterocyclic group, may be attached to another ring, such as a cycloalkyl group, to form a spirobicyclic ring system, i.e., the two rings have a common carbon atom. Those skilled in the art will appreciate that the combinations of substituents contemplated by the present invention are those that are stable or chemically achievable. The substituents are, for example but not limited to, C₁₋₈ alkyl, C₂₋₈ alkenyl, C₂₋₈ alkynyl, C₃₋₈ cycloalkyl, 3- to 12-membered heterocyclic, aryl, Heteroaryl, halogen, hydroxy, carboxy (-COOH), C₁₋₈ aldehyde, C₂₋₁₀ acyl, C₂₋₁₀ ester, amino.

For convenience and in accordance with conventional understanding, the term "optionally substituted" or "optionally substituted" applies only to sites which are capable of being substituted by a substituent, and does not include those which are not chemically achievable.

As used herein, unless otherwise specified, the term "pharmaceutically acceptable salt" refers to a salt that is suitable for contact with the tissue of a subject (eg, a human) without causing unpleasant side effects. In some embodiments, a pharmaceutically acceptable salt of a compound of the invention includes a salt (eg, a potassium salt, a sodium salt, a magnesium salt, a calcium salt) of a compound of the invention having an acidic group or is basic A salt of a compound of the invention (e.g., a sulfate, a hydrochloride, a phosphate, a nitrate, a carbonate).

### Utility:

The present invention provides a compound of the formula (I) or formula (II), or a deuterated derivative thereof, a salt thereof, an isomer (enantiomer or diastereomer, if present In the case of a hydrate, a pharmaceutically acceptable carrier or excipient for inhibiting NLRP3.

The compounds of the invention are useful as NLRP3 inhibitors.

The present invention is a single inhibitor of NLRP3 for the purpose of preventing, alleviating or curing disease by modulating the enzymatic activity of NLRP3. The referred diseases include Cryopyrin Protein-Associated Periodic Syndrome (CAPS), which is primarily due to acquired mutations in NLRP3, and encompasses types such as Muckle - Wells Syndrome (MWS), Familial Cold Autoinflammatory Syndrome (FCAS), and Neonatal Onset Multi-System Inflammatory Disease.

In addition, the diseases referred to include the following categories:
Genetic disorders, such as sickle cell anemia and valine-containing tyrosine peptide protein disorders.
Autoimmune diseases, including, but not limited to, rheumatoid arthritis, knee osteoarthritis, septic arthritis, gangrenous pyoderma, acute febrile neutrophilic dermatosis, chronic non-infectious myelitis, systemic lupus erythematosus, inflammatory bowel disease (ulcerative colitis and Crohn's disease), Behcetts Syndrome, Schnitzler's Syndrome, Familial Mediterranean Fever, Tumor Necrosis Factor Receptor-Associated Cyclic Fever , hyper IgD syndrome and macrophage activation syndrome;
Central nervous system disorders, including but not limited to Alzheimer's disease, Parkinson's disease, multiple sclerosis, dementia, etc;
Metabolism-related diseases, including but not limited to type 1 diabetes, type 2 diabetes, obesity, gout, pseudogout, atherosclerosis, and metabolic syndrome
Lung diseases, including but not limited to asthma, pulmonary fibrosis, idiopathic pulmonary fibrosis, pulmonary ischemia-reperfusion injury, chronic obstructive disease, lithiasis, and silicosis
Diseases of the eye, including, but not limited to, age-related macular degeneration, diabetic retinopathy, and optic nerve damage
Liver disease, including, but not limited to, nonalcoholic fatty liver disease, hepatic ischemia-reperfusion injury, fulminant hepatitis, hepatic fibrosis, and hepatic failure Kidney disease, including but not limited to renal calcium deposits and renal fibrosis
Heart disease, including but not limited to cardiac hypertrophy and fibrosis, heart failure, aortic aneurysm with dissection, cardiac injury due to metabolic disorders, atrial fibrillation, and high blood pressure
Skin disorders including, but not limited to, psoriasis, atopic dermatitis, contact allergic reactions, hidradenitis suppurativa, acne vulgaris, and nodular disease;

In addition, the types of diseases that can be alleviated or cured by inhibiting NLRP3 enzyme activity include inflammatory nociceptive hypersensitivity, neuralgia, and control of infections from a wide range of bacteria, viruses, fungi, and worms.

NLRP3 is also involved in the development of a variety of cancers, including myelofibrosis, B-cell lymphoma, monocytic leukemia, splenomegaly, eosinophilic leukocytosis syndrome, primary thrombocytopenia, systemic giant cell disease, hepatocellular carcinoma, rectal carcinoma, bladder carcinoma, pharyngeal carcinoma, non-small-cell lung carcinoma, small-cell lung carcinoma, adenocarcinoma of the lung, squamous lung carcinoma, breast carcinoma, prostate carcinoma, glioma, and ovarian carcinoma. glioblastoma, ovarian cancer, head and neck squamous cancer, cervical cancer, esophageal cancer, renal cancer, pancreatic cancer, colon cancer, skin cancer, lymphoma, gastric cancer, multiple myeloma, and many other solid tumors and hematological tumors.

The compound of the present invention and its deuterated derivatives, and pharmaceutically acceptable salts or isomers thereof, if present, or hydrates and/or compositions thereof, and pharmaceutically acceptable excipients or The carriers are formulated together and the resulting compositions are administered to mammals, such as men, women and animals, for the treatment of conditions, conditions and diseases. The composition may be: a tablet, a pill, a suspension, a solution, an emulsion, a capsule, an aerosol, a sterile injectable solution, sterile powder, etc. In some embodiments, pharmaceutically acceptable excipients include microcrystalline cellulose, lactose, sodium citrate, calcium carbonate, calcium hydrogen phosphate, mannitol, hydroxypropyl-beta-cyclodextrin, beta-cyclodextrin. (increased), glycine, disintegrants (such as starch, croscarmellose sodium, complex silicates and high molecular weight polyethylene glycols), granulating binders (such as polyvinylpyrrolidone, sucrose, gelatin and Acacia gum) and lubricants (such as magnesium stearate, glycerin and talc). In a preferred embodiment, the pharmaceutical composition is a dosage form suitable for oral administration, including but not limited to tablets, solutions, suspensions, capsules, granules, powders. The amount of the compound or pharmaceutical composition of the invention administered to a patient is not fixed and is usually administered in a pharmaceutically effective amount. At the same time, the amount of the compound actually administered can be determined by the physician based on the actual conditions, including the condition being treated, the route of administration selected, the actual compound administered, the individual condition of the patient, and the like. The dosage of the compound of the invention depends on the particular use of the treatment, the mode of administration, the condition of the patient, and the judgment of the physician. The proportion or concentration of a compound of the invention in a pharmaceutical composition depends on a variety of factors including dosage, physicochemical properties, route of administration, and the like.

It is to be understood that within the scope of the present invention, the various technical features of the present invention and the various technical features specifically described hereinafter (as in the embodiments) may be combined with each other to constitute a new or preferred technical solution.

### General synthetic schemes

The compound of formula I of the present invention can be prepared by the following method:

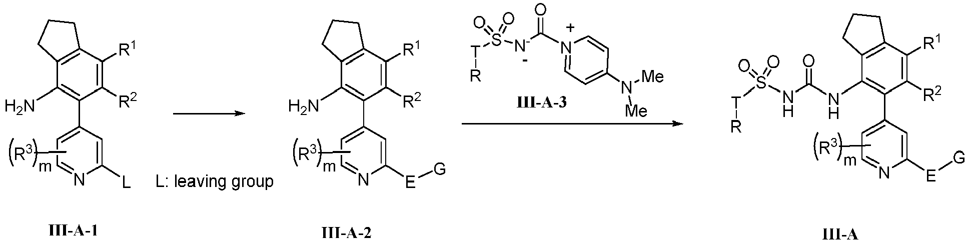

In the above formulas, the expressions of m, E, G, R1, R2, and R3 are the same as those in formula III above. The expressions of T and R are the same as those of Formula I of above.

In an inert solvent, substitution reaction takes place to obtain the intermediate III-A-2 with III-A-1 as the starting material, which then reacts with the reactive compound III-A-3 to obtain the target compound III-A.

Compounds of formula I-A of the present invention can be prepared by the following methods:

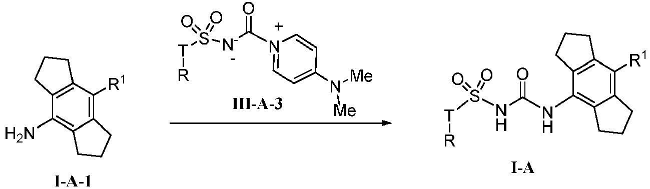

In the above formulas, the expressions of R¹ are the same as R¹ described in formula IIa above. The expressions of T and R are the same as T and R described in formula I above.

In an inert solvent, substitution reaction takes place to obtain the target compound I-A, which employed the the starting material I-A-1 to react with compound III-A-3.

Compounds of formula XII-A of the present invention can be prepared by the following methods:

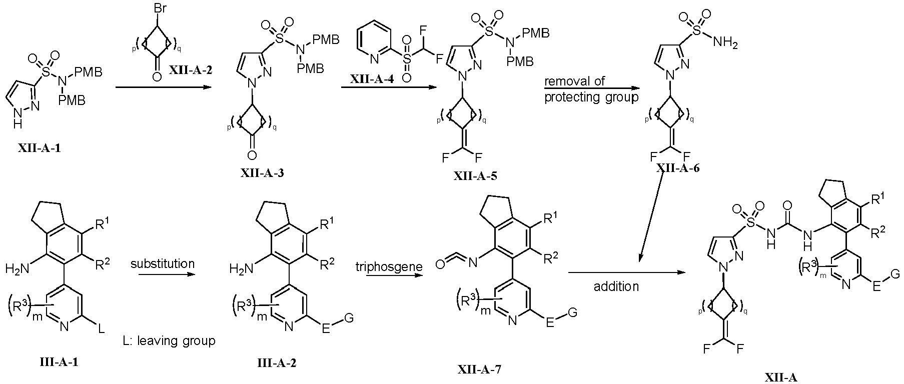

In the above formulas, the expressions of p, q, m, E, G, R1, R2 and R3 are the same as the description in formula XII above.

In an inert solvent, substitution reaction takes placebetween the starting material XII-A-1 and XII-A-2 to obtain compound XII-A-3, which then reacts with reagent XII-A-4 to get the di-F compounds XII-A-5. Then intermediate XII-A-6 is obtained by removing the protecting group under acidic conditions. In an inert solvent, substitution reaction takes place to obtain the intermediate III-A-2 that employed III-A-1 as the starting material, which then reacts with triphosgene to get the isocyanate compounds XII-A-7. The target compound XII-A was obtained through addition reaction with XII-A-6.

Compounds of formula XII-B of the present invention can be prepared by the following methods:

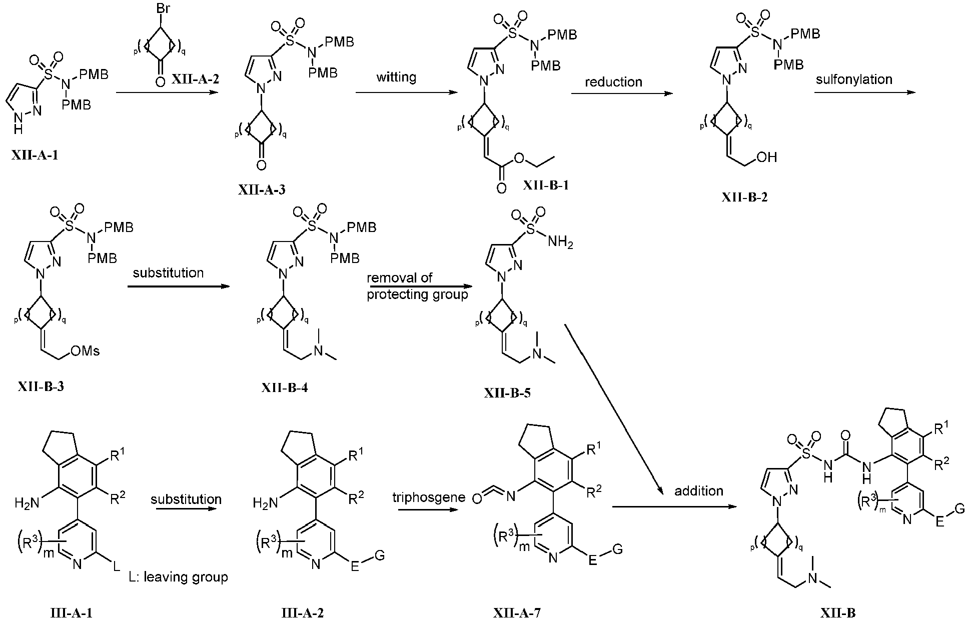

In the above formulas, the expressions of p, q, m, E, G, R1, R2 and R3 are the same as the description in formula XII above.

In an inert solvent, substitution reaction takes place to obtain compound XII-A-3 between the starting material XII-A-1 and XII-A-2, and then witting reaction is performed to get XII-B-1. Reduction of ester group gives hydroxy compound XII-B-2. The reactive ester compounds XII-B-3 are obtained through sulfonylation, which then react with amines to afford the compound XII-B-4. Then intermediate XII-B-5 is obtained by removing the protecting group. In an inert solvent, substitution reaction takes place to obtain the intermediate III-A-2 that employed III-A-1 as the starting material, which then reacts with triphosgene to get the isocyanate compounds XII-A-7. The target compound XII-B was obtained through reaction of XII-A-7 with XII-B-5.

### Pharmaceutical composition and method of administration

Since the compound of the present invention has excellent inhibitory activity against a series of protein kinases, the compound of the present invention and various crystal forms thereof, pharmaceutically acceptable inorganic or organic salts, hydrates or solvates, and compounds containing the present invention are The pharmaceutical composition of the main active ingredient can be used to treat, prevent, and alleviate diseases associated with EGFR, FAK, SYK, FLT-3, Axl, CDK, JAK activity or expression levels.

The pharmaceutical compositions of the present invention comprise a safe or effective amount of a compound of the present invention, or a pharmaceutically acceptable salt thereof, and a pharmaceutically acceptable excipient or carrier. By "safe and effective amount" it is meant that the amount of the compound is sufficient to significantly improve the condition without causing serious side effects. In general, the pharmaceutical compositions contain from 1 to 2000 mg of the compound of the invention per agent, more preferably from 5 to 200 mg of the compound of the invention per agent. Preferably, the "one dose" is a capsule or tablet.

"Pharmaceutically acceptable carrier" means: one or more compatible solid or liquid fillers or gel materials which are suitable for human use and which must be of sufficient purity and of sufficiently low toxicity. By "compatibility" it is meant herein that the components of the composition are capable of intermingling with the compounds of the invention and with each other without significantly reducing the efficacy of the compound. Examples of pharmaceutically acceptable carriers are cellulose and its derivatives (such as sodium carboxymethylcellulose, sodium ethylcellulose, cellulose acetate, etc.), gelatin, talc, solid lubricants (such as stearic acid). , magnesium stearate), calcium sulfate, vegetable oils (such as soybean oil, sesame oil, peanut oil, olive oil, etc.), polyols (such as propylene glycol, glycerin, mannitol, sorbitol, etc.), emulsifiers (such as Tween^{®}), run Wet agents (such as sodium lauryl sulfate), colorants, flavoring agents, stabilizers, antioxidants, preservatives, pyrogen-free water, and the like.

The mode of administration of the compound or pharmaceutical composition of the present invention is not particularly limited, and representative modes of administration include, but are not limited to, oral, intratumoral, rectal, parenteral (intravenous, intramuscular or subcutaneous), and topical administration.

Solid dosage forms for oral administration include capsules, tablets, pills, powders, and granules. In these solid dosage forms, the active compound is mixed with at least one conventional inert excipient (or carrier), such as sodium citrate or dicalcium phosphate, or mixed with: (a) a filler or compatibilizer, for example, Starch, lactose, sucrose, glucose, mannitol and silicic acid; (b) a binder such as hydroxymethylcellulose, alginate, gelatin, polyvinylpyrrolidone, sucrose, and acacia; (c) a humectant such as glycerin; (d) a disintegrant, for example, Agar, calcium carbonate, potato starch or tapioca starch, alginic acid, certain complex silicates, and sodium carbonate; (e) a slow solvent such as paraffin; (f) an absorption accelerator, for example, a quaternary amine compound; (g) Wetting agents such as cetyl alcohol and glyceryl monostearate; (h) an adsorbent, for example, kaolin; and (i) a lubricant such as talc, calcium stearate, magnesium stearate, solid polyethylene glycol, sodium lauryl sulfate, or a mixture thereof. In capsules, tablets and pills, the dosage form may also contain a buffer.

Solid dosage forms such as tablets, dragees, capsules, pills, and granules can be prepared with coatings and shells such as enteric coatings and other materials known in the art. They may contain opacifying agents and the release of the active compound or compound in such compositions may be released in a portion of the digestive tract in a delayed manner. Examples of embedding components that can be employed are polymeric and waxy materials. If necessary, the active compound may also be in microencapsulated form with one or more of the above-mentioned excipients.

Liquid dosage forms for oral administration include pharmaceutically acceptable emulsions, solutions, suspensions, syrups or elixirs. In addition to the active compound, the liquid dosage form may contain inert diluents conventionally employed in the art, such as water or other solvents, solubilizers and emulsifiers, for example, ethanol, isopropanol, ethyl carbonate, ethyl acetate, propylene glycol, 1 , 3-butanediol, dimethylformamide and oils, especially cottonseed oil, peanut oil, corn germ oil, olive oil, castor oil and sesame oil or a mixture of these substances.

In addition to these inert diluents, the compositions may contain adjuvants such as wetting agents, emulsifying and suspending agents, sweetening agents, flavoring agents and perfumes.

In addition to the active compound, the suspension may contain suspending agents, for example, ethoxylated isostearyl alcohol, polyoxyethylene sorbitol and sorbitan esters, microcrystalline cellulose, aluminum methoxide and agar or mixtures of these and the like.

Compositions for parenteral injection may comprise a physiologically acceptable sterile aqueous or nonaqueous solution, dispersion, suspension or emulsion, and a sterile powder for reconstitution into a sterile injectable solution or dispersion. Suitable aqueous and nonaqueous vehicles, diluents, solvents or vehicles include water, ethanol, polyols, and suitable mixtures thereof.

Dosage forms for the compounds of the invention for topical administration include ointments, powders, patches, propellants and inhalants. The active ingredient is admixed under sterile conditions with a physiologically acceptable carrier and any preservatives, buffers, or, if necessary, propellants.

The compounds of the invention may be administered alone or in combination with other pharmaceutically acceptable compounds.

When a pharmaceutical composition is used, a safe and effective amount of a compound of the invention is administered to a mammal (e.g., a human) in need of treatment wherein the dosage is a pharmaceutically effective effective dosage, for a 60 kg body weight, The dose to be administered is usually from 1 to 2000 mg, preferably from 5 to 500 mg. Of course, specific doses should also consider factors such as the route of administration, the health of the patient, etc., which are within the skill of the skilled physician.

### The main advantages of the invention include:

1. A compound of formula I is provided.
2. Provided is a novel inhibitor of activity of NLRP3, and preparation and application thereof, wherein the inhibitor can inhibit the above NLRP3 activity at a very low concentration.
3. A class of pharmaceutical compositions for treating diseases associated with NLRP3 activity is provided.
4. Provides a well-absorbed NLRP3 inhibitor for oral administration.

The invention is further illustrated below in conjunction with specific embodiments. It is to be understood that the examples are not intended to limit the scope of the invention. The experimental methods in the following examples which do not specify the specific conditions are usually in accordance with conventional conditions or according to the conditions recommended by the manufacturer. Percentages and parts are by weight unless otherwise stated.

### Example 1: Preparation of Compound 1

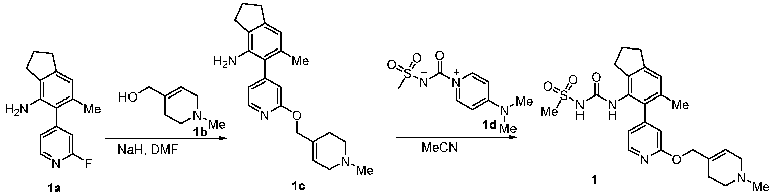

### Compound 1a and 1d were synthesized according to WO2020035465

To a solution of 1b (18 mg, 0.14 mmol) in DMF (1.5 mL) was added NaH (6 mg, 0.14 mmol) at 0 at. The reaction mixture was stirred 1 h under N₂. Then a solution of **1a** (14 mg, 0.06 mmol) in DMF (0.8 mL) was added. The reaction was stirred at 60 °C for about 2 h. The mixture was cooled to RT (room temperature) and quenched with water. Then the mixture was extracted with EA (ethyl acetate) (3×5 mL), and the collected organic phase was washed with sat.NaCl, dried with anhydrous sodium sulfate, filterated and concentrated to give the crude product **1c.** The crude product was used to the next step without further purification. LCMS: m/z 350.5 [M+H]⁺.

The crude product **1c** (20 mg, 0.06 mmol) and **1d** (14 mg, 0.06 mmol) was dissolved in MeCN (acetonitrile) (2.5 mL). The mixture was stirred at 60 °C for 1 h. The mixture was cooled to RT and concentrated to give the crude. The crude was purifired by Pre-TLC (thin layer chromatography) (DCM (dichloromethane): MeOH (methanol) = 8:1, 2% NH₃/H₂O) to afford a white solid 1(9 mg, 33% yield). ¹H NMR (500 MHz, CD₃OD) δ 8.16 (d, *J* = 5.0 Hz, 1H), 7.06 (s, 1H), 6.86 (d, *J* = 5.0 Hz, 1H), 6.68 (s, 1H), 5.78 (s, 1H), 4.94 (d, *J* = 13.5 Hz, 1H), 4.71 (d, *J* = 13.5 Hz, 1H), 3.67-3.52 (m, 2H), 3.28-3.13 (m, 2H), 2.98-2.89 (m, 3H), 2.88-2.84 (m, 1H), 2.83 (s, 3H), 2.79 (s, 3H), 2.53-2.38 (m, 2H), 2.14-2.07 (m, 2H), 2.06 (s, 3H) ppm. LCMS: m/z 471.5 [M+H]⁺.

### Example 2: Preparation of Compound 2

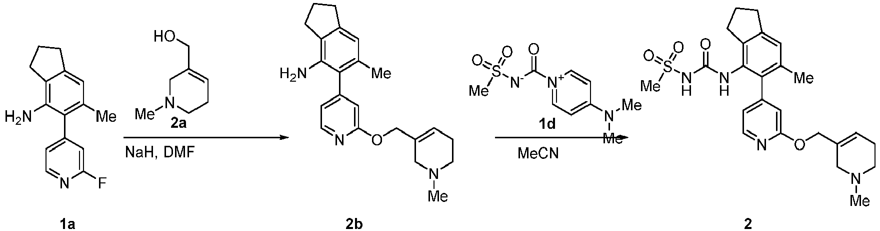

To a solution of (1-methyl-1,2,5,6-tetrahydropyridine-3-yl) methanol 2a (20 mg, 0.16 mmol) in DMF(2 mL) was added NaH (7 mg, 0.17 mmol) at 0 °C. The reaction mixture was stirred 0.5 h. Then a solution of **1a** (20 mg, 0.08 mmol) in DMF (1 mL) was added. The reaction was stirred at 60 °C for about 4 h under N₂. TLC indicated the reaction was completed. The mixture was concentrated to give the crude product. The crude was purifired by Pre-TLC (DCM: MeOH = 15:1) to afford a white solid **2b** (13 mg, 45%). LCMS: *m*/*z* 350.5 [M+H]⁺.

The crude product **2b** (13 mg, 0.04 mmol) and **1d** (10 mg, 0.04 mmol) was dissolved in MeCN (5 mL). The mixture was stirred at 60 °C for 3 h. TLC indicated the reaction was completed. The mixture was concentrated to give the crude product. The crude product was purified by Pre-TLC (DCM:MeOH: Ammonia = 10:1:0.1) to afford a white solid 2(3 mg, 17% yield).¹H NMR (500 MHz, CD₃OD) δ 8.14 (d, *J* = 5.0 Hz, 1H), 7.08 (s, 1H), 6.84 (d, *J* = 5.0 Hz, 1H), 6.70 (s, 1H), 6.05 (s, 1H), 5.00 (d, *J* = 12.0 Hz, 1H), 4.70 (d, *J* = 12.0 Hz, 1H), 3.66 (s, 2H), 3.22-3.08 (m, 2H), 2.99-2.74 (m, 10H), 2.51-2.41 (m, 2H), 2.12-2.00 (m, 5H) ppm. LCMS: *m*/*z* 471.4 [M+H]⁺.

### Example 3: Preparation of Compound 3

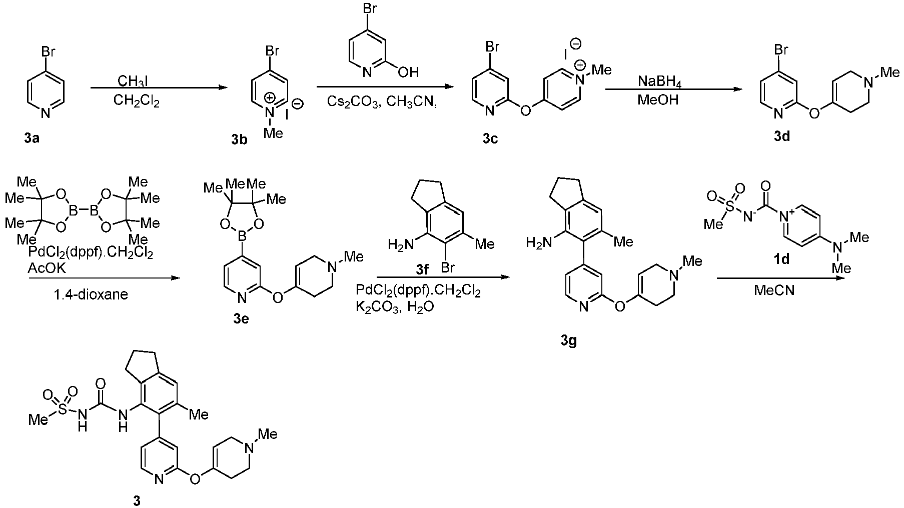

### Compound 3f was synthesized according to WO2020035465

To a solution of 4-Bromopyridine (1.12 g, 7.09 mmol) in DCM (5 mL) was added MeI (Methyl iodide) (2.01 g, 14.18 mmol) at 0 °C. The reaction mixture was stirred 16 h at RT. Then the mixture was filterated and the solid was washed with PE (petroleum ether) to give a brown solid **3b** (1.70 g, 80% yield). ¹H NMR (500 MHz, D₂O) δ 8.59 (d, *J* = 6.5 Hz, 2H), 8.24 (d, *J* = 6.5 Hz, 2H), 4.28 (s, 3H) ppm.

The **3b** (0.70 g, 2.33 mmol) and 4-Bromo-2-hydroxypyridine (0.40 g, 2.33 mmol) was dissolved in MeCN (10 mL). The mixture was added Cs₂CO₃ (1.14 g, 3.50 mmol) in portions with stirring at RT. Then the reaction mixture was stirred 3 h at RT. Then the reaction was filterated and the solid was washed with MeCN to give give the crude product **3c** (2.0 g). LCMS: m/z 265.2 & 267.2 [M+H]⁺ .

The crude product **3c** (2.0 g) was dissolved in MeOH (30 mL). The mixture was added NaBH₄ (173 mg, 4.6 mmol) in portions with stirring at 0 °C. Then the reaction mixture was stirred 1 h at 0 °C. The reaction was adjusted to pH = 8 with sat. NaHCO₃ slowly and stirred for another 5 min. Then the mixture was diluted with EA (50 mL), dried with anhydrous sodium sulfate, filterated and concentrated to give the crude product. The crude product was purified by column chromatography (0 - 10% MeOH: DCM) to afford a brown solid **3d** (0.3 g, 50% yield). ¹H NMR (500 MHz, CD₃OD) δ 7.44 (d, *J* = 7.0 Hz, 1H), 6.80 (d, *J* = 2.0 Hz, 1H), 6.60 (dd, *J* = 7.0, 2.0 Hz, 1H), 5.93-5.80 (m, 1H), 3.19 (dd, *J* = 6.0 Hz, *J* = 3.0 Hz, 2H), 2.79 (t, *J* = 6.0 Hz, 2H), 2.54 (td, *J* = 6.0 Hz, *J* = 3.0 Hz, 2H), 2.44 (s, 3H) ppm.

To a mixture of **3d** (120 mg, 0.445 mmol), Bisphenol borate (169 mg, 0.668 mmol), KOAc(131 mg, 1.34 mmol) and Pd(dppf)Cl₂ (36 mg, 0.045 mmol) was added dioxane (1 mL). The mixture was stirred for 16 h at 90 °C under N₂. The reaction was cooled and the mixture of **3e** was used to the next step. To a solution of **3e** was added **3f** (50 mg, 0.22 mmol), K₂CO₃ (123 mg, 0.891 mmol), water (0.1 mL) and Pd(dppf)Cl₂ (18 mg, 0.022 mmol) under N₂. The mixture was stirred for 4 h at 80 °C. Then the mixture was cooled to RT, diluted with EA (50 mL), washed with sat.NaCl, dried with anhydrous sodium sulfate, filterated and concentrated to give the crude product. The crude product was purified by column chromatography (0-20% MeOH: DCM, 1% NH₃/H₂O) to afford a brown solid **3g** (70 mg, 46% yield). ¹H NMR (500 MHz, CD₃OD) δ 7.56 (d, *J* = 7.0 Hz, 1H), 6.56 (s, 1H), 6.38 (d, *J* = 1.5 Hz, 1H), 6.27 (dd, *J* = 7.0 Hz, *J* = 1.5 Hz, 1H), 5.98-5.87 (m, 1H), 3.25-3.23 (m, 2H), 2.86-2.82 (m, 4H), 2.76-2.68 (m, 2H), 2.66-2.60 (m, 2H), 2.47 (s, 3H), 2.11-2.04 (m, 2H), 2.04 (s, 3H) ppm. LCMS: m/z 336.4 [M+H]⁺ .

The **3g** (25 mg, 0.07 mmol) and **1d** (18 mg, 0.07 mmol) was dissolved in MeCN (5 mL). The mixture was stirred at 60 °C for 3 h. TLC indicated the reaction was completed. The mixture was concentrated to give the crude product. The crude product was purified by Pre-TLC (DCM: MeOH: Ammonia = 10:2:0.1) to afford a white solid **3** (5 mg, 15% yield).

To a solution of **3** (1.72 mg, 0.0038 mmoL) in MeCN (1 mL) was added 0.01 mol/L NaOH solution (0.38 mL) at RT. The reaction solution was stirred for 5 minutes and freeze dried to obtain a white solid sodium salt of compound **3** (1.8 mg, 100% yield). MS *m*/*z* 457.5 [M+H]⁺.

### Example 4: Preparation of Compound 4

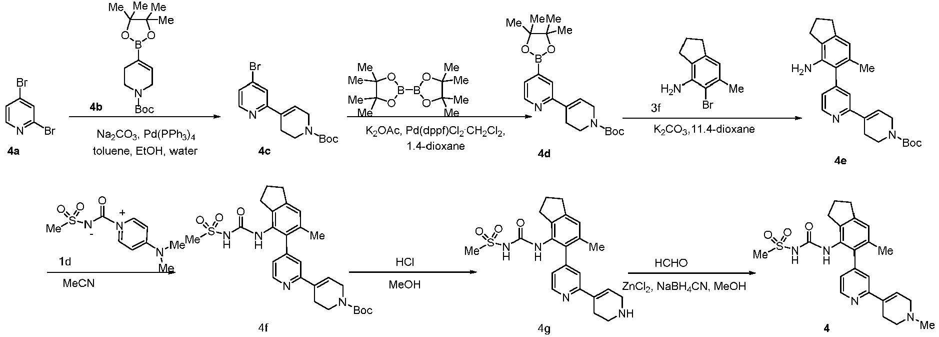

To a mixture of **4a** (1.00 g, 4.22 mmol), **4b** (1.31 g, 4.22 mmol), Na₂CO₃ (894 mg, 8.44 mmol) and Pd(PPh₃)₄ (487 mg, 0.42 mmol) was added toluene/EtOH/water (8:4:2, v:v:v) (14 mL). The mixture was stirred for 2 h at 100 °C under N₂. The reaction was cooled to RT and filterated through diatomite and concentrated to give the crude product. The crude product was purified by column chromatography (PE: EA = 10:1) to afford a white solid **4c** (815 mg, 57% yield). LCMS: *m*/*z* 340.2 [M+H]⁺.

To a mixture of **4c** (180 mg, 0.53 mmol), Bisphenol borate (139 mg, 0.55 mmol), KOAc (208mg, 2.12 mmol) and Pd(dppf)Cl₂ (41 mg, 0.05 mmol) was added dioxane (5 mL). The mixture was stirred for 1 h at 100 °C under N₂. LCMS indicated the reaction was completed. The reaction was cooled and added the mixture of **3f** (80 mg, 0.35 mmol) in dioxane (2 mL), K₂CO₃ (219 mg, 1.59 mmol) and water (1 mL). The mixture was stirred for overnight at 100 °C. LCMS indicated the reaction was completed. The reaction was cooled to RT, filterated through diatomite and concentrated to give the crude product. The crude product was purified by Pre-TLC (DCM: MeOH = 20:1) to afford a white solid **4e** (27 mg, 19% yield). LCMS: *m*/*z* 406.5 [M+H]⁺.

The **4e** (27 mg, 0.07 mmol) and **1d** (16 mg, 0.07 mmol) was dissolved in MeCN (5 mL). The mixture was stirred at 60 °C for 3 h. TLC indicated the reaction was completed. The mixture was concentrated to give the crude. The crude was purified by Pre-TLC (DCM: MeOH: Ammonia = 15:1:0.1) to afford an off-white solid **4f** (22 mg, 63% yield).LCMS: *m*/*z* 527.6 [M+H]⁺.

To a solution of **4f** (22 mg, 0.04 mmol) in MeOH (5 mL) was added 4 M HCl/MeOH solution (0.5 mL). The mixture was stirred at 50 °C for 2 h. TLC indicated the reaction was completed. The mixture was concentrated to give the crude product. The crude product was purified by Pre-TLC (DCM: MeOH = 10:1) to afford a white solid **4g** (15 mg, 83% yield). LCMS: *m*/*z* 427.5 [M+H]⁺.

To a solution of **4g** (15 mg, 0.04 mmol) in MeOH (5 mL) was added paraformaldehyde (2 mg, 0.07mmol), ZnCl₂ (14 mg, 0.11 mmol) and NaBH₃CN (7 mg, 0.11 mmol). The mixture was stirred at 60 °C for 2 h. TLC indicated the reaction was completed. The mixture was concentrated to give the crude product. The crude product was purified by Pre-TLC (DCM: MeOH: Ammonia = 15:1:0.1) to afford a pale solid **4** (3 mg, 19% yield). ¹H NMR (500 MHz, CD₃OD) δ 8.57 (d, *J* = 5.0 Hz, 1H), 7.43 (s, 1H), 7.20 (dd, *J* = 5.0, 1.5 Hz, 1H), 7.15 (s, 1H), 6.68-6.63 (m, 1H), 3.92 (s, 2H), 3.47 (t, *J* = 6.0 Hz, 2H), 3.03 (s, 3H), 3.01-2.95 (m, 6H), 2.93-2.84 (m, 3H), 2.15-2.08 (m, 2H), 2.04 (s, 3H) ppm. LCMS: *m*/*z* 441.4 [M+H]⁺.

### Example 5: Preparation of Compound 5

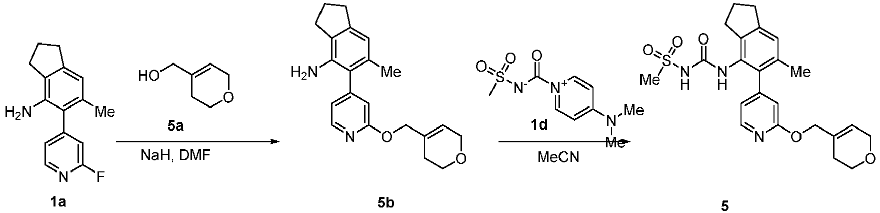

Using the method in Example 2, yellow solid compound **5b** (15 mg, 26% yield) was obtained from compound **1a** (30 mg, 0.12 mmol) and compound **5a** (28 mg, 0.24 mmol).

Using the method in Example 2, white solid compound **5** (12 mg, 59% yield) was obtained from compound **5b** (15 mg, 0.04 mmol) and **1d** (11 mg, 0.04 mmol). ¹H NMR (500 MHz, CD₃OD) δ 8.14 (d, *J* = 5.0 Hz, 1H), 7.10 (s, 1H), 6.81 (dd, *J* = 5.0, 1.0 Hz, 1H), 6.68 (s, 1H), 5.87-5.83 (m, 1H), 4.79-4.69 (m, 2H), 4.16-4.12 (m, 2H), 3.84-3.80 (m, 2H), 2.98 (s, 3H), 2.94 (t, *J* = 7.5 Hz, 2H), 2.86 (t, *J* = 7.5 Hz, 2H), 2.24 -2.18 (m, 2H), 2.12-2.04 (m, 2H), 2.06 (s, 3H) ppm. LCMS: *m*/*z* 458.4 [M+H]⁺.

### Example 6: Preparation of Compound 6

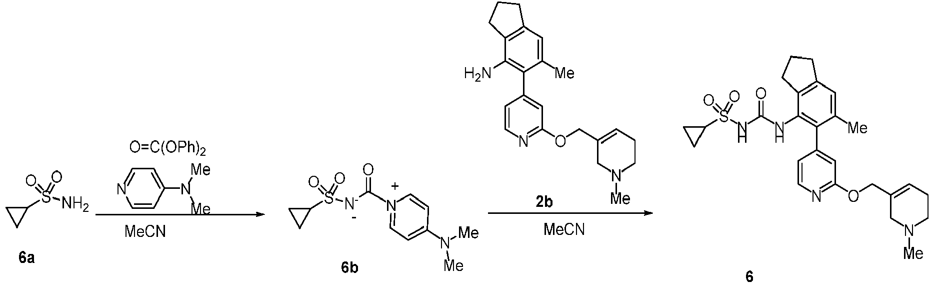

The cyclopropyl Sulfonamide **6a** (2.00 g, 16.51 mmol) and 4-Dimethylaminopyridine (4.03 g, 33.02 mmol) was dissolved in MeCN (28 mL). The mixture was stirred at RT for 10 min. Then diphenyl carbonate (3.89 g, 18.16 mmol) was added. The mixture was stirred at RT for 48 h to obtain a solution of **6b**, which was used to the next step directly.

The **2b** (40 mg, 0.11 mmol) and **6b** (0.5 mL) was dissolved in MeCN (5 mL). The mixture was stirred at 60 °C for 3 h. TLC indicated the reaction was completed. The mixture was concentrated to give the crude product. The crude product was purifired by Pre-TLC (DCM:MeOH: Ammonia = 10:2:0.1) to afford a white solid **6** (17 mg, 30% yield).¹H NMR (500 MHz, CD₃OD) δ 8.14 (d, *J* = 5.2 Hz, 1H), 7.07 (s, 1H), 6.83 (d, *J* = 5.2 Hz, 1H), 6.69 (s, 1H), 6.01 (s, 1H), 4.94 (d, *J* = 12.5 Hz, 1H), 4.69 (d, *J* = 12.5 Hz, 1H), 3.53 (s, 2H), 3.03 (dd, *J* = 10.9, 5.6 Hz, 2H), 2.94 (t, *J* = 7.4 Hz, 2H), 2.90-2.84 (m, 2H), 2.73 (s, 3H), 2.65-2.59 (m, 1H), 2.48-2.39 (m, 2H), 2.13-2.06 (m, 2H), 2.05 (s, 3H), 1.04-0.97 (m, 2H), 0.91-0.84 (m, 2H) ppm. MS *m*/*z* 497.5 [M+H]⁺.

To a solution of **6** (14.36 mg, 0.0289 mmol) in MeCN (2 mL) was added 0.01 mol/L NaOH solution (2.89 mL) at RT. The reaction solution was stirred for 5 minutes and freeze dried to obtain a white solid sodium salt of compound **6** (15 mg, 100% yield). MS m/z 497.5 [M+H]⁺.

### Example 7: Preparation of Compound 7

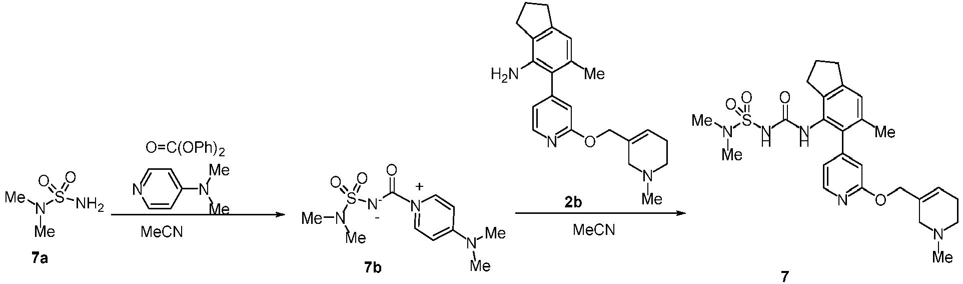

The N, N-dimethylamino Sulfonamide (2.00 g, 16.11 mmol) and 4-Dimethylaminopyridine (3.94 g, 32.22 mmol) was dissolved in MeCN (28 mL). The mixture was stirred at RT for 10 min. Then diphenyl carbonate (3.80 g, 17.72 mmol) was added. The mixture was stirred at RT for overnight and white solid precipitated. The reaction was filterated and the solid was washed with MTBE (methyl tert-butyl ether). The solid was dried to obtain compound **7b** (1.20 g, 27% yield). ¹H NMR (500 MHz, DMSO-*d*₆) δ 8.80 (d, *J* = 7.6 Hz, 2H), 6.98 (d, *J* = 7.6 Hz, 2H), 3.25 (s, 6H), 2.66 (s, 6H) ppm.

Using the method in Example 2, white solid compound **7 (15** mg, 26% yield) was obtained from compound **2b** (40 mg, 0.11 mmol) and **7b** (63 mg, 0.23 mmol). ¹H NMR (500 MHz, CD₃OD) δ 8.16 (d, *J* = 5.2 Hz, 1H), 7.09 (s, 1H), 6.81 (d, *J* = 5.2 Hz, 1H), 6.67 (s, 1H), 6.00 (s, 1H), 4.89 (d, *J* = 12.6 Hz, 1H), 4.72 (d, *J* = 12.3 Hz, 1H), 3.49-3.39 (m, 2H), 2.98-2.90 (m, 4H), 2.87-2.82 (m, 2H), 2.70-2.63 (m, 9H), 2.46-2.34 (m, 2H), 2.12-2.06 (m, 2H), 2.05 (s, 3H) ppm. MS *m*/*z* 500.5 [M+H]⁺.

To a solution of **7** (13.41 mg, 0.0268 mmol) in MeCN (2 mL) was added 0.01 mol/L NaOH solution (2.68 mL) at RT. The reaction solution was stirred for 5 minutes and freeze dried to obtain a white solid sodium salt of compound **7** (15 mg, 100% yield). MS *m*/*z* 500.7 [M+H]⁺.

### Example 8: Preparation of Compound 8

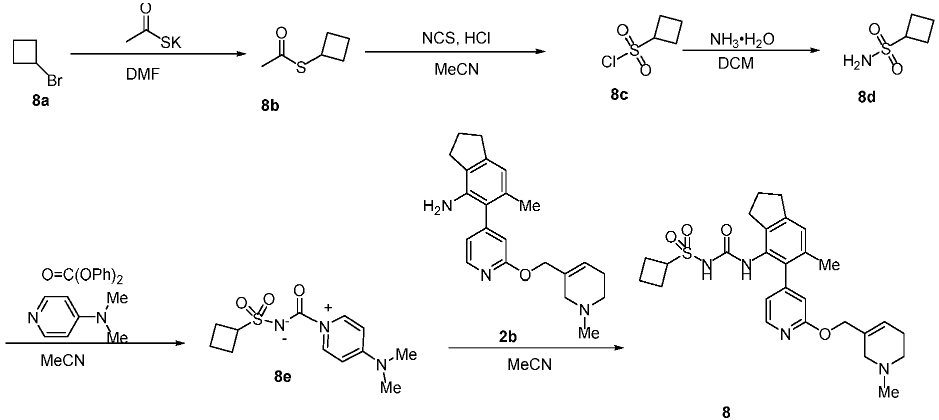

The compound **8a** (1.00 g, 7.41mmol) and potassium thioacetate (1.69 g, 14.81 mmol) was dissolved in DMF (10 mL). The mixture was stirred at 60 °C for 4 h. TLC indicated the reaction was completed. The reaction was added sat.NaCl and extracted with EA (3 × 50 mL). The collected organic phase was washed with sat.NaCl, dried with anhydrous sodium sulfate, filterated and concentrated to give the crude product. The crude product was purified by column chromatography (PE: EA = 5:1) to afford yellow oil **8b** (670 mg, 69%). ¹H NMR (500 MHz, CDCl₃) δ 4.15-4.06 (m, 1H), 2.49-2.38 (m, 2H), 2.27 (s, 3H), 2.11-1.93 (m, 4H) ppm.

To a solution of NCS (N-Chlorosuccinimide) (824 mg, 6.17 mmol) in MeCN (10 mL) was added Con. HCl (1.5 mL). The mixture was stirred at RT for 10 min. Then a solution of 8b (670 mg, 5.15 mmol) in MeCN (5 mL) was added to the reaction at 0 °C. The mixture was stirred at 0 °C for 10 min. TLC indicated the reaction was completed. The reaction was quenched with sat. NaHCO₃, extracted with MTBE (3 × 30 mL). The collected organic phase was washed with sat. NaCl, dried with anhydrous sodium sulfate, filterated and concentrated to give the yellow oil **8c** (548 mg, 69%), which was used to the next step directly.

To a solution of **8c** (548 mg, 3.56 mmol) in DCM (5 mL) was added Con. ammonia (1.5 mL). The mixture was stirred at RT for 1 h. TLC indicated the reaction was completed. The mixture was concentrated to give the crude. The crude was purified by column chromatography c to give compound **8d** (234 mg, 34%). ¹H NMR (500 MHz, DMSO-*d*₆) δ 6.72 (s, 2H), 3.75-3.70 (m, 1H), 2.34-2.16 (m, 4H), 1.97-1.82 (m, 2H) ppm.

The **8d** (234 mg, 1.73mmol) and 4-Dimethylaminopyridine (423 mg, 3.46 mmol) was dissolved in MeCN (3 mL). The mixture was stirred at RT for 48 h. Then diphenyl carbonate (3.89 g, 18.16 mmol) was added. The mixture was stirred at RT for 48 h to obtain a solution of **8e,** which was used to the next step directly.

The **2b** (40 mg, 0.11 mmol) and **8e** (0.5 mL) was dissolved in MeCN (5 mL). The mixture was stirred at 60 °C for 3 h. TLC indicated the reaction was completed. The mixture was concentrated to give the crude product. The crude product was purified by Pre-TLC (DCM: MeOH: Ammonia = 10:1:0.1) to afford white solid **8** (11 mg, 19% yield). ¹H NMR (500 MHz, CD₃OD) δ 8.15 (d, *J* = 5.2 Hz, 1H), 7.06 (s, 1H), 6.82 (d, *J* = 5.1 Hz, 1H), 6.66 (s, 1H), 6.02 (s, 1H), 4.93 (d, *J* = 12.5 Hz, 1H), 4.71 (d, *J* = 12.1 Hz, 1H), 3.98-3.91 (m, 1H), 3.55 (s, 2H), 3.12-3.00 (m, 2H), 2.93 (t, *J* = 7.4 Hz, 2H), 2.88-2.82 (m, 2H), 2.74 (s, 3H), 2.48-2.40 (m, 2H), 2.40-2.32 (m, 2H), 2.21-2.13 (m, 2H), 2.11-2.05 (m, 2H), 2.04 (s, 3H), 2.00-1.85 (m, 2H). MS m/z 511.6 [M+H]⁺.

To a solution of **8** (9.59 mg, 0.0188 mmoL) in MeCN (2 mL) was added 0.01 mol/L NaOH solution (1.88 mL) at RT. The reaction solution was stirred for 5 minutes and freeze dried to obtain a white solid sodium salt of compound **8** (10 mg, 100% yield). MS *m*/*z* 511.5 [M+H]⁺.

### Example 9: Preparation of Compound 9

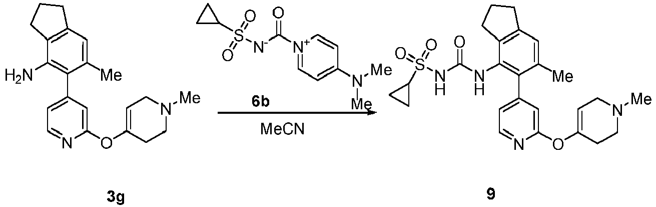

Using the method in Example 6, white solid compound **9** (11 mg, 19% yield) was obtained from compound **3g** (40 mg, 0.12 mmol) and **6b** (0.5 mL reaction solution). ¹H NMR (500 MHz, CDCl₃) δ 7.74 (brs, 1H), 7.29 (d, *J* = 6.8 Hz, 1H), 7.02 (s, 1H), 6.32 (s, 1H), 6.13 (d, *J* = 6.7 Hz, 1H), 5.77 (s, 1H), 4.80 (brs, 1H), 3.19-3.04 (m, 2H), 2.98-2.77 (m, 6H), 2.74-2.61 (m, 2H), 2.59-2.50 (m, 1H), 2.43 (s, 3H), 2.12 (s, 3H), 2.10-2.00 (m, 2H), 1.20-1.10 (m, 2H), 0.90-0.80 (m, 2H) ppm. MS *m*/*z* 483.5 [M+H]⁺.

To a solution of **9** (10.52 mg, 0.0218 mmoL) in MeCN (2 mL) was added 0.01 mol/L NaOH solution (2.18 mL) at RT. The reaction solution was stirred for 5 minutes and freeze dried to obtain a white solid sodium salt of compound **9** (11.0 mg, 100% yield). MS *m*/*z* 483.6 [M+H]⁺.

### Example 10: Preparation of Compound 10

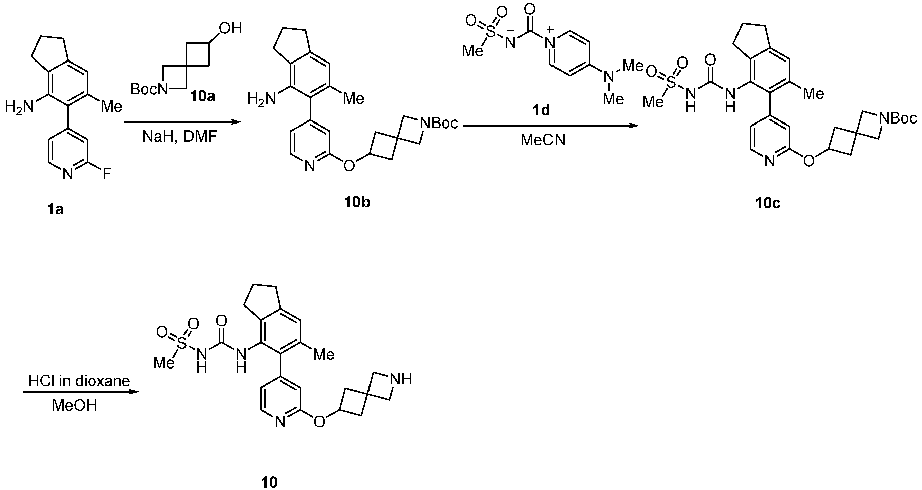

Using the method in Example 1, compound **10b** crude product (97 mg, 55% purity) was obtained from compound **1a** (30 mg, 0.12 mmol) and **10a** (66 mg, 0.31 mmol).

Using the method in Example 1, compound **10c** (20 mg, 29% yield) was obtained from compound **10b** crude product (97 mg, 55% purity) and **1d** (30 mg, 0.12 mmol).

To a solution of **10c** (20 mg, 0.04 mmol) in MeOH (3 mL) was added 4 M HCl/MeOH solution (0.5 mL). The mixture was stirred at 40 °C for 1 h. After cooling the reaction solution, ammonia water was added to adjust the pH to neutral. The mixture was concentrated to give the crude. The crude was purifired by Pre-TLC (DCM: MeOH: Ammonia = 2:1:0.02) to afford a white solid **10** (7 mg, 43% yield). ¹H NMR (500 MHz, CD₃OD) δ 8.15 (d, *J* = 5.2 Hz, 1H), 7.12 (s, 1H), 6.83 (d, *J* = 4.2 Hz, 1H), 6.60 (s, 1H), 5.07-5.01 (m, 1H), 4.16 (s, 2·H), 4.10 (s, 2H), 3.05 (s, 3H), 2.95 (t, *J* = 7.4 Hz, 2H), 2.90-2.82 (m, 4H), 2.46-2.36 (m, 2H), 2.13-2.07 (m, 2H), 2.06 (s, 3H) ppm. MS *m*/*z* 457.4 [M+H]⁺.

To a solution of **10** (3.82 mg, 0.0084 mmoL) in MeCN (1 mL) was added 0.01 mol/L NaOH solution (0.84 mL) at RT. The reaction solution was stirred for 5 minutes and freeze dried to obtain a white solid sodium salt of compound **10** (11.0 mg, 100% yield). MS *m*/*z* 483.6 [M+H]⁺.

### Example 11: Preparation of Compound 11

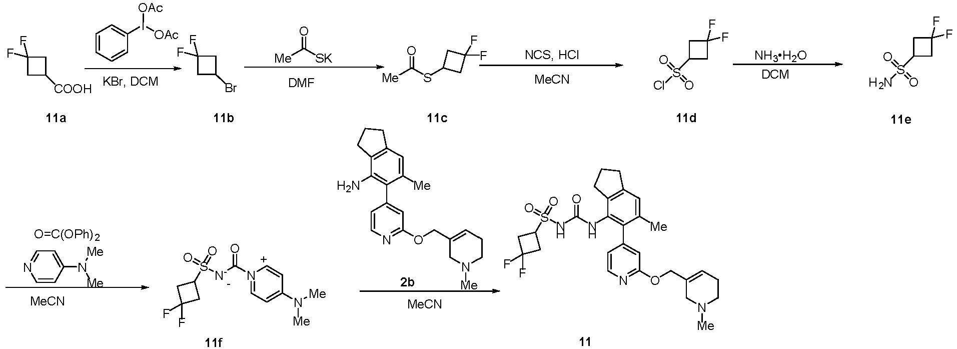

To a mixture of **11a** (4.00 g, 29.39mmol) and iodophenyl diacetate (14.20 g, 44.09mmol) in DCM was added KBr (5.95 g, 49.96mmol). The mixture was stirred for 48 h at RT under 365 nm fluorescent lamp irradiation. Filtered the reaction mixture, distilled the filtrate at 70 °C under atmospheric pressure until there is no fraction, and then distilled at 60 °C under reduced pressure to obtain a light yellow liquid compound **11b** (2.07 g, 41% yield). ¹H NMR (500 MHz, CDCl₃) δ 4.28-4.13 (m, 1H), 3.38-3.19 (m, 2H), 3.06-2.89 (m, 2H) ppm

Using the method in Example **8,** yellow oil compound **11c** (150 mg, 15% yield) was obtained from compound **11b** (1.00 g, 5.85 mmol) and potassium thioacetate (1.27 g, 7.41 mmol).

Using the method in Example **8, 11e** (20 mg, 17% yield) was obtained from compound **11c** (100 mg, 0.60mmol). ¹H NMR (500 MHz, CDCl₃) δ 7.11 (s, 2H), 3.76 - 3.66 (m, 1H), 3.02 - 2.83 (m, 4H)

The **11e** (20 mg, 0.12 mmol) and 4-Dimethylaminopyridine (29 mg, 0.23 mmol) was dissolved in MeCN (1 mL). The mixture was stirred at RT for 10 min. Then diphenyl carbonate (28 mg, 0.13 mmol) was added. The mixture was stirred at RT for 48 h to obtain a solution of **11f**, which was used to the next step directly.

Using the method in Example **6**, white solid compound **11** (5 mg, 13% yield) was obtained from compound **2b** (20 mg, 0.06 mmol) and **11f** (1.0 mL, reaction solution). ¹H NMR (500 MHz, CD₃OD) δ 8.14 (d, *J* = 5.1 Hz, 1H), 7.05 (s, 1H), 6.83 (dd, *J* = 5.2, 1.1 Hz, 1H), 6.70 (s, 1H), 6.07 (s, 1H), 5.01 (d, *J* = 12.7 Hz, 1H), 4.69 (d, *J* = 11.2 Hz, 1H), 3.88-3.79 (m, 1H), 3.72 (m, 2H), 3.27-3.18 (m, 2H), 2.96-2.84 (m, 6H), 2.87 (s, 3H), 2.80-2.70 (m, 2H), 2.52-2.46 (m, 2H), 2.12-2.02 (m, 2H), 2.05 (s, 3H). MS *m*/*z* 547.6 [M+H]⁺.

To a solution of **11** (4.58 mg, 0.0084 mmoL) in MeCN (1 mL) was added 0.01 mol/L NaOH solution (0.84 mL) at RT. The reaction solution was stirred for 5 minutes and freeze dried to obtain a white solid sodium salt of compound **11** (11.0 mg, 100% yield). MS *m*/*z* 547.8 [M+H]⁺.

### Example 12: Preparation of Compound 12

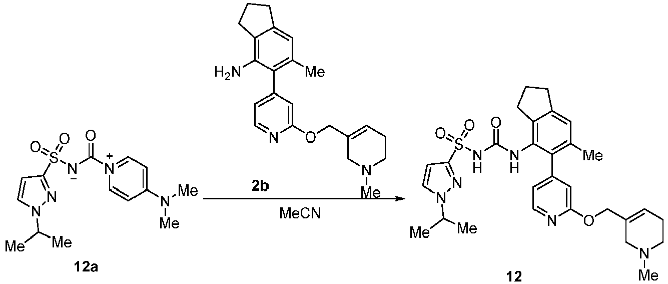

### Compound 12a was synthesized according to WO2020104657

Using the method in Example 6, white solid compound **12** (30 mg, 90% yield) was obtained from compound **2b** (21 mg, 0.06 mmol) and **12a** (40 mg, 0.12 mmol). ¹H NMR (500 MHz, CD₃OD) δ 8.15 (d, *J* = 4.5 Hz, 1H), 7.81 (d, *J* = 2.4 Hz, 1H), 7.11 (s, 1H), 6.79 (d, *J* = 4.5 Hz, 1H), 6.68 (d, *J* = 2.4 Hz, 1H), 6.66 (s, 1H), 6.14 (s, 1H), 5.04 (d, *J* = 12.8 Hz, 1H), 4.76 (d, *J* = 12.3 Hz, 1H), 4.61 (dt, *J* = 13.4, 6.7 Hz, 1H), 3.88 (s, 2H), 3.41-3.35 (m, 2H), 2.98 (s, 3H), 2.92 (t, *J* = 7.4 Hz, 2H), 2.64-2.51 (m, 4H), 2.06 (s, 3H), 2.04-1.97 (m, 2H), 1.51 (d, *J* = 6.7 Hz, 6H) ppm. MS *m*/*z* 565.8 [M+H]⁺.

To a solution of **12** (24 mg, 0.0426 mmoL) in MeCN (2 mL) was added 0.01 mol/L NaOH solution (4.26 mL) at RT. The reaction solution was stirred for 5 minutes and freeze dried to obtain a white solid sodium salt of compound **12** (25.0 mg, 100% yield). MS m/z 565.7 [M+H]⁺.

### Example 13: Preparation of Compound 13

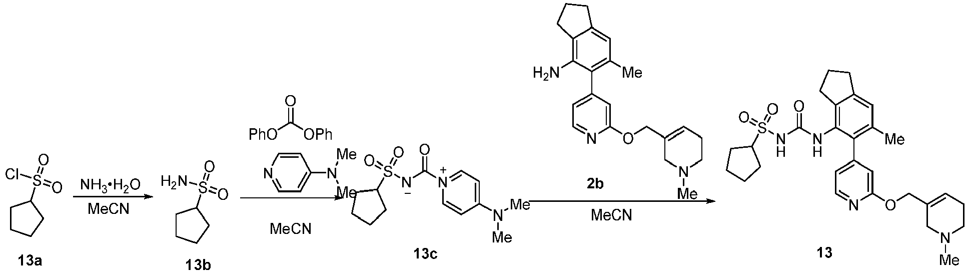

To a solution of **3a** (100 mg, 0.59 mmol) in MeCN (1 mL) was added Con. ammonia (1.0 mL). The mixture was stirred at RT for 2 h. TLC indicated the reaction was completed. The mixture was concentrated to give the crude product. The crude product was purified by column chromatography to give a white solid compound **13b** (71 mg, yield 80%). ¹H NMR (500 MHz, DMSO-*d*₆) δ 6.70 (s, 2H), 3.43-3.35 (m, 1H), 1.94-1.81 (m, 4H), 1.70-1.60 (m, 2H), 1.59- 1.50 (m, 2H) ppm.

The **13b** (70 mg, 0.47 mmol) and 4-Dimethylaminopyridine (115 mg, 0.94 mmol) was dissolved in MeCN (3 mL). The mixture was stirred at RT for 5 min. Then diphenyl carbonate (28 mg, 0.13 mmol) was added. The mixture was stirred at RT for 48 h to obtain a solution of **13c,** which was used to the next step directly.

Using the method in Example **6,** white solid compound **13** (2.8 mg, 32% yield) was obtained from compound **2b** (6 mg, 0.02 mmol) and **13c** (0.4 mL reaction solution). ¹H NMR (500 MHz, DMSO-*d*₆) δ 8.16 (d, *J* = 5.2 Hz, 1H), 7.41 (s, 1H), 7.07 (s, 1H), 6.74 (d, *J* = 5.1 Hz, 1H), 6.57 (s, 1H), 5.83 (s, 1H), 4.70 (s, 2H), 3.71-3.63 (m, 1H), 2.94-2.85 (m, 3H), 2.74 (t, *J* = 7.3 Hz, 2H), 2.65-2.62 (m, 1H), 2.44 (t, *J* = 5.6 Hz, 2H), 2.38-2.34 (m, 1H), 2.27 (s, 3H), 2.14 (s, 2H), 2.03-2.00 (m, 1H), 1.98 (s, 3H), 1.80-1.71 (m, 4H), 1.64-1.56 (m, 2H), 1.56-1.48 (m, 2H) ppm. MS m/z 525.7 [M+H]⁺.

To a solution of **13** (1.27 mg, 0.0024 mmoL) in MeCN (0.2 mL) was added 0.01 mol/L NaOH solution (0.24 mL) at RT. The reaction solution was stirred for 5 minutes and freeze dried to obtain a white solid sodium salt of compound **13** (1.32 mg, 100% yield). MS *m*/*z* 525.5 [M+H]⁺.

### Example 14: Preparation of Compound 14

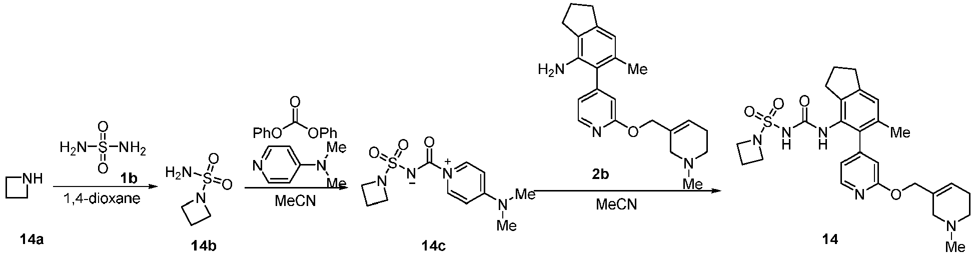

To a solution of sulfonamide (353 mg, 3.68 mmol) in dioxane (4 mL) was added **14a** (200 mg, 3.50 mmol). The mixture was stirred at 90 °C for 24 h. TLC indicated the reaction was completed. The mixture was concentrated and dissolved in CHCl₃. The mixture was concentrated to give the white crude product **14b,** which was used to the next step directly.

The **14b** (477 mg) and 4-Dimethylaminopyridine (855 mg, 7.00 mmol) was dissolved in MeCN (5 mL). The mixture was stirred at RT for 5 min. Then diphenyl carbonate (900 mg, 4.20 mmol) was added. The mixture was stirred at RT for 3 d to obtain a solution of **14c,** which was used to the next step directly.

Using the method in Example 6, white solid compound **14** (6.37 mg, 35% yield) was obtained from compound **2b** (12 mg, 0.03 mmol) and **14c** (1.0 mL reaction solution).

To a solution of **14** (2.34 mg, 0.0046 mmoL) in MeCN (0.5 mL) was added 0.01 mol/L NaOH solution (0.24 mL) at RT. The reaction solution was stirred for 5 minutes and freeze dried to obtain a white solid sodium salt of compound **14** (2.44 mg, 100% yield). MS *m*/*z* 512.6 [M+H]⁺.

### Example 15: Preparation of Compound 15

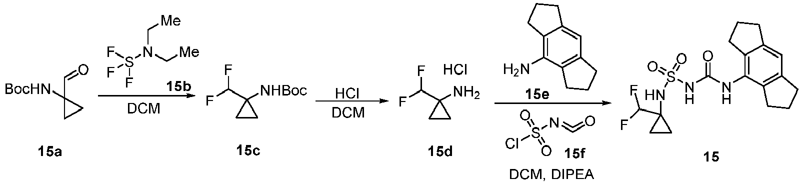

To a solution of **15a** (200 mg, 1.08 mmol) in DCM (5 mL) was added DAST (diethylaminosulphur trifluoride) 15**b** (261 mg, 1.62 mmol) slowly at 0 °C. The reaction mixture was stirred overnight at RT. When the reaction was completed, the reaction was quenched with sat. NaHCO₃, extracted with DCM (3×15 mL). The collected organic phase was washed with sat.NaCl, dried with anhydrous sodium sulfate, filterated and concentrated to to give the crude product. The crude product was purified by column chromatography (PE: EA = 8:1) to give a white solid compound **15c** (45 mg, 20% yield). ¹H NMR (500 MHz, DMSO-*d*₆) δ 7.52 (s, 1H), 5.90 (t, *J* = 57.1 Hz, 1H), 1.38 (s, 9H), 0.97-0.91 (m, 2H), 0.84-0.76 (m, 2H) ppm.

To a solution of **15c** (45 mg, 0.22 mmol) in DCM (4 mL) was added 4 M HCl/dioxane solution (1 mL). The mixture was stirred overnight at RT. When the reaction was completed, the mixture was concentrated to give brown solid **15d** (31 mg, 100% yield). ¹H NMR (500 MHz, DMSO-*d*₆) δ9.11 (brs, 3H), 5.96 (t, *J* = 53.8 Hz, 1H), 1.21-1.14 (m, 2H), 1.04-1.00 (m, 2H) ppm.

Under ice water bath, Chlorosulfonyl isocyanate **15f** (7 mg, 0.05 mmol) was dissolved in dichloromethane (3 mL) and then compound **15e** (8 mg, 0.05 mmol) was added. The reaction mixture was stirred in an ice water bath for 10 minutes. TLC indicated the reaction was completed. A mixture of dichloromethane (2 mL) of compound (13 mg, 0.09 mmol) and diisopropylethylamine (2 drops) was slowly added to the reaction solution. The reaction mixture was stirred at room temperature for 1 hour. After the reaction is completed, the reaction solution is concentrated to obtain the crude product. The crude product was purified by Pre-TLC (DCM: MeOH = 15:1, 2% ammonia) to afford a white solid **15** (4 mg, 22% yield). ¹H NMR (500 MHz, DMSO-*d*₆) δ 10.29 (s, 1H), 7.89 (s, 1H), 6.91 (s, 1H), 6.12 (t, *J* = 57.6 Hz, 1H), 2.80 (t, *J =* 7.3 Hz, 4H), 2.70 (t, *J* = 7.3 Hz, 4H), 2.02-1.92 (m, 4H), 1.10-1.05 (m, 2H), 0.96-0.91 (m, 2H) ppm. MS *m*/*z* 386.5 [M+H]⁺.

To a solution of **15** (2.89 mg, 0.0075 mmoL) in MeCN (1.0 mL) was added 0.01 mol/L NaOH solution (0.75 mL) at RT. The reaction solution was stirred for 5 minutes and freeze dried to obtain a white solid sodium salt of compound **15** (3.05 mg, 100% yield). MS *m*/*z* 386.3 [M+H]⁺.

### Example 16: Preparation of Compound 16

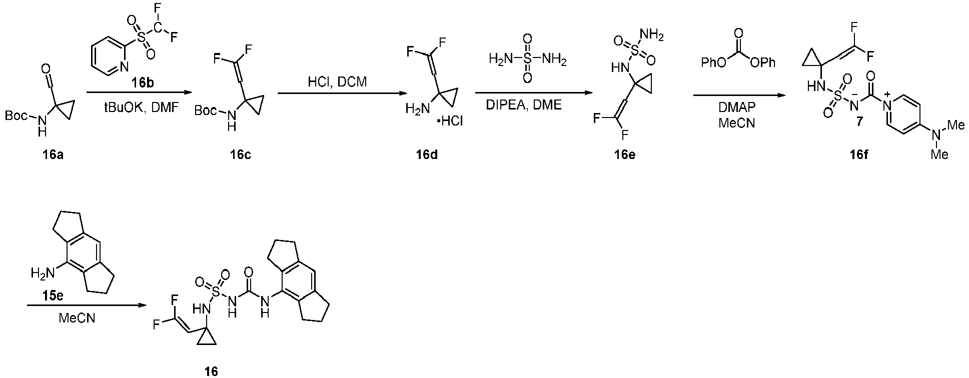

To a solution of tBuOK (549 mg, 4.89 mmol) in DMF (5 mL) was added **16a** (503 mg, 2.72 mmol) and **16b** (472 mg, 2.44 mmol) at -50 °C under N₂. The reaction mixture was stirred for 1 h at -50 °C. Then sat.NH₄Cl (10.0 mL) and Con. HCl (5 mL) was added. The reaction was stirred for 3 h at RT. TLC indicated the reaction was completed. Water (30 mL) was added and extracted with EA (3 × 30 mL). The collected organic phase was washed with sat.NaCl, dried with anhydrous sodium sulfate, filterated and concentrated to to give the crude product. The crude product was purified by column chromatography (PE: EA= 20:1) to give a white solid **16c** (353 mg, 59% yield). ¹H NMR (500 MHz, CDCl₃) δ 5.01 (s, 1H), 4.54 (d, *J* = 24.5 Hz, 1H), 1.44 (s, 9H), 1.04-0.90 (m, 4H) ppm.

To a solution of **16c** (353 mg, 1.61 mmol) in DCM (10 mL) was added 4 M HCl/dioxane solution (3 mL). The mixture was stirred overnight at 40 °C. TLC indicated the reaction was completed. The mixture was concentrated to give the crude product **16d,** which was used to the next step directly.

To a solution of **16d** (250 mg, 1.61 mmol) in ethylene glycol dimethyl ether (5 mL) was added diisopropylethylamine (5 drops) and sulfonamide (185 mg, 1.93 mmol). The reaction mixture was stirred overnight at 90 °C. TLC indicated the reaction was completed. The reaction was concentrated to to give the crude product. The crude product was purified by column chromatography (PE: EA = 2:1) to give a white solid **16e** (82 mg, 26% yield). ¹H NMR (500 MHz, DMSO-*d*₆) δ 7.30 (s, 1H), 6.63 (s, 2H), 4.90 (dd, *J* = 25.9, 3.3 Hz, 1H), 1.12 (q, *J* = 4.8 Hz, 2H), 0.78 (q, *J* = 5.0 Hz, 2H) ppm.

The **16e** (82 mg, 0.41 mmol) and 4-Dimethylaminopyridine (101 mg, 0.83 mmol) was dissolved in MeCN (2 mL). The mixture was stirred at RT for 5 min. Then diphenyl carbonate (98 mg, 0.46 mmol) was added. The mixture was stirred at RT for 48 h to obtain a solution of **16f,** which was used to the next step directly.

Using the method in Example 6, white solid compound **16** (3.0 mg, 13% yield) was obtained from compound **15e** (10 mg, 0.06 mmol) and **16f** (1.0 mL reaction solution). ¹H NMR (500 MHz, CD₃OD) δ 6.98 (s, 1H), 4.82 (dd, *J* = 24.8, 2.4 Hz, 1H), 2.87 (t, *J* = 7.3 Hz, 4H), 2.79 (t, *J* = 7.3 Hz, 4H), 2.11 - 2.02 (m, 4H), 1.24 (q, *J* = 5.0 Hz, 2H), 0.94 (q, *J* = 5.0 Hz, 2H) ppm. MS *m*/*z* 398.4 [M+H]⁺.

To a solution of **16** (2.41 mg, 0.0061 mmoL) in MeCN (1 mL) was added 0.01 mol/L NaOH solution (0.61 mL) at RT. The reaction solution was stirred for 5 minutes and freeze dried to obtain a white solid sodium salt of compound **16** (2.54 mg, 100% yield). MS *m*/*z* 398.3 [M+H]⁺.

### Example 17: Preparation of Compound 17

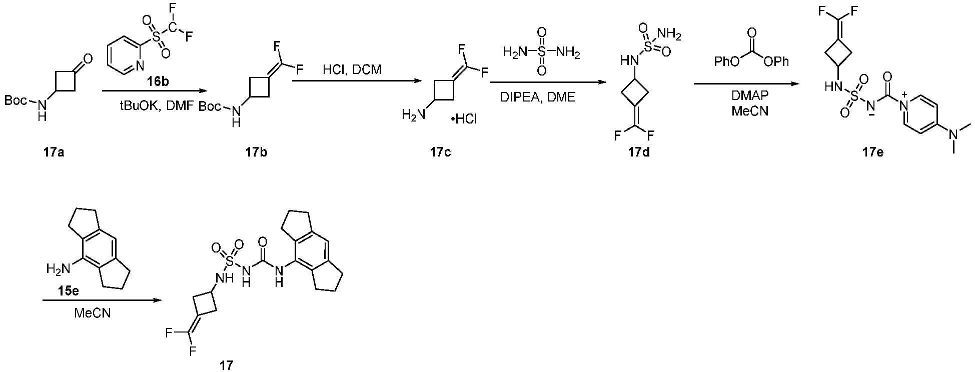

Using the method in Example **16,** white solid compound **17b** (384.0 mg, 32% yield) was obtained from compound **17a** (1.00 g, 5.40 mmol), tBuOK (1.09 g, 9.72 mmol) and **16b** (939 mg, 4.86 mmol). ¹H NMR (500 MHz, CDCl₃) δ 4.79 (brs, 1H), 4.23 (brs, 1H), 3.13-2.99 (m, 2H), 2.60-2.48 (m, 2H), 1.44 (s, 9H) ppm

Using the method in Example **16,** crude compound **17c** was obtained from compound **17b** (148 mg, 0.68 mmol), which was used to the next step directly.

Using the method in Example **16,** white solid compound **17d** (50.0 mg, 37% yield) was obtained from compound **17c** (105 mg, 0.67 mmol) and sulfonamide (78 mg, 0.81 mmol). ¹H NMR (500 MHz, DMSO-*d*₆) δ 7.16 (d, *J* = 8.8 Hz, 1H), 6.62 (s, 2H), 3.92-3.76 (m, 1H), 3.03-2.91 (m, 2H), 2.74-2.62 (m, 2H) ppm.

The **17d** (50 mg, 0.25 mmol) and 4-Dimethylaminopyridine (62 mg, 0.50 mmol) was dissolved in MeCN (2 mL). The mixture was stirred at RT for 5 min. Then diphenyl carbonate (59 mg, 0.28 mmol) was added. The mixture was stirred at RT for 48 h to obtain a solution of **17e,** which was used to the next step directly.

Using the method in Example 6, white solid compound **17** (7 mg, 20% yield) was obtained from compound **15e** (15 mg, 0.09 mmol) and **17e** (1.0 mL reaction solution). ¹H NMR (500 MHz, CD₃OD) δ 6.98 (s, 1H), 4.10-4.02 (m, 1H), 3.11-3.03 (m, 2H), 2.87 (t, *J* = 7.4 Hz, 4H), 2.79 (t, *J* = 7.4 Hz, 4H), 2.77-2.72 (m, 2H), 2.09-2.03 (m, 4H) ppm. MS *m*/*z* 398.4 [M+H]⁺.

To a solution of **17**(6.4 mg, 0.0161 mmoL) in MeCN (1 mL) was added 0.01 mol/L NaOH solution (1.61 mL) at RT. The reaction solution was stirred for 5 minutes and freeze dried to obtain a white solid sodium salt of compound **17** (6.74 mg, 100% yield). ¹H NMR (500 MHz, CD₃OD) δ 6.88 (s, 1H), 3.99 - 3.90 (m, 1H), 3.05 - 2.94 (m, 2H), 2.88 - 2.74 (m, 8H), 2.74 -2.61 (m, 2H), 2.11 - 1.95 (m, 4H) ppm MS m/z 398.3 [M+H]⁺.

### Example 18: Preparation of Compound 18

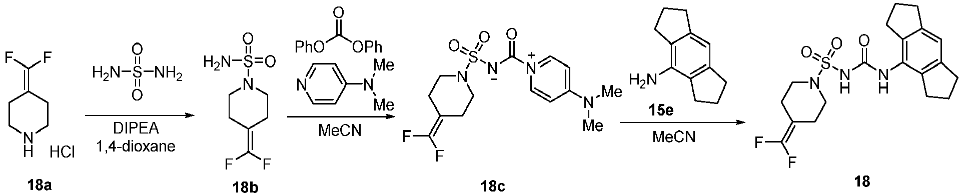

The **18a** (90 mg, 0.53 mmol) was dissolved in dioxane (1.5 mL), and diisopropylethylamine (2 drops) was added to adjust the pH of the mixture to alkaline. After this, sulfonamide (62 mg, 0.64 mmol) was added. The mixture was stirred overnight at 95 °C. The mixture was cooled to room temperature, filtered and washed the reaction solution. The obtained filtrate is concentrated to obtain a white solid crude product **18b,** which was used to the next step directly.

The crude product **18b** (111 mg) and 4-Dimethylaminopyridine (62 mg, 0.50 mmol) was dissolved in MeCN (3 mL). The mixture was stirred at RT for 5 min. Then diphenyl carbonate (123 mg, 0.58 mmol) was added. The mixture was stirred at RT for 2 d to obtain a solution of **18c,** which was used to the next step directly.

Using the method in Example 6, white solid compound **18** (7.3 mg, 31% yield) was obtained from compound **15e** (10 mg, 0.06 mmol) and **18c** (0.5 mL reaction solution). ¹H NMR (500 MHz, CDCl₃) δ 7.00 (s, 1H), 3.41-3.22 (m, 4H), 2.93-2.81 (m, 4H), 2.81-2.68 (m, 4H), 2.39-2.19 (m, 4H), 2.13-1.96 (m, 4H) ppm.MS *m*/*z* 412.4 [M+H]⁺.

To a solution of **18** (6.93 mg, 0.0168 mmoL) in MeCN (1 mL) was added 0.01 mol/L NaOH solution (1.68 mL) at RT. The reaction solution was stirred for 5 minutes and freeze dried to obtain a white solid sodium salt of compound **18** (7.30 mg, 100% yield). ¹H NMR (500 MHz, CD₃OD) δ 6.87 (s, 1H), 3.22 (t, *J* = 5.5 Hz, 4H), 2.87-2.77 (m, 8H), 2.27 (t, *J* = 5.5 Hz, 4H), 2.08-1.98 (m, 4H). MS *m*/*z* 412.4 [M+H]⁺.

### Example 19: Preparation of Compound 19

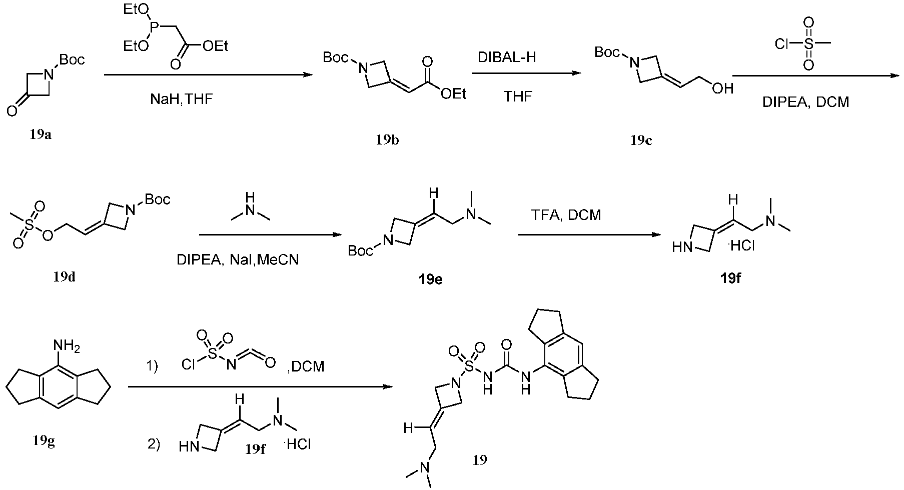

To a solution of NaH (642 mg, 60%, 16.06 mmol) in THF (25 mL) was added ethyl 2-(Diethoxyphosphoryl) acetate (3.60 g, 16.06 mmol) at -78 °C under N₂. The reaction mixture was warmed to RT and stirred 0.5 h. Then **19a** (2.50 g, 14.60 mmol) was added at 0 °C. The mixture was stirred overnight at RT. TLC indicated the reaction was completed. The mixture was quenched with sat.NaHCO₃ and extracted with EA (3×50 mL). The collected organic phase was washed with sat.NaCl, dried with anhydrous sodium sulfate, filterated and concentrated. The crude was purified by column chromatography to give the **19b** (2.50 g, 71%).

To a solution of **19b** (590 mg, 2.45 mmol) in THF (tetrahydrofuran) (10 mL) was added DIBAL-H (3.60 g, 16.06 mmol) at -78 °C under N₂. After this, the mixture was stirred for 1 h at - 78 °C. TLC indicated the reaction was completed. Warmed the temperature of the reaction solution to 0 °C, then water (0.2 mL), 15% sodium hydroxide aqueous solution (0.2 mL), and water (0.4 mL) was added successively. The reaction mixture was stirred at RT for 15 minutes. Anhydrous sodium sulfate was added and the reaction was stirred at RT for another 15 minutes. The mixture was filterated and concentrated. The crude product was purified by column chromatography to give the product **19c** (372 mg, 76%).

To a solution of **19c** (200 mg, 1.00 mmol) in DCM (5 mL) was added diisopropylethylamine (388 mg, 3.01 mmol) and methanesulfonyl chloride (185 mg, 1.93 mmol) at 0 °C. The reaction mixture was stirred for 2 h at RT. TLC indicated the reaction was completed. A solution of sat. NaCl was added and extracted with DCM (3×20 mL). The collected organic phase was washed with sat. NaCl, dried with anhydrous sodium sulfate, filterated and concentrated to give the crude product **19d** (208 mg, 75%), which was used to the next step directly.

The compound **19d** (100 mg, 0.36 mmol), NaI (5 mg, 0.04 mmol) and 1 M dimethylamine tetrahydrofuran solution (2 mL, 2.00 mmol) was dissolved in MeCN (2 mL). The mixture was stirred at 60 °C for 3 h under sealed tube. TLC indicated the reaction was completed. The mixture was concentrated to give the crude product. The crude product was purified by column chromatography to give the product **19e** (51 mg, 63%). MS *m*/*z* 227.4 [M+H]⁺.

To a solution of **19e** (30 mg, 0.13 mmol) in DCM (1 mL) was added TFA (trifluoroacetic acid) (0.5 mL). The reaction mixture was stirred for 3 h at 40 °C. TLC indicated the reaction was completed. The mixture was concentrated, dissolved in MeCN (1 mL) and con.HCl (0.1 mL). The mixture was freeze dried to obtain 19f (21 mg, 97%). MS *m*/*z* 127.2 [M+H]⁺.

To a solution of chlorosulfonyl isocyanate (6 mg, 0.04 mmol) in DCM (1 mL) was added **19g** (8 mg, 0.04 mmol) at 0 °C. The reaction mixture was stirred for 10 min and **19f** (11 mg, 0.09 mmol) was added. TLC indicated the reaction was completed. The mixture was quenched with water and extracted with DCM (3×5 mL). The collected organic phase was washed with sat.NaCl, dried with anhydrous sodium sulfate, filterated and concentrated. The crude product was purified by reverse column chromatography to give **19** (0.7 mg, 4%). MS *m*/*z* 405.4 [M+H]⁺.

### Example 20: Preparation of Compound 20

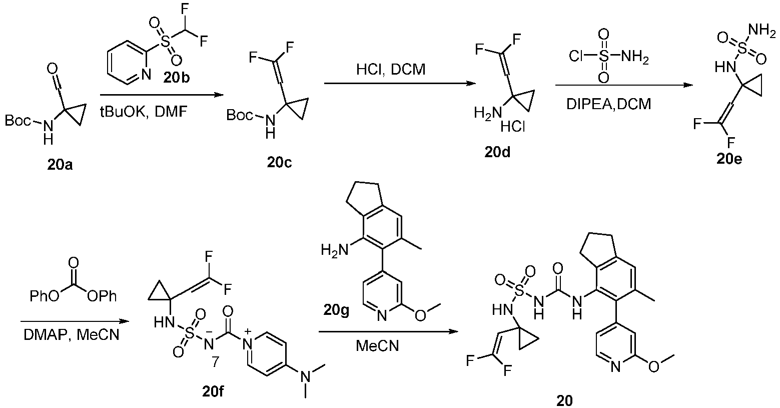

To a solution of tBuOK (163 mg, 1.46 mmol) in DMF (2 mL) was added **20a** (150 mg, 0.81 mmol) and 20b (140 mg, 0.73 mmol) at -50 °C under N₂. The reaction mixture was stirred for 1 h at -50 °C. TLC indicated the reaction was completed. Then Con. HCl (1 mL) was added. The reaction was stirred for 2 h at RT. Water (20 mL) was added and extracted with EA (3 × 30 mL). The collected organic phase was washed with sat.NaCl, dried with anhydrous sodium sulfate, filterated and concentrated to to give the crude product. The crude product was purified by column chromatography to give **20c** (63 mg, 36%). ¹H NMR (500 MHz, CDCl₃) δ 5.01 (s, 1H), 4.54 (d, *J* = 24.5 Hz, 1H), 1.44 (s, 9H), 1.02-0.96 (m, 2H), 0.94-0.89 (m, 2H) ppm.

To a solution of **20c** (45 mg, 0.29 mmol) in DCM (2 mL) was added 4 M HCl/dioxane solution (1 mL). The mixture was stirred overnight at RT. TLC indicated the reaction was completed. The mixture was concentrated to give the crude product **20d,** which was used to the next step directly. ¹H NMR (500 MHz, DMSO-*d*₆) δ 8.73 (s, 3H), 4.94 (dd, *J* = 1.4 Hz, *J* = 25.1 Hz, 1H), 1.22 (t, *J* = 6.3 Hz, 2H), 0.94 (t, *J* = 6.3 Hz, 2H) ppm.

To a solution of **20d** (45 mg, 0.29 mmol) in DCM (2 mL) was added diisopropylethylamine (148 mg, 1.15 mmol) and sulfamyl chloride (40 mg, 0.35 mmol) at 0 °C. The reaction mixture was stirred for 0.5 h at 0 °C. TLC indicated the reaction was completed. The mixture was concentrated to give the crude product. The crude product was purified by Pre-TLC to give the compound **20e** (27 mg, 47%).

The **20e** (27 mg, 0.14 mmol) and 4-Dimethylaminopyridine (33 mg, 0.27 mmol) was dissolved in MeCN (1 mL). The mixture was stirred at RT for 10 min. Then diphenyl carbonate (32 mg, 0.15 mmol) was added. The mixture was stirred at RT for 48 h to obtain a solution of **20f,** which was used to the next step directly.

### Compound 20g was synthesized according to WO20190211463

The compound **20g** (14 mg, 0.06 mmol) and **20f** (1 mL, reaction mixture) was dissolved in MeCN (1 mL). The mixture was stirred at 60 °C for 3 h. TLC indicated the reaction was completed. The mixture was concentrated to give the crude product. The crude product was purified by Pre-TLC (DCM: MeOH: Ammonia = 10:1:0.1) to give the compound **20** (3 mg, 11%). ¹H NMR (500 MHz, CD₃OD) δ 8.17 (d, *J* = 5.2 Hz, 1H), 7.12 (s, 1H), 6.78 (dd, *J* = 5.2, 1.3 Hz, 1H), 6.63 (s, 1H), 4.68 (dd, *J* = 24.6, 2.4 Hz, 1H), 3.93 (s, 3H), 2.95 (t, *J* = 7.4 Hz, 2H), 2.86-2.81 (m, 2H), 2.12-2.05 (m, 2H), 2.04 (s, 3H), 1.32-1.26 (m, 2H), 1.10-1.03 (m, 2H) ppm. MS *m*/*z* 479.5 [M+H]⁺ .

### Example 21: Preparation of Compound 21

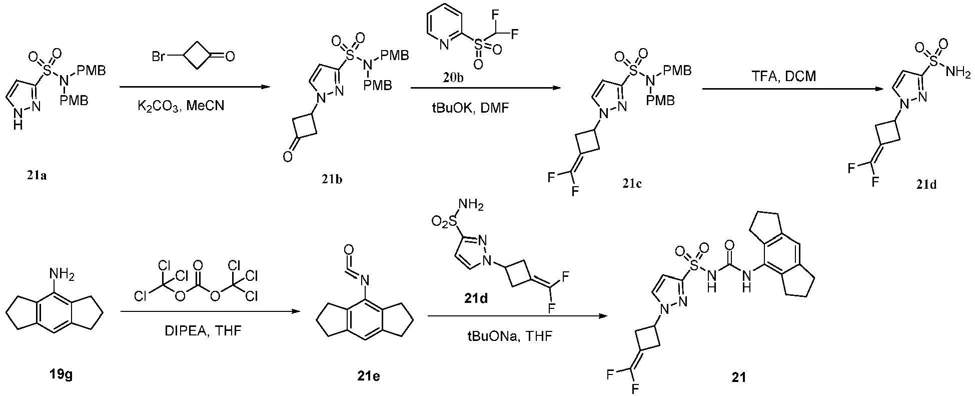

### Compound 21a was synthesized according to WO2019034690

To a solution of **21a** (700 mg, 1.81 mmol) in THF (14 mL) was added 3-bromo cyclobutanone (404 mg, 2.71 mmol) and K₂CO₃ (499 mg, 3.61mmol). The reaction mixture was stirred for 3 h. TLC indicated the reaction was completed. Water (30 mL) was added and the mixture was extracted with EA (3 × 30 mL). The collected organic phase was washed with sat. NaCl, dried with anhydrous sodium sulfate, filterated and concentrated to to give the crude product. The crude product was purified by column chromatography to give **21b** (505 mg, 61%). ¹H NMR (500 MHz, CDCl₃) δ 7.54 (d, *J* = 2.0 Hz, 1H), 7.12-7.07 (m, 4H), 6.80-6.76 (m, 4H), 6.72 (d, *J* = 1.9 Hz, 1H), 5.08-4.99 (m, 1H), 4.33 (s, 4H), 3.79 (s, 6H), 3.74-3.65 (m, 2H), 3.61-3.52 (m, 2H). MS *m*/*z* 456.3 [M+H]⁺.

A solution of tBuOK (111 mg, 0.99 mmol) in DMF (3 mL) was cooled to -50 °C under N₂, to which was then added a solution of **21b** (250 mg, 0.55 mmol) and **20b** (95 mg, 0.49 mmol) in DMF (2 mL). The reaction mixture was stirred for 1 h at -50 °C. TLC indicated the reaction was completed. Then Con. HCl (1 mL) was added. The reaction was stirred for 2 h at RT. Water (20 mL) was added and the mixture was extracted with EA (3 × 30 mL). The collected organic phase was washed with sat. NaCl, dried with anhydrous sodium sulfate, filterated and concentrated to to give the crude product. The crude product was purified by column chromatography to give **21c** (72 mg, 27%). MS *m*/*z* 490.4 [M+H]⁺.

To a solution of **21c** (72 mg, 0.15 mmol) in DCM (2 mL) was added TFA (2 mL). The reaction mixture was stirred overnight at RT. TLC indicated the reaction was completed. The mixture was concentrated to give the crudeproduct. The crude product was purifired by Pre-TLC to give the compound **21d** (10 mg, 27%).

To a solution of **19g** (15 mg, 0.09 mmol) in THF (1 mL) was added diisopropylethylamine (22 mg, 0.17 mmol) and triphosgene (13 mg, 0.04 mmol). The reaction mixture was stirred for 2.0 h at RT. TLC indicated the reaction was completed. The mixture was concentrated, Beated with petroleum ether, filter, and the filtrate was concentrated to give the crude product **21e,** which was used to the next step directly.

To a solution of **21d** (5 mg, 0.02 mmol) in THF (0.5 mL) was added tBuONa (2 mg, 0.02 mmol. The reaction mixture was stirred for 10 min at RT. Then **21e** (4 mg, 0.02 mmol) was added. The reaction mixture was stirred for another 2 h at RT. TLC indicated the reaction was completed. The mixture was concentrated to give the crude product. The crude product was purified by Pre-TLC to give the white solid **21** (8 mg, 89%). ¹H NMR (500 MHz, CD₃OD) δ 7.87 (d, *J* = 2.3 Hz, 1H), 6.96 (s, 1H), 6.83 (d, *J* = 2.3 Hz, 1H), 5.16-5.08 (m, 1H), 3.29-3.24 (m, 4H), 2.89-2.80 (m, 4H), 2.74-2.63 (m, 4H), 2.07-1.97 (m, 4H) ppm. MS *m*/*z* 449.3 [M+H]⁺.

### Example 22: Preparation of Compound 22

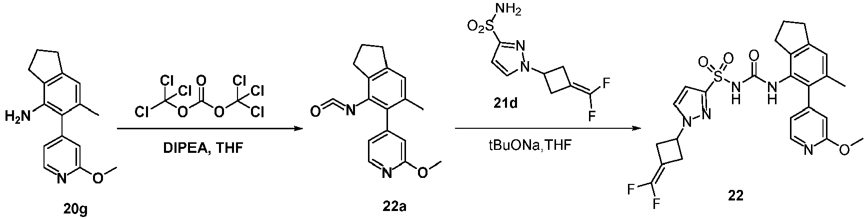

To a solution of **20g** (12 mg, 0.05 mmol) in THF (1 mL) was added diisopropylethylamine (12 mg, 0.10 mmol) and triphosgene (7 mg, 0.02 mmol). The reaction mixture was stirred for 2.0 h at RT. TLC indicated the reaction was completed. The mixture was concentrated, beated with petroleum ether, filtered, and the filtrate was concentrated to give the crude product **22a,** which was used in the next step directly.

To a solution of **21d** (6 mg, 0.02 mmol) in THF (0.5 mL) was added tBuONa (2 mg, 0.02 mmol. The reaction mixture was stirred for 10 min at RT. Then **22a** (7 mg, 0.02 mmol) was added. The reaction mixture was stirred for another 2 h at RT. TLC indicated the reaction was completed. The mixture was concentrated to give the crude product. The crude product was purified by Pre-TLC to give the white solid 22 (6 mg, 51%). ¹H NMR (500 MHz, CD₃OD) δ 8.09 (d, *J* = 5.1 Hz, 1H), 7.81 (d, *J* = 1.2 Hz, 1H), 7.08 (s, 1H), 6.72 (d, *J* = 5.2 Hz, 1H), 6.63 (d, *J* = 1.4 Hz, 1H), 6.59 (s, 1H), 5.13-5.06 (m, 1H), 3.92 (s, 3H), 3.28-3.24 (m, 4H), 2.92 (t, *J* = 7.4 Hz, 2H), 2.72-2.66 (m, 2H), 2.05-1.99 (m, 5H) ppm. MS *m*/*z* 530.5 [M+H]⁺.

### Example 23: Preparation of Compound 23

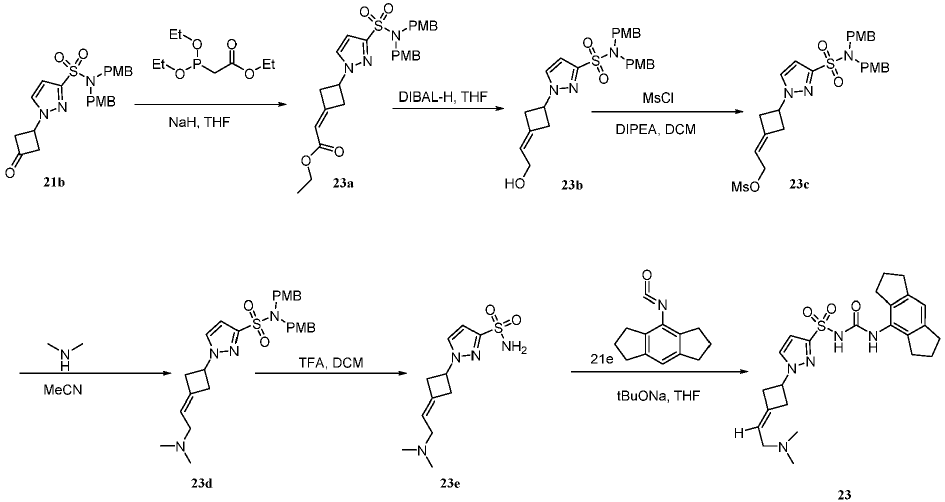

To a solution of NaH (26 mg, 60%, 0.64 mmol) in THF (2 mL) was added ethyl 2-(Diethoxyphosphoryl) acetate (143 mg, 0.64mmol) at - 78 °C under N₂. The reaction mixture was warmed to RT and stirred 0.5 h. Then **21b** (264 mg, 0.58 mmol) was added at 0 °C. The mixture was stirred overnight at RT. TLC indicated the reaction was completed. The mixture was quenched with sat.NaHCO₃ and extracted with EA (3×50 mL). The collected organic phase was washed with sat.NaCl, dried with anhydrous sodium sulfate, filterated and concentrated. The crude product was purified by column chromatography to give the **23a** (179 mg, 59%). MS *m*/*z* 526.5 [M+H]⁺.

To a solution of **23a** (179 mg, 0.34 mmol) in THF (5 mL) was added DIBAL-H (0.85 mL, 0.85 mmol) at -78 °C under N₂. After this, the mixture was stirred for 1 h at -78 °C. TLC indicated the reaction was completed. Warmed the temperature of the reaction solution to 0 °C, then water (0.05 mL), 15% sodium hydroxide aqueous solution (0.05 mL), and water (0.1 mL) was added successively. The reaction mixture was stirred at RT for 15 minutes. Anhydrous sodium sulfate was added and the reaction was stirred at RT for another 15 minutes. The mixture was filterated and concentrated. The crude product was purified by column chromatography to give the product **23b** (116 mg, 70%).

To a solution of **23b** (116 mg, 0.24 mmol) in DCM (3 mL) was added diisopropylethylamine (93 mg, 0.72 mmol) and methanesulfonyl chloride (55 mg, 0.48 mmol) at 0 °C. The reaction mixture was stirred for 2 h at RT. TLC indicated the reaction was completed. The mixture was concentrated to give the crude product **23c** (134 mg), which was used to the next step directly. The compound **23c** (134 mg, 0.24 mmol), NaI (2 mg, 0.01mmol) and 1 M dimethylamine tetrahydrofuran solution (2 mL, 2.00 mmol) was dissolved in MeCN (2 mL). The mixture was stirred at 60 °C for 3 h under sealed tube. TLC indicated the reaction was completed. The mixture was concentrated to give the crude product. The crude product was purified by column chromatography to give the product **23d** (54 mg, 44%). MS *m*/*z* 511.7 [M+H]⁺.

To a solution of **23d** (54 mg, 0.11mmol) in DCM (1 mL) was added TFA (1 mL). The reaction mixture was stirred for overnight at RT. TLC indicated the reaction was completed. The mixture was concentrated to give the crude. The crude was purified by Pre-TLC to give the white solid **23e** (16 mg, 56%).

To a solution of **23e** (8 mg, 0.03 mmol) in THF (1 mL) was added tBuONa (3 mg, 0.03 mmol). The reaction mixture was stirred for 10 min at RT. Then **21e** (6 mg, 0.03 mmol) was added. The reaction mixture was stirred for another 2 h at RT. TLC indicated the reaction was completed. The mixture was concentrated to give the crude product. The crude product was purified by Pre-TLC to give the white solid **23** (8 mg, 56%).¹H NMR (500 MHz, CD₃OD) δ 7.84 (d, *J* = 2.4 Hz, 1H), 6.93 (s, 1H), 6.79 (d, *J* = 2.4 Hz, 1H), 5.54-5.48 (m, 1H), 5.10-5.04 (m, 1H), 3.62 (d, *J* = 7.8 Hz, 2H), 3.43-3.33 (m, 4H), 2.86-2.81 (m, 10H), 2.67 (t, *J* = 7.3 Hz, 4H), 2.01 (p, *J* = 7.4 Hz, 4H) ppm. MS m/z 470.5 [M+H]⁺.

### Example 24: Preparation of Compound 24

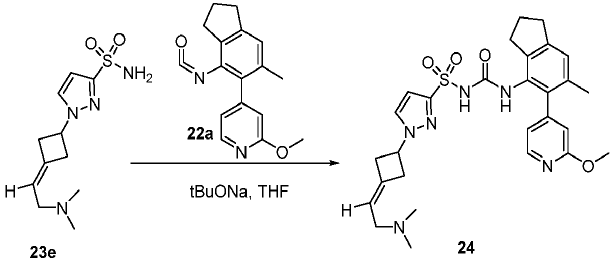

To a solution of **23e** (8 mg, 0.03 mmol) in THF (1 mL) was added tBuONa (3 mg, 0.03 mmol). The reaction mixture was stirred for 10 min at RT. Then **22a** (8 mg, 0.03 mmol) was added. The reaction mixture was stirred for another 2 h at RT. TLC indicated the reaction was completed. The mixture was concentrated to give the crude product. The crude product was purified by Pre-TLC to give the white solid **24** (8 mg, 52%). ¹H NMR (500 MHz, CD₃OD) δ 8.08 (d, *J* = 5.0 Hz, 1H), 7.82 (d, *J* = 2.3 Hz, 1H), 7.09 (s, 1H), 6.71 (d, *J* = 5.0 Hz, 1H), 6.61 (d, *J* = 2.3 Hz, 1H), 6.58 (s, 1H), 5.55-5.49 (m, 1H), 5.11-5.04 (m, 1H), 3.92 (s, 3H), 3.65 (d, *J* = 7.9 Hz, 2H), 3.43-3.30 (m, 4H), 2.92 (t, *J* = 7.4 Hz, 2H), 2.86 (s, 6H), 2.72-2.65 (m, 2H), 2.07-1.99 (m, 5H) ppm. MS *m*/*z* 551.6 [M+H]⁺.

### Example 25: Study of the compound's inhibitory activity of NLRP3

### Step 1: IL1 β induction

60 µL/well polylysine was added to 96-well cell culture plate and incubated at 37 °C for 30 min. Discard the solution in the plate and wash the cell culture plate twice with PBS. THP1 (50000 cells/well/95 µL) was planted in cell culture plates, 5 µL of PMA was added to each well (the final concentration was 50 ng/mL), and cultured overnight in a thermostat incubator at 37°C. The supernatant was gently sucked out, the cell culture plate was washed with PBS twice, and 80 µL cell culture medium without FBS, followed by 5 µL/ well LPS (final concentration 500 ng/mL) was added to each well. The plate was cultured in a thermostat incubator at 37 °C for 3 hours. The compound (10 mM storage solution, DMSO dissolved) was diluted accordingly with 100% DMSO. Add 2 µL diluted compound into 98 µL medium and mix well. 5 µL/well diluted compound was added into the cell plate and cultured in a thermostat incubator at 37 °C for 1 hour. Then 10 µL/well nigericin (final concentration 10 µg/mL) was added to the cell plate and cultured in a thermostat incubator at 37°C for 0.5 hours. The cell supernatant was collected into a new cell plate and frozen at -80°C for future use.

### Step 2: IL1 β detection

IL1β antibody was diluted 60 times with coating buffer, and 100 µL of diluted antibody was added to each well of ELISA plate, and incubated at 4°C overnight. Discard the liquid in the ELISA plate and wash it 4 times with eluent. Add 300 µL/well reagent diluent and incubate the ELISA plate at room temperature for 1.5 hours. Wash the plate with eluent for four times. Then 100 µL/well samples were added to ELISA plate coated with IL1β and incubated at room temperature for 2 h. Wash the plate with eluent for four times. Each well was added with 100 µL detection antibody and incubated at 37 °C for 2 h. Wash the plate with eluent for four times. Add 100 µL of HRP labeled secondary antibody to each well. Incubate at 37 °C for 1 hour. Wash the plate with eluent for four times. Add 100 µL of A+B substrate per well. Incubate at 37°C for 30 minutes. Add 100 µL STOP solution per well. Gently shake the plate for a few seconds. Read the absorption value at 450 nm on the EnVision multifunction reader and calculate the Inhibitory rate according to the following formula: Inhibition% = (Ave_H-Sample)/(Ave_H-Ave_L). Among them, Ave_H represents the average value of DMSO well, Sample represents the average value of compound well, and Ave_L represents the average value of 10 µM positive control well. The log value of concentration was taken as the X-axis and the percentage inhibitory rate as the Y-axis. The dose-effect curve was fitted with log(inhibitor) vs. response-Variable slope from software GraphPad Prism 5 to obtain the IC50 value of each compound. Calculation formula: Y=Bottom + (Top-Bottom)/(1+10^((LogIC50-X)*HillSlope)).

**Table 1: Inhibition of NLRP3 activity by compounds at 300 nM**

| **Compounds** | **Inhibition at 300 nM** | **IC₅₀ (nM)** |
|---|---|---|
| 1 | >50% | |
| 6* | >50% | |
| 7* | >50% | |
| 8* | >50% | |
| 13* | >50% | |
| 14* | >50% | |
| 22 | >50% | <25 |
| MCC950* | >50% | <100 |

*The representative compounds are sodium salt.

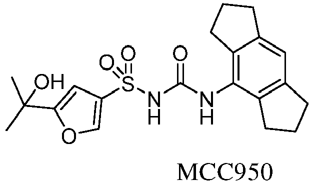

### Example 26 Pharmacokinetic study in rats

Instruments: XEVO TQ-S LC/MS instrument produced by Waters. All measurement data is collected and processed by Masslynx V4.1 software, and the data is calculated and processed by Microsoft Excel. Using WinNonLin 8.0 software, the statistical moment method was used to calculate the pharmacokinetic parameters. Mainly include kinetic parameters Tmax, T1/2, Cmax, AUClast etc. Column: ACQUITY UPLC BEH C18 (2.1 mm × 50 mm, 1.7 µm); column temperature 40 °C; mobile phase A is water (0.1% formic acid), mobile phase B is acetonitrile, flow rate is 0.350 ml/min, gradient elution is adopted, the elution gradient is 0.50 min: 10% B; 1.50 min: 90% B; 2.50 min: 90% B; 2.51 min: 10% B; 3.50 min: stop. Injection volume: 1 µL.

Animals: 3 male SD rats with a weight range of 200-220 g. After purchase, they will be kept in the laboratory of the Experimental Animal Center for 2 days and then used. They will be fasted for 12 hours predose and 4 hours after dosing. Drinking water is free during the test. After the rats were gavage, blood samples were taken according to the established time point.

Solvent: 0.4% ethanol + 0.4% Tween80 + 99.2% (0.5% methylcellulose M450). Preparation of the solution for intragastrical administration: accurately weigh the compound, add it to the solvent, and use ultrasound at room temperature for 5 minutes to completely dissolve the drug, and prepare a 0.3 mg/mL medicinal solution.

Pharmaceutical samples: Generally, multiple samples with similar structures (with a molecular weight difference of more than 2 units) are taken, accurately weighed, and administered together (cassette PK). In this way, multiple compounds can be screened at the same time and their oral absorption rates can be compared. A single administration was also used to study the pharmacokinetics of the drug sample in rats.

After intragastrical administration, blood was taken from the orbit at 0.25, 0.5, 1, 2, 4, 8, 10 and 24 hours. Then take 50 µL of the plasma sample, add 200 µL of acetonitrile (including internal standard control verapamil 2 ng/mL), vortex for 3 min and centrifuge at 20000 rcf , 4 °C for 10 min. The supernatant plasma was used for LC-MS/ MS analysis.

Accurately weigh the compounds to prepare different concentrations, perform quantitative analysis on mass spectrometry to establish a standard curve, and then test the concentration of the above-mentioned compound in the plasma to obtain the compound concentration at different time points. All measurement data are collected and processed by relevant software, and the statistical moment method is used to calculate the pharmacokinetic parameters (mainly including kinetic parameters Tmax, T1/2, Cmax, AUClast etc). The kinetic parameters of some representative compounds are shown in Table 2.

**Table 2: Pharmacokinetic parameters of the compounds in SD rats**

| Compound | Oral dosage | Tₘₐₓ (h) | T_{1/2} (h) | Cₘₐₓ (ng/mL) | AUC₍₀₋₂₄₎(ng/mL*h) |
|---|---|---|---|---|---|
| **22** | 3 mg/kg | 1.00 | 5.60 | 1529.58 | 6708.67 |

All documents mentioned in the present application are hereby incorporated by reference in their entirety.-In addition, it should be understood that various modifications and changes may be made by those skilled in the art in the form of the appended claims.

## Claims

1. A compound of formula (I), or optical isomers, pharmaceutically acceptable salts, prodrugs, deuterated derivatives, hydrates, ro solvates thereof: 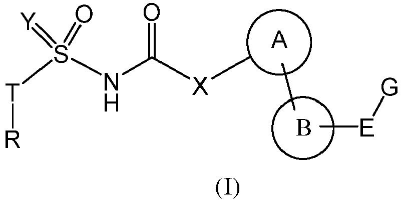 wherein:
ring A is selected from the group consisting of substituted or unsubstituted 8-15 membered bicyclic or tricyclic fused ring systems; wherein, the "substituted" means the hydrogen atoms on the group are substituted by one or more R^{e}; the bicyclic or tricyclic fused ring system comprises at least one aromatic ring and one or two saturated or unsaturated rings fused with the aromatic ring, and the connecting site of ring A and X locating on the aromatic ring;
ring B is selected from the group consisting of none, substituted or unsubstituted aryl, substituted or unsubstituted 5-12 membered heterocycle(including partially unsaturated or saturated heterocycle), or substituted or unsubstituted heteroaryl; wherein, the "substituted" means the hydrogen atoms on the group are substituted by one or more R^{f}; and when B is none, E and G are absent;
X is selected from -NR⁵-, -CR⁶R⁷-;
Y is selected from O, -NR⁵-;
T is selected from chemical bond, -NR⁵-, -(CR^{a}R^{b})₁₋₂-, C₃₋₆cycloalkyl, 3-8 membered heterocyclyl, aryl, and heteroaryl;
R is selected from the group consisting of hydrogen, C₁₋₄ alkyl, C₁₋₄haloalkyl, C₂₋₄ alkenyl, C₂₋₄alkynyl, C₃₋₈cycloalkyl, 3-8membered heterocyclyl, aryl, heteroaryl, and NR⁸R⁹; wherein, the alkyl, alkenyl, alkynyl, cycloalkyl, heterocyclyl, aryl, or heteroaryl groups are optionally substituted by one or more substituents selected from the group consisting of halogen, C₁₋₄ alkyl, C₁₋₄haloalkyl, C₂₋₄haloalkenyl, C₁₋₄alkoxy, C₁₋₄haloalkoxy, NR⁸R⁹, CN, NO₂, SR^{h}, C (O) R^{t}, C (O) OR^{h}, C(O)NR^{h}R^{h}, NR^{h}C(O)R^{t}, NRhS(O)₂R^{t}, and S(O)₂R^{t}; or the cycloalkyl or heterocyclic group is substituted by =M, where M is selected from O or CR¹⁰R¹¹;
E is selected from the group consisting of chemical bond, -O-, -O(CR^{a}R^{b})₁₋₂-, -(CR^{a}R^{b})₁₋₂O-, -S-, -S(CR^{a}R^{b})₁₋₂-, -(CR^{a}R^{b})₁₋₂S-, -NR⁵-, -(CR^{a}R^{b})₁₋₂NR⁵-, -NR⁵(CR^{a}R^{b})₁₋₂-, C₁₋₂alkylene, -C=C-, -C=C-, and C₃₋₆cycloalkyl;
G is selected from the group consisting of hydrogen, C₁₋₄ alkyl, C₁₋₄haloalkyl, C₂₋₄ alkenyl, C₂₋₄alkynyl, saturated C₃₋₈cycloalkyl, unsaturated C₃₋₈cycloalkyl, saturated 3-12 membered heterocyclyl, unsaturated 3-12 membered heterocyclyl, aryl, heteroaryl, and NR⁸R⁹; wherein, the cycloalkyl, heterocyclyl, aryl, or heteroaryl groups are optionally substituted by one or more substituents selected from the group consisting of halogen, C₁₋₄ alkyl, C₁₋₄haloalkyl, C₂₋₄haloalkenyl, C₁₋₄alkoxy, C₁₋₄haloalkoxy, NR⁸R⁹, CN, NO₂, SR^{h}, C(O)R^{t}, C(O)OR^{h}, C(O)NR^{h}R^{h}, NR^{h}C(O)R^{t}, NR^{h}S(O)₂R^{t}, and S(O)₂R^{t};
R⁵ is selected from the group consisting of H, C₁₋₄ alkyl, and C₃₋₆ cycloalkyl;
R⁶ and R⁷ are independently selected from the group consisting of hydrogen, halogen, C₁₋₄ alkyl, and C₃₋₆cycloalkyl; or R⁶ and R⁷ together with their connected carbon atoms form a C₃₋₆cycloalkyl, or a 4-6 membered heterocyclylhaving 1 or 2 heteroatoms selected from N, O, S;
R⁸ and R⁹ are independently selected from the group consisting of hydrogen, C₁₋₄ alkyl, C₃₋₆cycloalkyl, and 4-8 membered heterocyclyl; the cycloalkyl or heterocyclyl is optionally substituted by "=M", wherein M is selected from O or CR¹⁰R¹¹; or R⁸ and R⁹ together with their connected nitrogen atoms form a 4-8 membered heterocyclyl, wherein the heterocyclyl contains 1 or 2 N atoms and 0 or 1 heteroatom selected from O, S;
and when ring A is a tricyclic fused ring system, and R is not an unsaturated C₃₋₈cycloalkyl (nonaromatic) or unsaturated 3-8 membered heterocyclyl (nonaromatic), R is a C₃₋₈cycloalkyl or 3-8 membered heterocyclyl, and R is at least substituted by one =M; or R is a C₃₋₈cycloalkyl or 3-8 membered heterocyclyl, and T is - NR⁵-, and R is at least substituted by one substituent selected from the group consisting of fluorine, C₁₋₄ fluoroalkyl, and C₂₋₄fluoroalkenyl;
and when ring A is a bicyclic fused ring system, and G is not an unsaturated 3-12 membered heterocyclyl (nonaromatic), saturated 3-12 membered spiro-heterocyclyl, saturated 3-12 membered fused-heterocyclyl, and saturated 3-12 membered bridge-heterocyclyl, R is C₃₋₈cycloalkyl or 3-8 membered heterocyclyl, and R is at least substituted by one =M; or R is C₃₋₈cycloalkyl or 3-8 membered heterocyclyl, and T is - NR⁵-, and R is at least substituted by one substituent selected from the group consisting of fluorine, C₁₋₄ fluoroalkyl, and C₂₋₄fluoroalkenyl;
R¹⁰ and R¹¹ are independently selected from the group consisting of hydrogen, deuterium, halogen, and C₁₋₄ alkyl; wherein, the alkyl is optionally substituted by one or more substituents selected from the group consisting of hydrogen, halogen, C₁₋₄ alkyl, C₁₋₄haloalkyl, C₁₋₄alkoxy, C₁₋₄ haloalkoxy, NR⁸R⁹, C₃₋₈cycloalkyl, 3-8 membered heterocyclyl, aryl, and heteroaryl; or R¹⁰ and R¹¹ together with their connected carbon atoms form a 3-6 membered cycloalkyl, or a 4-8 membered heterocyclyl having 1 or 2 heteroatoms selected from N, O, and S;
R^{a} and R^{b} are independently selected from the group consisting of H, halogen, C₁₋₄ alkyl, or C₃₋₆ cycloalkyl;
R^{e} and R^{f} are independently selected from the group consisting of deuterium, halogen, C₁₋₄ alkyl, C₁₋₄haloalkyl, C₂₋₄ alkenyl, C₂₋₄alkynyl, C₃₋₆ cycloalkyl, 4-8 membered heterocyclyl, aryl, heteroaryl, CN, NO₂, OR^{h}, SR^{h}, NR^{h}R^{h}, C(O)R^{t}, C(O)NR^{h}R^{h};
R¹ is C₁₋₄ alkyl, C₂₋₄ alkenyl, C₂₋₄alkynyl, C₃₋₈cycloalkyl, 4-8 membered heterocyclyl, aryl, or heteroaryl;
each R^{h} is independently hydrogen or C₁₋₄ alkyl; or two R^{h} together with their connected nitrogen atoms form a 3-8 membered heterocyclyl having 1 or 2 N atoms and 0 or 1 heteroatom selected from O and S;
wherein, each of the abovementioned alkyl, alkenyl, alkynyl, cycloalkyl, heterocyclyl, aryl, and heteroaryl is optionally and independently substituted by 1-3 substituents selected from the group consisting of halogen, C₁₋₄ alkyl, C₁₋₄haloalkyl, C₂₋₄ alkenyl, C₂₋₄alkynyl, C₃₋scycloalkyl, 3-8 membered heterocyclyl, aryl, heteroaryl, CN, NO₂, OR^{h}, SR^{h}, NR^{h}R^{h}, C(O)R^{t}, C(O)OR^{h}, C(O)NR^{h}R^{h}, NR^{h}C(O)R^{t}, NR^{h}S(O)₂R^{t}, and S(O)₂R^{t}, the premise is that the resulting chemical structure is stable and meaningful; wherein, R^{h} and R'are as described above;
unless otherwise specified, the aryl described above is aromatic group containing 6-12 carbon atoms; the heteroaryl is 5-15 membered (preferably 5-12 membered) heteroaromatic groups.

2. The compound of claim 1, or optical isomers, pharmaceutically acceptable salts, prodrugs, deuterated derivatives, hydrates, or solvates thereof, wherein, R is selected from C₃₋₈cycloalkyl or 3-8 membered heterocyclyl; wherein, the cycloalkyl or heterocyclyl is optionally substituted by one or more substituents selected from the group consisting of halogen, C₁₋₄ alkyl, C₁₋₄ haloalkyl, C₂₋₄haloalkenyl, C₁₋₄alkoxy, C₁₋₄haloalkoxy, NR⁸R⁹, and =M, wherein, M is selected from O or CR¹⁰R¹¹;
A, B, E, G, X, Y, T, R⁸, R⁹, R¹⁰, R¹¹ are as described in claim 1.

3. The compound of claim 2, or optical isomers, pharmaceutically acceptable salts, prodrugs, deuterated derivatives, hydrates, or solvates thereof, wherein, R is selected from C₃₋₈cycloalkyl or 3-8 membered heterocyclyl; wherein, the cycloalkyl or heterocyclyl is at least substituted by one =M, wherein M is CR¹⁰R¹¹; wherein, R¹⁰ and R¹¹ are as described in claim 1.

4. The compound of any one of claims 1-3, or optical isomers, pharmaceutically acceptable salts, prodrugs, deuterated derivatives, hydrates, or solvates thereof, wherein 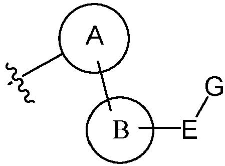 is selected from 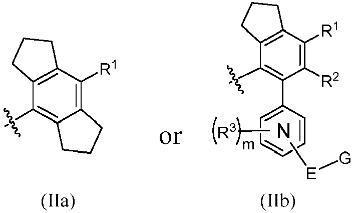
" 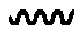 " represents the connecting site of the above structural fragments of formula (IIa) or (IIb) with the rest moiety of formula(I);
R¹ and R² are independently selected from the group consisting of hydrogen, deuterium, halogen, C₁₋₄ alkyl, C₁₋₄haloalkyl, C₁₋₄alkoxy, and C₁₋₄haloalkoxy;
each R³ is independently selected from the group consisting of hydrogen, deuterium, halogen, and C₁₋₄alkyl;
m is 0, 1, 2 or 3; 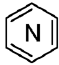 is pyridine, pyrimidine, or pyridazine;
E is selected from the group consisting of chemical bond, -O-, -O(CR^{a}R^{b})₁₋₂-, -(CR^{a}R^{b})₁₋₂O-, -S-, -S(CR^{a}R^{b})₁₋₂-, -(CR^{a}R^{b})₁₋₂, -NR⁵-, -(CR^{a}R^{b})₁₋₂NR⁵-, -NR⁵(CR^{a}R^{b})₁₋₂-, C₁₋₂alkylene, -C=C-, and -C=C-; wherein, R^{a} and R^{b} are independently selected from hydrogen and C₁₋₄alkyl;
G is selected from unsaturated C₃₋₈cycloalkyl or unsaturated 3-12 membered heterocyclyl; wherein the cycloalkyl or heterocyclyl is optionally substituted by one or more substituents selected from the group consisting of halogen, C₁₋₄ alkyl, C₁₋₄haloalkyl, C₂₋₄haloalkenyl, C₁₋₄alkoxy, C₁₋₄haloalkoxy, NR⁸R⁹, CN, NO₂, SR^{h}, C(O)R^{t}, C(O)OR^{h}, C(O)NR^{h}R^{h}, NR^{h}C(O)R^{t}, NR^{h}S(O)₂R^{t}, and S(O)₂R^{t};
R⁵, R⁸, R⁹, R^{h} and R'are as described in claim 1.

5. The compound of any one of claims 1-4, or optical isomers, pharmaceuticallyacceptable salts, prodrugs, deuterated derivatives, hydrates, or solvates thereof, wherein, formula(I) has the structure of formula (III): 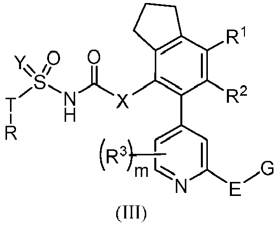
R¹ and R² are independently selected from the group consisting of hydrogen, deuterium, halogen, C₁₋₄ alkyl, C₁₋₄haloalkyl, C₁₋₄alkoxy, and C₁₋₄haloalkoxy;
each R³ is independently selected from the group consisting of hydrogen, deuterium, halogen, and C₁₋₄alkyl;
m is 0, 1 or 2;
E is selected from the group consisting of chemical bond, -O-, -O(CR^{a}R^{b})₁₋₂-, -(CR^{a}R^{b})₁₋₂O-, -S-, -S(CR^{a}R^{b})₁₋₂-, -(CR^{a}R^{b})₁₋₂S-, -NR⁵-, -(CR^{a}R^{b})₁₋₂NR⁵-, -NR⁵(CR^{a}R^{b})₁₋₂-, C₁₋₂alkylene, -C=C-, -C=C-; wherein, R^{a} and R^{b} are independently selected from hydrogen and C₁₋₄alkyl;
G is selected from unsaturated C₃₋₈cycloalkyl or unsaturated 3-12 membered heterocyclyl; wherein, the cycloalkyl or heterocyclyl is optionally substituted by one or more substituents selected from the group consisting of halogen, C₁₋₄ alkyl, C₁₋₄haloalkyl, C₂₋₄haloalkenyl, C₁₋₄alkoxy, C₁₋₄haloalkoxy, NR⁸R⁹, CN, NO₂, SR^{h}, C(O)R^{t}, C(O)OR^{h}, C(O)NR^{h}R^{h}, NR^{h}C(O)R^{t}, NR^{h}S(O)₂R^{t}, and S(O)₂R^{t};
X, Y, T, R, R⁵, R⁸, R⁹, R^{h} and R^{t} are as described in claim 1.

6. The compound of claim 5, or optical isomers, pharmaceutically acceptable salts, prodrugs, deuterated derivatives, hydrates, or solvates thereof, wherein,
E is selected from the group consisting of chemical bond, -O-, -O(CR^{a}R^{b})₁₋₂-, -(CR^{a}R^{b})₁₋₂O-, -NR⁵-, - (CR^{a}R^{b})₁₋₂NR⁵-, - NR⁵(CR^{a}R^{b})₁₋₂-, and -C=C-; wherein, R^{a} and R^{b} are independently selected from hydrogen and C₁₋₄alkyl.

7. The compound of any one of claims 1-6, or optical isomers, pharmaceutically acceptable salts, prodrugs, deuterated derivatives, hydrates, or solvates thereof, wherein, formula(I) has the structure of formula (IV): 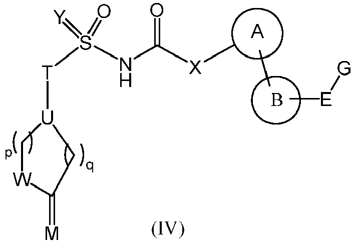
M is selected from CR¹⁰R¹¹; wherein, R¹⁰ and R¹¹ are as described in claim 1;
U is selected from N or CR¹²; wherein, R¹² is selected from hydrogen, halogen and C₁₋₄alkyl;
W is selected from chemical bond, -NR¹³(CR^{c}R^{d})₁₋₂-, and -O(CR^{c}R^{d})₁₋₂-; wherein, R¹³ is selected from hydrogen, C₁₋₄alkyl, C₃₋₆cycloalkyl, 3-6 membered heterocyclyl, aryl, heteroaryl, C(O)R^{t}, and S(O)₂R^{t}; R^{c} and R^{d} are independently selected from hydrogen and C₁₋₄alkyl;
p and q are independently selected from 0, 1, 2, 3, 4, 5 and 6; the premise is that p and q are not 0 at the same time;
A, B, E, G, X, Y, T and R'are as described in claim 1.

8. The compound of claim 7, or optical isomers, pharmaceutically acceptable salts, prodrugs, deuterated derivatives, hydrates, or solvates thereof, wherein, formula(I) has the structure of formula (Va) or formula (Vb): 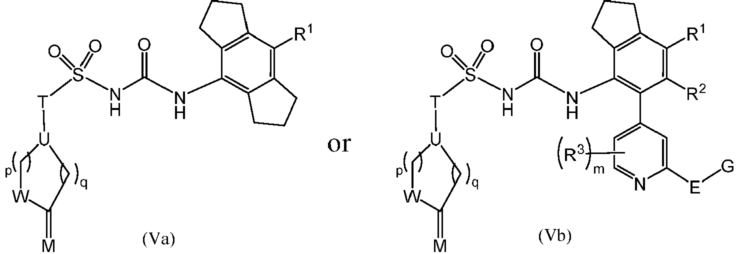
T is selected from chemical bond, -NR⁵-, aryl, and heteroaryl;
M, U, W, p, and q are as described in claim 7;
R¹, R², R³, and m are as described in claim 4; and
E, G, and R⁵ are as described in claim 1.

9. The compound of claim 7, or optical isomers, pharmaceutically acceptable salts, prodrugs, deuterated derivatives, hydrates, or solvates thereof, wherein, formula(I) has the structure of formula (VIa) or formula (VIb): 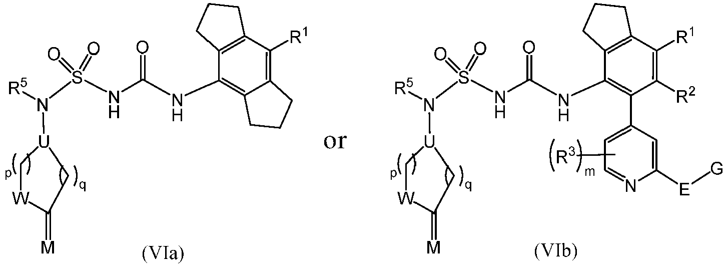
R⁵ is selected from hydrogen and C₁₋₄alkyl;
U is selected from CR¹²; wherein, R¹² is selected from hydrogen, and C₁₋₄alkyl;
M, W, p, and q are as described in claim 7;
R¹, R², R³, and m are as described in claim 4; and
E, and G are as described in claim 1.

10. The compound of claim 7, or optical isomers, pharmaceutically acceptable salts, prodrugs, deuterated derivatives, hydrates, or solvates thereof, wherein, formula(I) has the structure of formula (VIIa) or formula (VIIb): 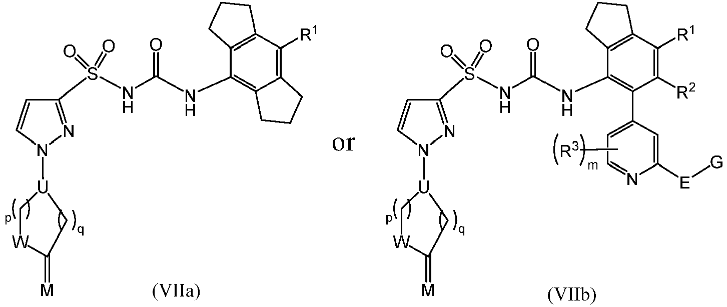
U is selected from CR¹²; wherein, R¹² is selected from hydrogen, and C₁₋₄alkyl;
M, W, p, and q are as described in claim 7;
R¹, R², R³, and m are as described in claim 4; and
E, and G are as described in claim 1.

11. The compound of claim 8, or optical isomers, pharmaceutically acceptable salts, prodrugs, deuterated derivatives, hydrates, or solvates thereof, wherein, formula(I) has the structure of formula (VIIIa) or formula (VIIIb): 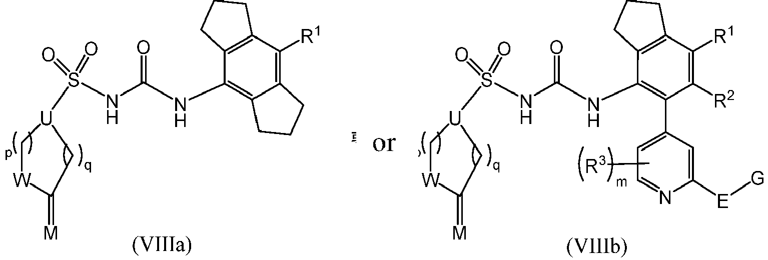 M, U, W, R¹, R², R³, E, G, m, p, and q are as described in claim 8.

12. The compound of claim 1, or optical isomers, pharmaceutically acceptable salts, prodrugs, deuterated derivatives, hydrates, or solvates thereof, wherein, formula(I) has the structure of formula (IXa) or formula (IXb): 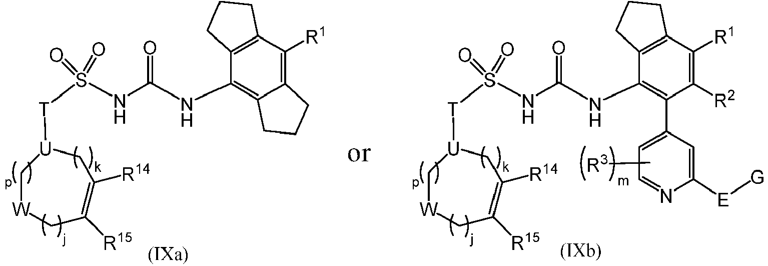
k and j are independently 0, 1 or 2;
R¹⁴ and R¹⁵ are independently selected from the group consisting of H, halogen, C₁₋₄ alkyl, and C₃₋₆ cycloalkyl; and
T, U, W, R¹, R², R³, E, G, m, and p are as described in claim 8.

13. The compound of claim 1, or optical isomers, pharmaceutically acceptable salts, prodrugs, deuterated derivatives, hydrates, and solvates thereof, wherein, formula(I) has the structure of formula (X): 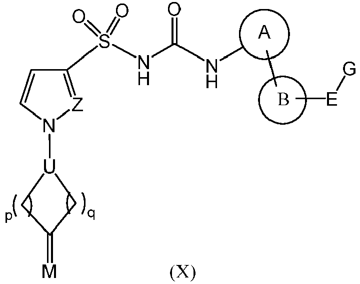
Z is selected from N and CR¹⁶; wherein, R¹⁶ is selected from hydrogen, halogen and C₁₋₄alkyl;
p and q are independently selected from 0, 1, 2, 3, 4, 5, and 6;
U is selected from CR¹²; wherein, R¹² is selected from hydrogen, and C₁₋₄alkyl;
M is selected from CR¹⁰R¹¹; wherein, R¹⁰ and R¹¹ are as described in claim 1; and
A, B, E and G are as described in claim 1.

14. The compound of claim 1, or optical isomers, pharmaceutically acceptable salts, prodrugs, deuterated derivatives, hydrates, or solvates thereof, wherein, formula(I) has the structure of formula (XIa) or formula (XIb): 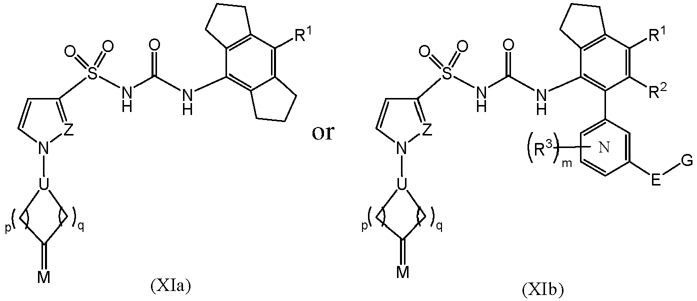 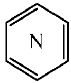 is selected from pyridine, pyrimidine, and pyridazine;
Z, U, M, p, and q are as described in claim 13;
R¹, R², R³, and m are as described in claim 4; and
E, and G are as described in claim 1.

15. The compound of claim 1, or optical isomers, pharmaceutically acceptable salts, prodrugs, deuterated derivatives, hydrates, or solvates thereof, wherein, formula(I) has the structure of formula (XII): 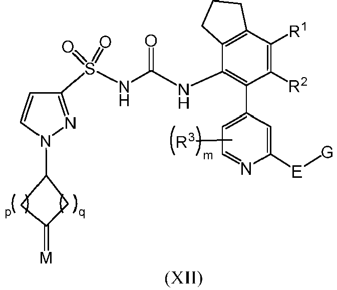
p and q are independently selected from0, 1, 2, 3, 4, 5, and 6;
M is selected from CR¹⁰R¹¹; wherein, R¹⁰ and R¹¹ are independently selected from the group consisting of hydrogen, fluorine, and C₁₋₂ alkyl; wherein, the alkyl is optionally substituted by one or more substituents selected from the group consisting of hydrogen, halogen, C₁₋₄ alkyl, C₁₋₄haloalkyl, C₁₋₄alkoxy, C₁₋₄ haloalkoxy, NR⁸R⁹, C₃₋₈cycloalkyl, and 3-8 membered heterocyclyl;
R¹ and R² are independently selected from hydrogen, halogen, C₁₋₄ alkyl, C₁₋₄haloalkyl, C₁₋₄alkoxy, and C₁₋₄haloalkoxy;
each R³ is independently selected from hydrogen, halogen, and C₁₋₄alkyl;
m is 0, 1 or 2;
E is selected from the group consisting of chemical bond, -O-, -O(CR^{a}R^{b})₁₋₂-, -(CR^{a}R^{b})₁₋₂O-, - S-, -NR⁵-, - (CR^{a}R^{b})₁₋₂NR⁵-, - NR⁵(CR^{a}R^{b})₁₋₂-, C₁₋₂alkylene, -C=C-, and C₃₋₆cycloalkyl; wherein, R^{a} and R^{b} are independently selected from group consisting hydrogen and C₁₋₄ alkyl; R⁵ is selected from the group consisting of H, C₁₋₄ alkyl, and C₃₋₆cycloalkyl;
G is selected from the group consisting of hydrogen, C₁₋₄ alkyl, C₁₋₄haloalkyl, C₂₋₄ alkenyl, C₂₋₄alkynyl, saturated C₃₋₈cycloalkyl, unsaturated C₃₋₈cycloalkyl, saturated 3-12 membered heterocyclyl, unsaturated 3-12 membered heterocyclyl, aryl, heteroaryl, and NR⁸R⁹; wherein, the cycloalkyl, heterocyclyl, aryl, or heteroaryl are optionally substituted by one or more substituents selected from the group consisting of halogen, C₁₋₄ alkyl, C₁₋₄haloalkyl, C₁₋₄alkoxy, C₁₋₄haloalkoxy, NR⁸R⁹, CN, C(O)R^{t}, and S(O)₂R^{t}; wherein, R^{t} is C₁₋₄ alkyl, C₂₋₄ alkenyl, C₂₋₄alkynyl, C₃₋₈cycloalkyl, 4-8-membered heterocyclyl, aryl, or heteroaryl;
R⁸ and R⁹ are independently selected from the group consisting of H, C₁₋₄ alkyl, C₃₋₆cycloalkyl, and 4-8 membered heterocyclyl;

16. The compound of claim 1, wherein, the compound of formula(I) is selected from group consisting of 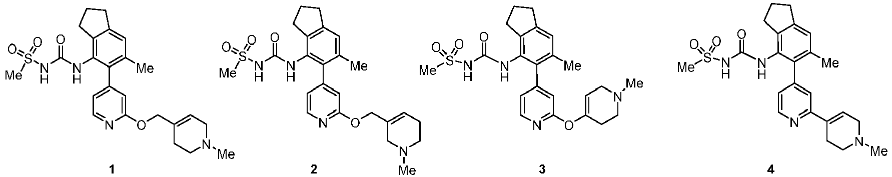 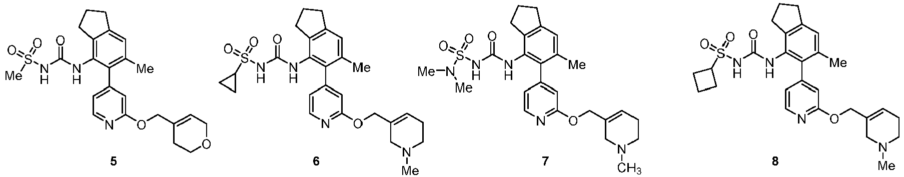 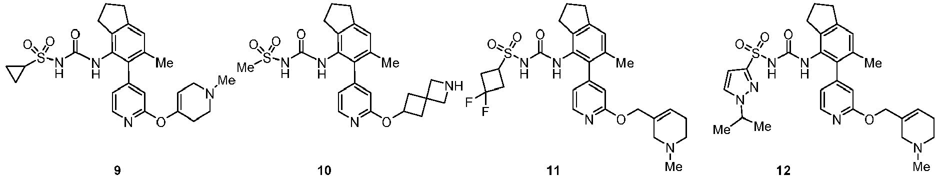 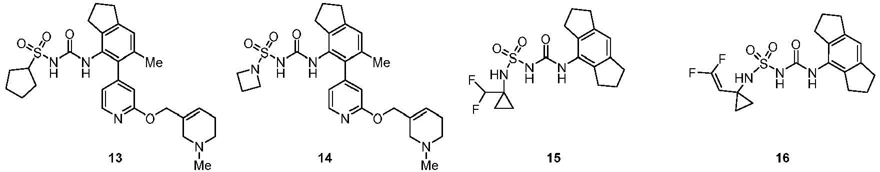 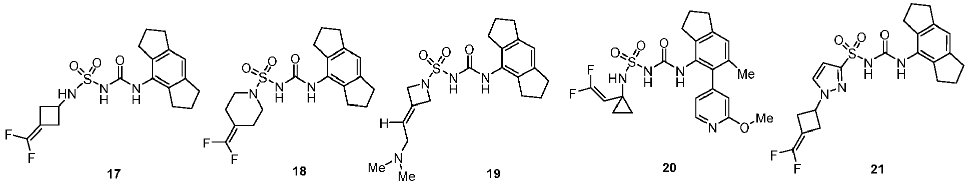 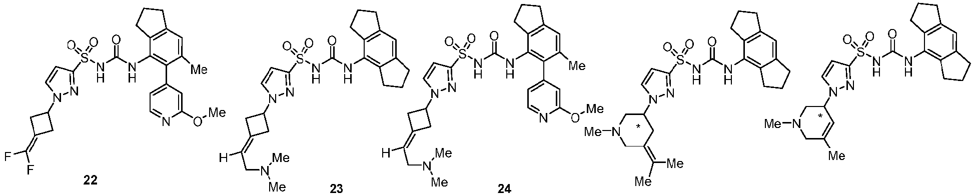 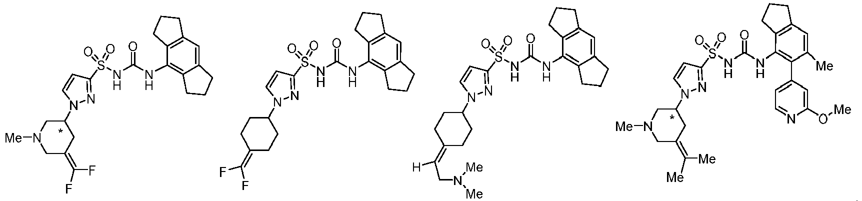 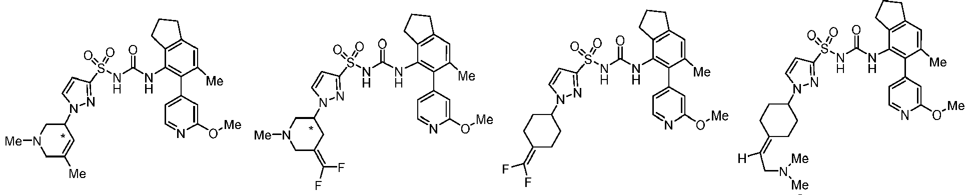 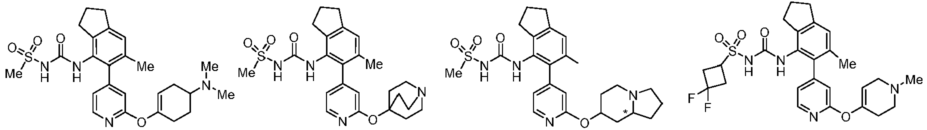 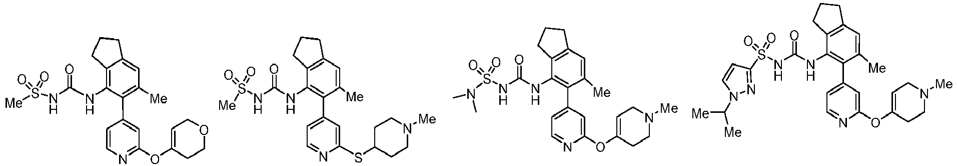 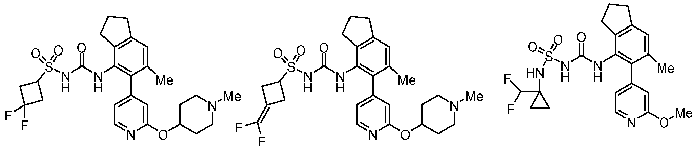 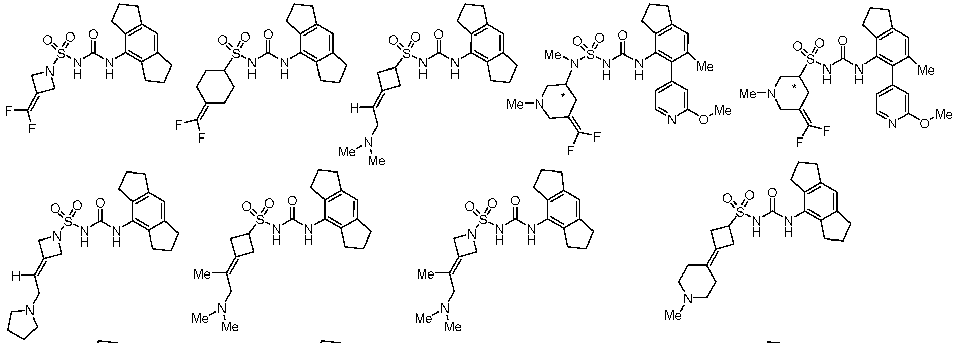 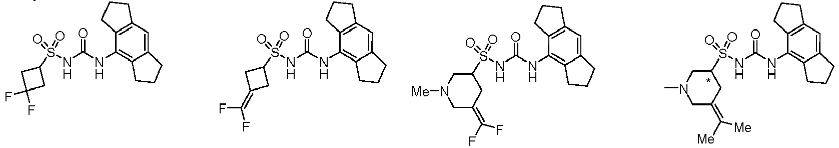 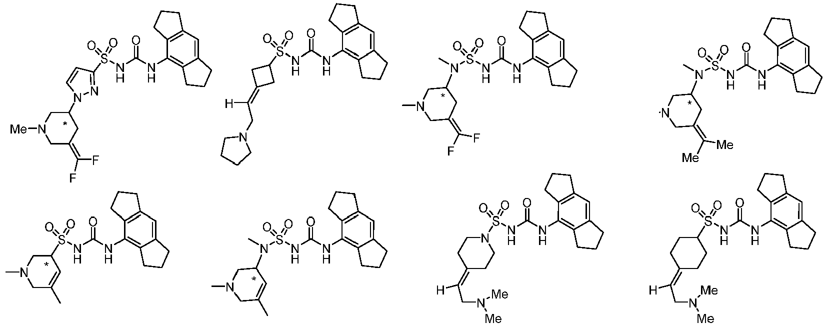 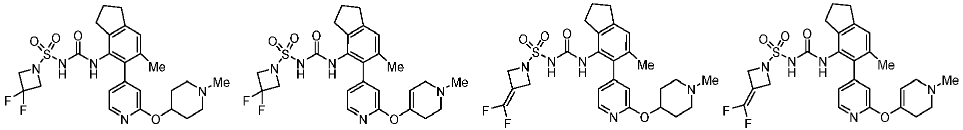 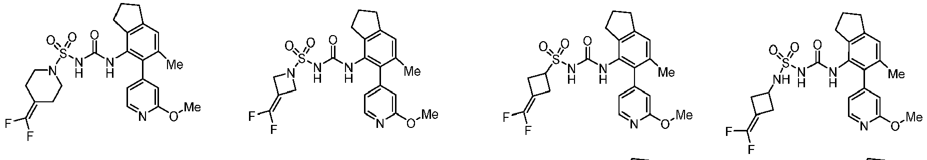 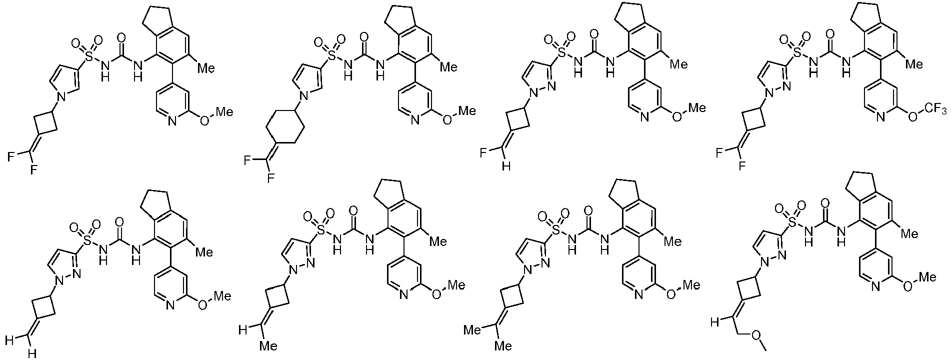 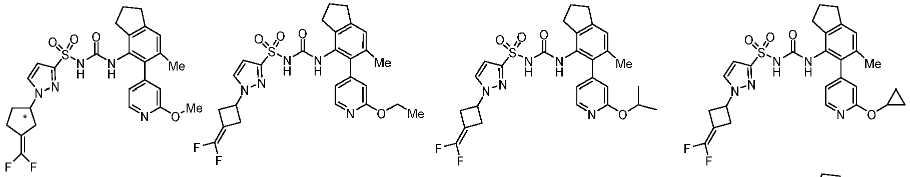 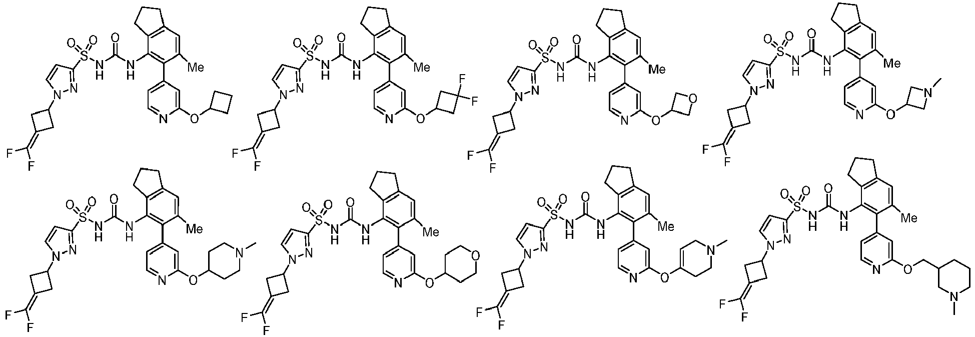 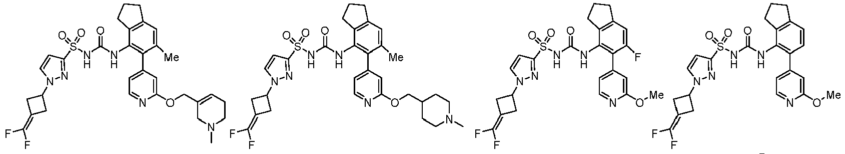 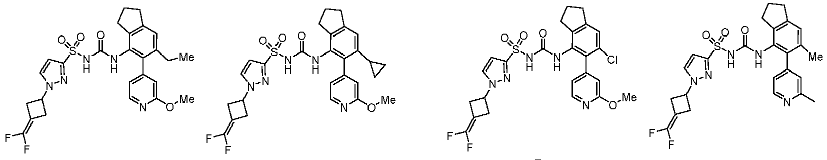 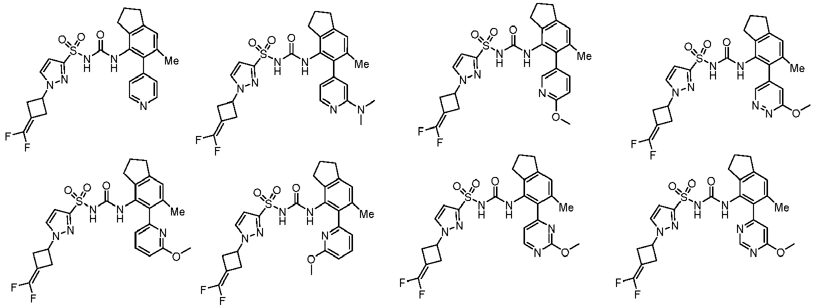 "*"represents chiral center.

17. The compound of any one of claims 1-16, or optical isomers, pharmaceutically acceptable salts, prodrugs, deuterated derivatives, hydrates, or solvates thereof, wherein, the pharmaceutically acceptable salt is alkali metal salts, preferably, is the salt selected from the group consisting of sodium salt, potassium salt, and lithium salt.

18. A pharmaceutical composition, comprising a compound of any one of claims 1-17, or optical isomers, pharmaceutically acceptable salts, prodrugs, deuterated derivatives, hydrates, or solvates thereof, and pharmaceutically acceptable carriers.

19. Use of a compound of any one of claims 1-17, or optical isomers, pharmaceutically acceptable salts, prodrugs, deuterated derivatives, hydrates, or solvates thereof, in the preparation of a pharmaceutical composition for the treatment of diseases, disorders or symptoms associated with NLRP3 activity or expression.

20. The use of claim 19, wherein, the disease, disorder or symptom is selected from the group consisting of inflammation, autoimmune disease, knee osteoarthritis, cancer, infection, central nervous system disease, metabolic disease, cardiovascular disease, respiratory disease, liver disease, kidney disease, ocular disease, skin disease, lymphatic condition, psychological disorder, graft versus host disease, allodynia, cryopyrin-associated periodic syndrome (CAPS), Muckle-Wells syndrome (MWS), familial cold autoinflammatory syndrome (FCAS), neonatal onset of multisystem inflammatory disease (NOMID), familial mediterranean fever (FMF), septic arthritis, pyoderma gangrenosum and acne syndrome (PAPA), hyperimmunoglobulinemia D and periodic fever syndrome (HIDS), tumor necrosis factor (TNF) receptor -associated periodic syndrome (TRAPS), systemic juvenile idiopathic arthritis, adult onset Still's disease (AOSD), relapsing polychondritis, schnitzler's syndrome, sweet syndrome, behcet's disease, anti synthetase syndrome, deficiency of interleukin-1 receptor antagonist (DIRA) and haploinsufficiency of A2o (HA2o).
